# EUROPÄISCHE PATENTSCHRIFT

(11) **EP 0 378 510 B1**
(45) Veröffentlichungstag und Bekanntmachung des Hinweises auf die Patenterteilung: **18.09.1996**
(21) Anmeldenummer: 90810004.3
(22) Anmeldetag: 04.01.1990
(51) Int. Cl.: C09B 69/00, C09B 29/08, C09B 23/14, C09B 1/26, C07D 211/76, C07D 223/10, C07C 271/60, C07C 333/12, C07C 275/28, D06P 1/16

(54) **Dispersionsfarbstoffe**
Disperse dyes
Colorants de dispersion

(30) Priorität: 13.01.1989 CH 107/89
(43) Veröffentlichungstag der Anmeldung: 18.07.1990
(73) Patentinhaber: CIBA-GEIGY AG, 4002 Basel (CH)
(72) Erfinder: Liechti, Peter, Dr., CH-4422 Arisdorf (CH); Trottmann, Martin, Dr., CH-4106 Therwil (CH)

(56) Entgegenhaltungen:
- EP-A- 0 284 560
- FR-A- 2 108 710
- GB-A- 824 443
- GB-A- 2 028 820

## Beschreibung

Die vorliegende Erfindung betrifft neue Dispersionsfarbstoffe, Verfahren zu deren Herstellung sowie Verfahren zum Färben und Bedrucken unter Verwendung dieser Farbstoffe.

Dispersionsfarbstoffe, d.h. Farbstoffe, welche keine wasserlöslichmachenden Gruppen enthalten, sind seit langem bekannt und werden als Farbstoffe z.B. zum Färben von hydrophobem Textilmaterial verwendet. Vielfach sind die erhaltenen Färbungen jedoch nicht ausreichend thermomigrierecht.

Zur Behebung dieses Mangels wurden schon spezielle Farbstoffe entwickelt, deren Diffusionsvermögen infolge ihrer molekularen Grösse und/oder Sperrigkeit möglichst niedrig ist. Dies erschwert jedoch oft die Anwendbarkeit solcher Farbstoffe, da man mit ihnen nicht oder nur schlecht nach dem Ausziehverfahren färben kann und diese Farbstoffe sogar im Thermosol-Verfahren oft unerwünscht hohe Fixiertemperaturen erfordern.

Gegenstand der vorliegenden Erfindung sind nun Dispersionsfarbstoffe, welche auf übliche Art und Weise applizierbarsind und durch eine thermische Nachbehandlung in eine thermomigrierechte Form übergeführt werden können.

Die erfindungsgemässen Farbstoffe entsprechen der Formel 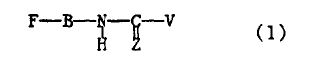 worin
F den Rest eines von wasserlöslichmachenden Gruppen freien Farbstoffes,
B ein Brückenglied oder eine direkte Bindung,
ZO oder S und
V den Rest einer Gruppe H-V bedeutet, wobei H-V ein Oxim der Formel 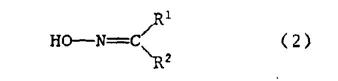 oder ein Lactam der Formel 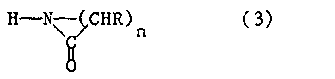 ist, worin
R¹ und R² unabhängig voneinander gegebenenfalls substituiertes Alkyl oder Aryl bedeuten oder R¹ und R² zusammen mit dem sie verbindenden C-Atom einen cycloaliphatischen Ring bilden,
R Wasserstoff oder Alkyl und
n eine ganze Zahl von 4 bis 11 bedeutet.

Aus den erfindungsgemässen Farbstoffen spaltet sich beim Erhitzen auf erhöhte Temperatur, z.B. auf eine Temperatur oberhalb 110°C, der Rest H-V ab und es entstehen Isocyanat- bzw. Isothiocyanatgruppen, welche mit geeigneten Gruppen in ihrer Umgebung reagieren können, z.B. mit den als Endgruppen des Polyesters oder dessen Oligomeren vorliegenden Hydroxylgruppen oder mit Aminen der Formel F-B-NH_{2;} die aus den Isocyanaten bzw. Isothiocyanaten durch Hydratation und Decarboxylierung entstehen und mit einem weiteren Isocyanat- bzw Isothiocyanatmolekül zu Verbindungen der Formel 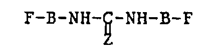 reagieren können.

Bei dem Rest F handelt es sich um den Rest eines wasserunlöslichen Chromophors, z.B. um den Rest eines der bekannten Dispersionsfarbstoffe. In Frage kommen z.B. Farbstoffreste aus folgenden Klassen:
Nitrofarbstoffe, z B. Nitrodiphenylaminfarbstoffe, Methinfarbstoffe, Chinolinfarbstoffe, Aminonaphthochinonfarbstoffe, Cumarinfarbstoffe und insbesondere Anthrachinonfarbstoffe, Tricyanvinylfarbstoffe und Azofarbstoffe, wie Monoazo und Disazofarbstoffe.

Bei dem Brückenglied B handelt es sich z B. um eine geradkettige oder verzweigte Alkylengruppe mit 2 bis 8 C-Atomen oder um eine der folgenden Gruppen 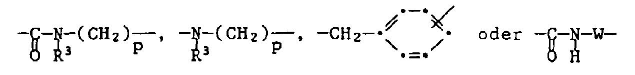 wobei R³ Wasserstoff oder C₁-C₆-Alkyl, p eine ganze Zahl von 1 bis 8 und W einen zweiwertigen organischen Rest bedeutet.

Geeignete Reste W sind z.B.: geradkettige oder verzweigte Alkylengruppen mit 2 bis 8 C-Atomen, ggf. durch C₁-C₄-Alkyl substituiertes Phenylen, C₁-C₄-Alkylenphenylen-C₁-C₄-Alkylen, Phenylen-C₁-C₄-Alkylen-Phenylen oder Reste, die sich von Isophorondiisocyanat ableiten. Bevorzugte Reste W sind die folgenden:
-(CH₂)₆-

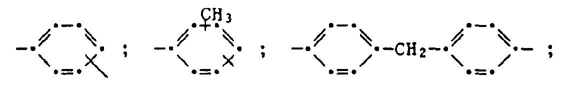

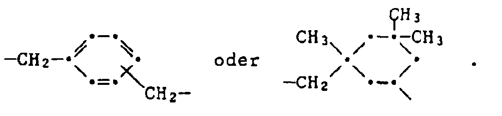

Unter diesen Brückengliedern sind die folgenden besonders bevorzugt: 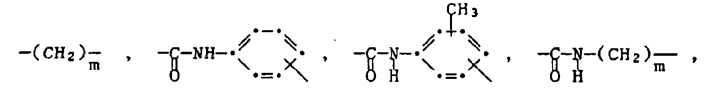 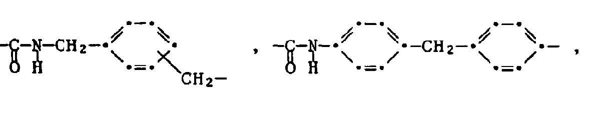 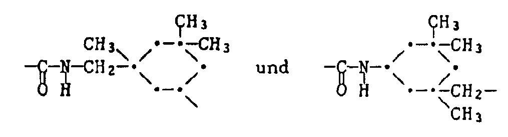 wobei m eine ganze Zahl von 2 bis 6 bedeutet.
Z bedeutet O oder S, vorzugsweise O.
R¹ und R² bedeuten unabhängig voneinander Alkyl, vorzugsweise C₁-C₁₂-Alkyl. oder Aryl, wobei der Arylrest aromatisch oder auch heteroaromatisch sein kann. Es handelt sich z.B. um Phenyl-, Naphthyl- oder Pyridylreste.
R¹ und R² können auch zusammen mit dem sie verbindenden C-Atom einen cycloaliphatischen Ring bilden, vorzugsweise einen 5- bis 7-gliedrigen Ring, wie Cyclopentyl, Cyclohexyl, Methylcyclohexyl oder Cycloheptyl.

Bei dem Alkylrest R handelt es sich vorzugsweise um einen C₁-C₄-Alkylrest, vor allem um Methyl. Die besonders bevorzugte Bedeutung von R ist jedoch Wasserstoff.

n ist vorzugsweise eine ganze Zahl von 4 bis 9, vor allem von 4 bis 7.

Bevorzugte erfindungsgemässe Farbstoffe entsprechen der Formel 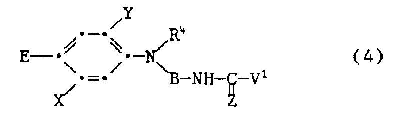 worin
E D-N=N- oder 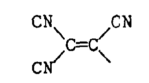 bedeutet, wobei D der Rest einer carbocyclischen oder heterocyclischen Diazokomponente ist,
X Wasserstoff, C₁-C₄-Alkyl, C₁-C₄-Alkoxy, Acylamino, Halogen, C₁-C₄-Alkylsulfonylamino oder eine Gruppe der Formel -NH-CO-NHQ, worin Q Wasserstoff, C₁-C₄-Alkyl oder Phenyl ist,
Y Wasserstoff, Halogen, C₁-C₄-Alkyl, C₁-C₄-Alkoxy, C₁-C₄-Alkoxy-C₁-C₄-alkyl oder C₁-C₄-Alkoxy-C₂-C₄-alkoxy,
R⁴ C₁-C₁₂-Alkyl, C₂-C₆-Alkenyl oder Phenyl, oder Y und R⁴, zusammen mit dem Stickstoffatom und den beiden sie verbindenden C-Atomen, einen 5- oder 6-gliedrigen Ring bilden,
B einen Rest der Formel 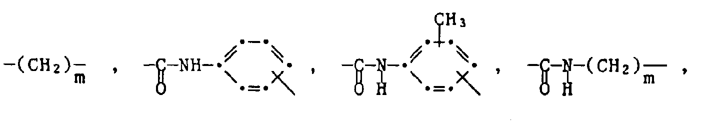 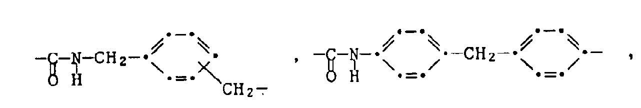 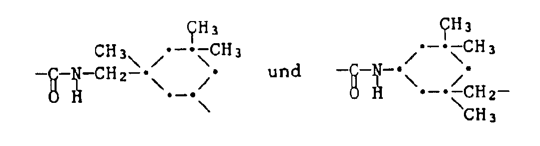 wobei
m eine ganze Zahl von 2 bis 6 bedeutet,
Z 0 oder S und
V1 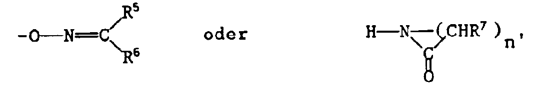 bedeutet, wobei
R⁵ und R⁶ unabhängig voneinander C₁-C₆-Alkyl bedeuten oder R⁵ und R⁶ zusammen mit dem sie verbindenden C-Atom einen Cyclopentyl-, Cyclohexyl- oder Cycloheptylrest bilden,
R⁷ Methyl oder Wasserstoff und
n' 4, 5, 6 oder 7 ist.

Unter diesen sind diejenigen besonders bevorzugt, bei denen E einen Rest D-N=N- bedeutet.

Falls in den Farbstoffen der Formel (4) E einen Rest D-N=N- darstellt, so bedeutet D den Rest einer homo- oder heterocyclischen Diazokomponente, z.B. aus der Reihe Thienyl, Phenylazothienyl, Thiazolyl, lsothiazolyl, 1,2,4-Thiadiazolyl, 1,3,4-Thiadiazolyl, Benzthiazolyl, Benzisothiazolyl, Pyrazolyl, 1,2,3-Triazolyl, 1,2,4-Triazolyl, Imidazolyl, oder Phenyl. Jedes dieser Systeme kann weitere Substituenten tragen wie Alkyl, Alkoxy oder Alkylthio mit je 1 bis 4 Kohlenstoffatomen, Phenyl, elektronegative Gruppen wie Halogen, besonders Chlor oder Brom, Trifluormethyl, Cyano, Nitro, Acyl, wie z.B. Acetyl oder Benzoyl, Carboalkoxy, besonders Carbomethoxy oder Carboethoxy, Alkylsulfon mit 1 bis 4 Kohlenstoffatomen, Phenylsulfon, Phenoxysulfon, Sulfonamido oder Arylazo, insbesondere Phenylazo. Je 2 benachbarte Substituenten der genannten Ringsysteme können auch zusammen weitere ankondensierte Ringe bilden, z.B. Phenylringe oder cyclische lmide.

Vorzugsweise bedeutet D einen Benzthiazolyl-, Benzisothiazolyl- oder Phenylrest, welcher unsubstituiert oder ein-oder zweimal durch einen der obengenannten Reste substituiert ist.

Als Benzthiazolyl- oder Benzisothiazolylrest stellt D vorallem einen unsubstituierten oder einen ein- oder mehrmals durch Methyl, Methoxy, Chlor, Brom, Methylsulfon oder Nitro substituierten Rest dar.

Die bevorzugte Bedeutung von D ist Phenyl, welches maximal mit 4, gegebenenfalls verschiedenen, Substituenten aus der oben genannten Aufzählung substituiert ist. Von den aufgezählten Substituenten sind elektronegative bevorzugt. Diese können vor allem Cyano, Methyl- oder Ethylsulfonyl, Nitro, Chlor, Brom, Formyl, Acetyl, Benzoyl, Carbomethoxy, Carboethoxy, Methoxy, Ethoxy oder Phenylazo sein.

Unter Alkylgruppen sind in dieser Anmeldung generell geradkettige oder verzweigte oder cyclische Alkylgruppen zu verstehen, z.B. solche mit 1 bis 12 Kohlenstoffatomen. Es handelt sich z.B. um Methyl, Ethyl, Propyl, i-Propyl, Butyl, i-Butyl, tert.-Butyl, Amyl, tert.-Amyl (1,1-Dimethylpropyl), 1,1,3,3-Tetramethylbutyl, Hexyl, 1-Methylpentyl, Neopentyl, 1-, 2- oder 3-Methylhexyl, Heptyl, n-Octyl, tert. Octyl, 2-Ethylhexyl, n-Nonyl, Isononyl, Decyl, Dodecyl, Cyclopentyl, Cyclohexyl, Methylcyclohexyl sowie die dazu gehörenden Isomeren.

Diese Alkylreste können substituiert sein, z.B. durch Hydroxy, Alkoxy mit 1 bis 4 Kohlenstoffatomen, insbesondere Methoxy, Halogen, wie Brom oder Chlor, Cyano oder Phenyl. Als weitere Substituenten sind geeignet Halogen, wie Fluor, Chlor oder Brom, oder -CO-U oder -O-CO-U, worin U Alkyl mit 1 bis 6 Kohlenstoffatomen oder Phenyl ist.

Als Alkenylreste kommen solche Reste in Betracht, welche sich von den oben aufgeführten Alkylresten durch Ersatz mindestens einer Einfachbindung durch eine Doppelbindung ableiten. Geeignete Reste sind z.B. Ethenyl oder Propenyl.

Geeignete Alkoxyreste sind z.B. Methoxy, Ethoxy, Propoxy, iso-Propoxy, n-Butoxy, i-Butoxy oder tert.-Butoxy.

Beispiele für geeignete substituierte Alkylreste sind: Methoxymethyl, Ethoxymethyl, Ethoxyethyl, Ethoxypropyl, n-Propoxymethyl, iso-Propoxymethyl, Butoxymethyl, Butoxyethyl, Butoxypropyl, Ethoxypentyl, Methoxybutyl, Ethoxypentyl, 2-Hydroxyethoxypentyl, Cyanethyl, Hydroxyethyl oder Acetoxyethyl.

Auch die Alkylenreste B können geradkettig oder verzweigt oder auch substituiert sein. Es kommen z.B. Ethylen, 1,3-Propylen, 1,5-Pentylen, 1,2-Propylen, 1,2-Butylen, 1,6-Hexylen, 2-Hydroxy-1,3-propylen oder 2-Chlor-1;3-propylen in Frage.

Falls X eine Acylaminogruppe darstellt, so handelt es sich z.B. um eine Gruppe der Formel 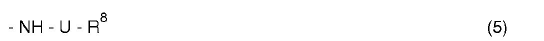 wobei U -CO- oder -S0₂- und R⁸ gegebenenfalls substituiertes Alkyl oder Phenyl bedeutet.

Es handelt sich bei dem Acylrest z.B. um den Acetyl-, Propionyl-, 2-Chlorethylcarbonyl-, 2-Bromethylcarbonyl-, Phenylcarbonyl-, 2-Methoxycarbonylethylcarbonyl-, 2-Ethoxycarbonylethylcarbonyl-, Methoxycarbonyl-, Ethoxycarbonyl-, Phenoxycarbonyl, Methoxyethylcarbonyl-, Hydroxyethylcarbonyl-, Methylsulfonyl- oder Ethylsulfonylrest.

Ist X eine Gruppe der Formel -NH-CO-NHQ, so handelt es sich z.B. um die Ureido-, Methylureido-, Ethylureido-oder Phenylureidogruppe.

Unter Phenylresten sind in dieser Anmeldung generell unsubstituierte oder substituierte Phenylreste zu verstehen. Als Substituenten kommen z.B. Cᵢ-C₄₋Alkyl, C₁-C₄-Alkoxy, Brom, Chlor, Nitro oder C₁-C₄-Alkylcarbonylamino in Betracht.

Halogen bedeutet in dieser Anmeldung generell Fluor, Brom oder vor allem Chlor.

R⁴ und Y können zusammen mit dem Stickstoffatom und den beiden sie verbindenden C-Atomen einen 5- oder 6-gliedrigen Ring bilden, welcher gegebenenfalls ein Sauerstoffatom als weiteres Heteroatom enthalten kann. Geeignete Substituenten für diese Ringe sind z.B. Hydroxy, Methyl, Methoxy, Chlor oder Phenyl. Vorzugsweise bilden R⁴ und Y zusammen mit dem Stickstoffatom und den beiden sie verbindenden C-Atomen einen 6-gliedrigen Ring, welcher unsubstituiert ist oder 1 bis 4 Methylgruppen trägt. Es handelt sich also vor allem um Di- oder Tetrahydrochinolinverbindungen mit 0 bis 4 Methylgruppen.

In besonders bevorzugten Dispersionsfarbstoffen ist D ein Benzthiazolylrest, welcher unsubstituiert ist oder ein-oder zweimal durch Chlor substituiert ist, oder ein Phenylrest, der ein- oder zweimal substituiert ist durch Nitro, Chlor, Cyan, Methyl- oder Ethylsulfonyl oder Phenylazo.

Die bevorzugten Bedeutungen von X sind Wasserstoff, Methyl, Methoxy, Chlor, Brom, Acetylamino oder Ureido, wobei Wasserstoff, Methyl, Chlor und Acetylamino besonders bevorzugt sind.

Y bedeutet vorzugsweise Chlor, Methyl, Methoxy, Methoxyethyl oder Methoxyethoxy oder vor allem Wasserstoff.

Besonders wertvolle erfindungsgemässe Farbstoffe entsprechen den Formeln 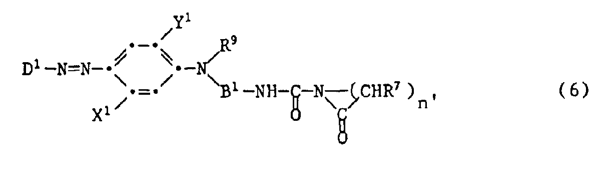 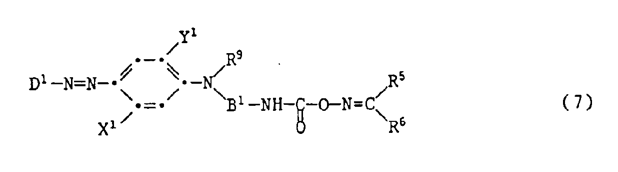 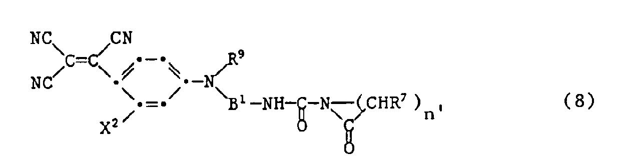 oder

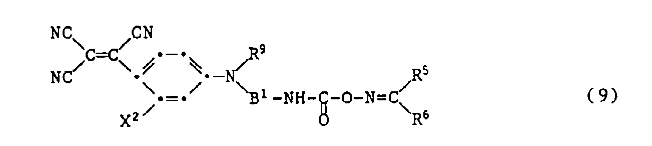

In den Formeln (6) bis (9) bedeuten:
D¹ einen Benzthiazolylrest, welcher unsubstituiert ist oder ein- oder zweimal durch Chlor substituiert ist oder einen Phenylrest, der ein- oder zweimal durch Nitro, Chlor, Cyan, Methyl- oder Ethylsulfonyl oder Phenylazo substituiert ist,
X¹ Wasserstoff, Methyl, Methoxy, Chlor, Brom oder Acetylamino,
^{y1} Chlor, Methyl, Methoxy, Methoxyethyl, Methoxyethoxy oder Wasserstoff,
X² Wasserstoff, Methyl, Methoxy, Chlor oder Brom,
R⁹ C₁-C₄-Alkyl, welches unsubstituiert ist oder durch Hydroxy, Cyan, C₁-C₄-Alkoxy oder Phenyl substituiert ist,
B¹ einen Rest der Formel 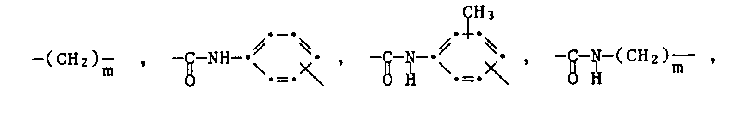 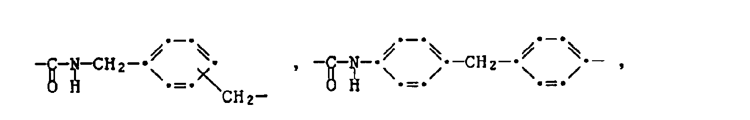 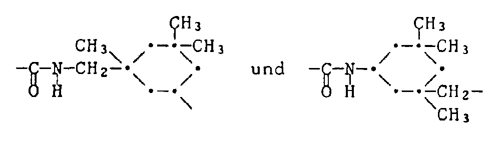 wobei
m eine ganze Zahl von 2 bis 6 bedeutet,
R⁵ und R⁶ unabhängig voneinander C₁-C₆-Alkyl oder R⁵ und R⁶ bilden zusammen mit dem sie verbindenden C-Atom einen Cyclopentyl-, Cyclohexyl- oder Cycloheptylrest,
_{R}⁷ Methyl oder Wasserstoff und
n' 4, 5, 6 oder 7.

Die Farbstoffe der Formel (4) stellt man z.B. her, indem man ein diazotiertes Amin der Formel 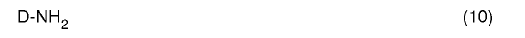 oder Tetracyanethylen mit einer Verbindung der Formel 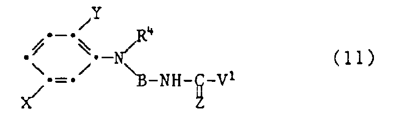 umsetzt.

Die Verbindungen der Formel (10) sind bekannt oder lassen sich in Analogie zur Herstellung ähnlicher Verbindungen herstellen.

Die Verbindungen der Formel (11) sind neu und stellen einen weiteren Gegenstand der vorliegenden Erfindung dar. Sie werden z.B. hergestellt, indem man Halogenalkylisocyanat oder Halogenalkylisothiocyanat der Formel 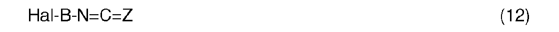 mit einem Oxim oder Lactam der Formel 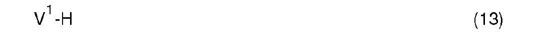 umsetzt und das erhaltene Reaktionsprodukt der Formel 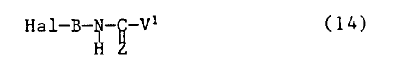 mit einer Verbindung der Formel 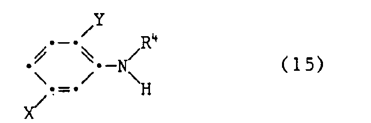 zur Reaktion bringt.

In den Verbindungen (10) bis (15) weisen D, X, Y, R⁴, B, Z, und V¹ die unter der Formel (4) angegebene Bedeutung auf und Hal bedeutet Chlor oder Brom.

Die Verbindungen der Formeln (12) bis (15) sind bekannt oder lassen sich auf an und für sich bekannte Art und Weise herstellen.

Bei der Umsetzung der Verbindung (12) mit der Verbindung (13) arbeitet man beispielsweise in einem inerten Lösungsmittel und in Gegenwart eines Katalysators, wie z B. Diazabicyclooctan, und bei einer Temperatur etwa zwischen 0°C und 20°C. Die Komponenten (12) und (13) können in stöchiometrischem Verhältnis eingesetzt werden, ein Ueberschuss an einer der Komponenten ist jedoch ebenfalls möglich. Nach Beendigung der Umsetzung wird die Verbindung (14) auf übliche Weise, z.B. durch Kristallisation isoliert. Es ist auch möglich, die Verbindungen (12) und (13) ohne Lösungsmittel miteinander umzusetzen.

Bei der Umsetzung der Verbindung (14) mit der Verbindung (15) arbeitet man beispielsweise in einem inerten organischen Lösungsmittel und in Gegenwart einer Base. Als inerte organische Lösungsmittel kommen z.B. Verbindungen mit einem Siedepunkt oberhalb 60°C in Frage, wie Alkohole, Ether, Ester, Nitrobenzol, Halogenbenzol, Toluol, Xylole etc. Vor allem geeignet sind Alkohole, wie z.B. Isopropanol, und als Base wird z.B. Natrium-oder Kaliumcarbonat verwendet. Die Umsetzung der Verbindung (14) mit der Verbindung (15) kann ebenfalls auch ohne Zusatz eines Lösungsmittels durchgeführt werden.

Die Komponenten (14) und (15) können in stöchiometrischem Verhältnis eingesetzt werden, ein Ueberschuss an einer der Komponenten, vorzugsweise der Verbindung (14), erweist sich jedoch oft als günstiger.

Die Reaktionstemperatur liegt etwa zwischen 50 und 120°C, vorzugsweise zwischen 60 und 100°C und die Reaktionsdauer beträgt, je nach Temperatur und Reaktionspartnern etwa 1 bis 20 Stunden. Nach Beendigung der Umsetzung wird die evtl. im Ueberschuss eingesetzte Komponente (15) und das Lösungsmittel entfernt und der Rückstand evtl. gereinigt, z.B. durch Umkristallisation. Die Diazotierung der Verbindung (10) und die Kupplung mit der Verbindung (11) erfolgen nach den üblichen Methoden.

Die Umsetzung der Verbindungen der Formel (11) mit Tetracyanethylen erfolgt auf an sich bekannte Art und Weise, vorzugsweise in einem inerten Lösungsmittel bei einer Temperatur zwischen etwa 20 und 100°C, wobei die Reaktionskomponenten in etwa äquivalenten Mengen eingesetzt werden.

Als inertes Lösungsmittel für die vorstehende Reaktion kommen z.B. Halogenverbindungen wie Chloroform oder Chlorbenzol, Ether, aromatische Verbindungen, wie Benzol, Toluol oder Xylol, insbesondere jedoch Tetrahydrofuran oder Dimethylformamid in Frage.

Die Isolierung der Farbstoffe der Formel (4) erfolgt z.B. indem man die Reaktionslösungen in Eiswasser giesst, den ausgeschiedenen Farbstoff abfiltriert, eventuell wäscht und trocknet.

Eine weitere Methode zur Herstellung von Farbstoffen der Formel (1) besteht darin, dass man einen Farbstoff der Formel F-H, worin F die unter der Formel (1) angegebene Bedeutung aufweist, mit einer Verbindung der Formel (14) umsetzt. Die Reaktionsbedingungen entsprechen den vorher beschriebenen Bedingungen für die Umsetzung einer Verbindung der Formel (14) mit einer Verbindung der Formel (15).

Die vorstehend beschriebenen Darstellungsweisen eignen sich vor allem für solche Verbindungen der Formel (4), bei denen V einen Lactamrest bedeutet.

Farbstoffe der Formel (1) oder (4), bei denen V bzw. V einen Oximrest bedeutet, stellt man vorzugsweise her, indem man eine Verbindung der Formel 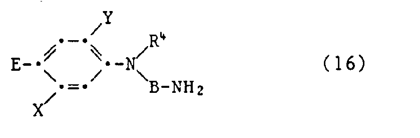 oder der Formel 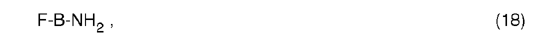 mit einem Halogenameisensäureester eines Oxims der Formel 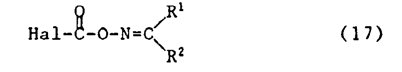 umsetzt. Die Symbole E, X, Y, R⁴, B, F, Hal, R¹ und R² weisen dabei die unter den Formeln (1) und (4) angegebenen Bedeutungen auf.

Die Verbindungen der Formeln (16), (17) und (18) sind bekannt oder lassen sich in Analogie zur Herstellung ähnlicher Verbindungen herstellen.

Bei der Umsetzung der Verbindung (16) bzw. (18) mit der Verbindung (17) arbeitet man vorzugsweise in einem inerten organischen Lösungsmittel und in Gegenwart einer Base. Als inerte organische Lösungsmittel kommen z.B. Kohlenwasserstoffe, Halogenkohlenwasserstoffe, Alkohole, Ether, Ester, Nitrobenzol, Halogenbenzol, Toluol, Xylole etc. in Frage. Vor allem geeignet sind halogenierte Kohlenwasserstoffe, wie z.B. Dichlormethan, und als Base wird z. B. Natrium- oder Kaliumcarbonat oder eine organische Base, z.B. ein Trialkylamin verwendet.

Die Komponenten (16) bzw. (18) und (17) können in stöchiometrischem Verhältnis eingesetzt werden, ein Ueberschuss an einer der Komponenten, vorzugsweise der Verbindung (17), erweist sich jedoch oft als günstiger.

Die Reaktionstemperatur liegt etwa zwischen 0 und 60°C, vorzugsweise zwischen 10 und 30°C und die Reaktionsdauer beträgt, je nach Temperatur und Reaktionspartnern etwa 0,1 bis 2 Stunden. Nach Beendigung der Umsetzung wird die evtl. im Ueberschuss eingesetzte Komponente (17) und das Lösungsmittel entfernt und der Rückstand evtl. gereinigt.

Farbstoffe der Formel (1) oder (4), bei denen B ein Brückenglied der Formel 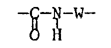 darstellt, wobei W einen zweiwertigen organischen Rest bedeutet, werden vorzugsweise hergestellt, indem man ein Diisocyanat der Formel 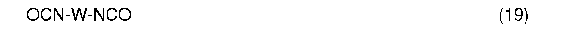 in beliebiger Reihenfolge mit einem Farbstoff, enthaltend eine reaktionsfähige -OH- oder 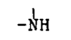 Gruppe, und mit einem Oxim der Formel (2) oder einem Lactam der Formel (3) umsetzt Die Reaktionsbedingungen entsprechen den bekannten Bedingungen für die Reaktion von Isocyanaten mit OH oder )NH-Gruppen enthaltenden Verbindungen.

Die erfindungsgemässen Farbstoffe können zum Färben und Bedrucken von halbsynthetischen und insbesondere synthetischen hydrophoben Fasermaterialien, vor allem Textilmaterialien, verwendet werden. Textilmaterialien aus Mischgeweben, die derartige halbsynthetische bzw. synthetische hydrophobe Textilmaterialien enthalten, können ebenfalls mit Hilfe der erfindungsgemässen Verbindungen gefärbt oder bedruckt werden.

Als halbsynthetsiche Textilmaterialien kommen vor allem solche aus Cellulose-2 1/2-Acetat und Cellulosetriacetat in Frage.

Synthetische hydrophobe Textilmaterialien bestehen vor allem aus linearen, aromatischen Polyestern, beispielsweise solchen aus Terephthalsäure und Glykolen, besonders Ethylenglykol oder Kondensationsprodukten aus Terephthalsäure und 1,4-Bis-(hydroxymethyi)-cyciohexan; aus Polycarbonaten, z.B. solchen aus α,α.-Dimethyl-4,4'-dihy- droxy-diphenylmethan und Phosgen, aus Fasern auf Polyvinylchlorid- sowie Polyamid-Basis.

Die Applikation der erfindungsgemässen Verbindungen auf die Textilmaterialien erfolgt nach bekannten Färbeverfahren. Beispielsweise färbt man Polyesterfasermaterialien im Ausziehverfahren aus wässriger Dispersion in Gegenwart von üblichen anionischen oder nicht ionischen Dispergiermitteln und gegebenenfalls üblichen Quellmitteln (Carriern) bei Temperaturen zwischen 80 und 140°C. Fasermaterialien aus Cellulose-2 1/2-acetat färbt man vorzugsweise zwischen ungefähr 65 bis 85°C und solche aus Cellulosetriacetat bei Temperaturen bis zu 115°C.

Einige der erfindungsgemässen Farbstoffe eignen sich aber vor allem zum Färben nach dem Thermosol-Verfahren.

Das genannte Textilmaterial kann dabei in den verschiedensten Verarbeitungsformen vorliegen, wie z.B. als Faser, Faden oder Vlies, als Gewebe oder Gewirke.

Es ist vorteilhaft, die erfindungsgemässen Farbstoffe vor ihrer Verwendung in ein Farbstoffpräparat überzuführen. Hierzu wird der Farbstoff vermahlen, so dass seine Teilchengrösse im Mittel zwischen 0,1 und 10 Mikron beträgt. Das Vermahlen kann in Gegenwart von Dispergiermitteln erfolgen. Beispielsweise wird der getrocknete Farbstoff mit einem Dispergiermittel gemahlen oder in Pastenform mit einem Dispergiermittel geknetet und hierauf im Vakuum oder durch Zerstäuben getrocknet. Mit den so erhaltenen Präparaten kann man nach Zugabe von Wasser färben und bedrucken.

Beim Bedrucken wird man die üblichen Verdickungsmittel verwenden, z.B. modifizierte oder nichtmodifizierte natürliche Produkte, beispielsweise Alginate, British-Gummi, Gummi arabicum, Kristallgummi, Johannisbrotkernmehl, Tragant, Carboxymethylcellulose, Hydroxyethylcellulose, Stärke oder synthetische Produkte, beispielsweise Polyacrylamide, Polyacrylsäure oder deren Copolymere oder Polyvinylalkohole.

Nach dem eigentlichen Färbeverfahren werden die erhaltenen Färbungen einer thermischen Nachbehandlung unterworfen, z.B. indem man sie 30 bis 500 Sekunden, vorzugsweise 45 bis 200 Sekunden auf eine Temperatur zwischen 100 und 240°C, vorzugsweise zwischen 180 und 220°C erhitzt. Durch diese thermische Nachbehandlung wird die Thermomigrierechtheit der Färbungen wesentlich verbessert, da die Farbstoffe unter Abspaltung der entsprechenden Reste in Farbstoffe mit Isocyanat- oder Isothiocyanatgruppen umgewandelt werden. Diese Gruppen können dann mit geeigneten Gruppen der Fasern oder der auf den Fasern vorhandenen Chemikalien, z.B mit Amin- oder Hydroxygruppen reagieren.

Falls die Färbungen nach dem Thermosolverfahren hergestellt wurden, welches normalerweise eine Hitzefixierung bei etwa 180 210°C beinhaltet, so kann bei einem Teil der erfindungsgemässen Farbstoffe eine gesonderte thermische Nachbehandlung entfallen. Andernfalls empfiehlt es sich, die Hitzefixierung bei erhöhterTemperatur, z. B. 200 bis 230°C und/oder während einer längeren Dauer, z.B. 120 bis 300 Sekunden durchzuführen.

Bei einem Teil der erfindungsgemässen Farbstoffe genügen zur Fixierung bereits Temperaturen von 110 bis 140°C. Bei diesen kann deshalb die thermische Nachbehandlung in einen HT-Färbevorgang integriert werden, bzw. ganz entfallen.

Die erfindungsgemässen Farbstoffe verleihen den genannten Materialien, vor allem dem Polyestermaterial, egale gelbe bis blaue Farbtöne von sehr guten Gebrauchs-Echtheiten, wie vor allem guter Lichtechtheit, Sublimierechtheit, Thermofixier-, Plissier-, Chlor- und Nassechtheit wie Wasser-, Schweiss- und Waschechtheit; die Ausfärbungen sind ferner gekennzeichnet durch gute Reibechtheit und Thermofixierechtheit. Es werden ausserdem sehr farbstarke Färbungen erzielt.

Die folgenden Beispiele veranschaulichen die Erfindung weiter, ohne sie darauf zu beschränken. Teile und Prozente beziehen sich, sofern nicht anders angegeben, auf das Gewicht. Die Temperaturen sind in Grad Celsius angegeben.

Beispiel 1: Zu einer Lösung von 19,7 g (0,1 Mol) N-2-Aminoethyl-N-ethylanilin (hergestellt durch Hydrolyse von Phthalimidoethyl-ethylanilin in wässrigem Bromwasserstoff) in 1000 ml Essigsäuretropft man innerhalb von 15 Minuten bei 7-10°C eine nach üblicher Methode hergestellte Diazoniumchloridlösung von 17,3 g (0,1 Mol) 2-Chlor-4-nitroanilin. Nach zweistündigem Nachrühren wird die violettrote Suspension abgenutscht, das Nutschgut in 400 ml Wasser aufgeschlämmt, mit 60 ml 30-prozentiger Natriumhydroxidlösung auf pH ca. 11 gestellt, abgenutscht, gewaschen und im Vakuumschrank getrocknet. Man erhält 27,6 g (79,3 % der berechneten Menge) des roten Farbstoffes der Formel 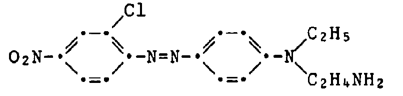 als graues Pulver vom Schmelzpunkt 133-134°C.

17,4 g (0,05 Mol) dieses Aminoethylfarbstoffes werden in 200 ml Dichlormethan gelöst, mit 5,5 g Triethylamin (0,055 Mol) versetzt und innerhalb von 20 Minuten tropfenweise unter Einhaltung einer Maximaltemperatur von 25°C mit 25 ml rohem Chlorameisensäureester von Acetonoxim (ca. 10% Ueberschuss) versetzt. Nach einer halben Stunde wird die Reaktionslösung zuerst mit Wasser, dann mit verdünnter Salzsäure und schliesslich wieder mit Wasser ausgeschüttelt. Nach dem Eindampfen der Dichlormethanschicht, Waschen des Rückstandes mit Isopropanol und Trocknen erhält man 20,9 g (93,6 % d.Th.) des Farbstoffes der Formel 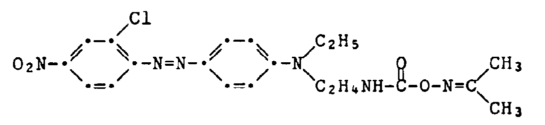 als violettes Pulver vom Schmelzpunkt 148-149°.

Beispiel 2: Ersetzt man im Beispiel 1 den Chlorameisensäureester von Acetonoxim durch eine äquivalente Menge desjenigen von 2-Butanonoxim, so erhält man unter sonst gleichen Bedingungen den Farbstoff der Formel 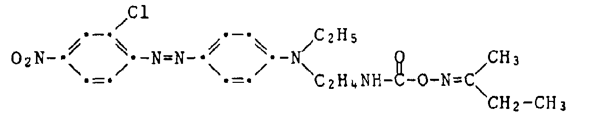 vom Schmelzpunkt 107-108°C

Beispiel 3: Ersetzt man im Beispiel 1 den Chlorameisensäureester von Acetonoxim durch eine äquivalente Menge des Chlorameisensäureesters von Cyclohexanonoxim, so erhält man unter sonst gleichen Bedingungen den Farbstoff der Formel 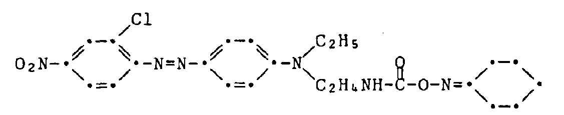 vom Schmelzpunkt 152-154°C.

Beispiel Verwendet man im Beispiel 1 anstelle des Aminoethyl-ethylanilins die äquivalente Menge N-Aminoethyl-N-ethyl-m-toluidin (erhalten durch Hydrolyse der Succinimidoethylverbindung), so erhält man den Farbstoff der Formel 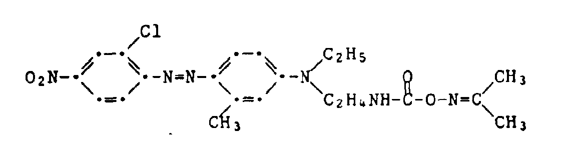 vom Schmelzpunkt 68°C.

Beispiel 5: 21,5 g (0,093 Mol) 3-N-Cyanoethyl-N-ethyl-amino-acetanilid wird in 100 ml wasserfreiem Ethanol und 30 g flüssigem Ammoniak in Gegenwart von 4,3 g Raneynickel bei 70-75°C unter einem Anfangsdruck von 150 Bar Wasserstoff hydriert. Nach 1,5-Stunden kommt die Wasserstoffaufnahme zum Stillstand. Man filtriert den Katalysator ab und dampft die Lösung ein, was 22,5 g (ungefähr die berechnete Menge) der rohen Verbindung der Formel 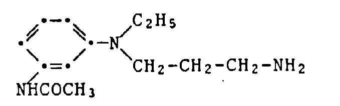 als hochviskoses braunes Oel ergibt

5,7 g (ca. 0,024 Mol) dieser rohen Kupplungskomponente wird nun in 200 ml 2 N Schwefelsäure mit der aus 3,45 g (0,02 Mol) 2-Chlor-4-nitroanilin hergestellten Diazoniumchloridlösung während 2 Stunden bei 0-10°C zur Reaktion gebracht. Dann wird der braune Farbstoff abfiltriert, mit 2 N Schwefelsäure gewaschen, in 200 ml Wasser aufgeschlämmt und mit Natriumhydroxid auf pH 10 gestellt. Erneutes Abnutschen, Waschen mit Wasser und Trocknen liefert 5,8 g (69 % d.Th.) als grünbraunes, chromatographisch uneinheitliches Pulver vom Schmelzpunkt 144-147° (Sintern bei 105°C).

2,1 g (0,05 Mol) davon werden in 20 ml Dichlormethan gelöst, mit 0,76 ml (ca. 10 % Ueberschuss) Triethylamin und dann innerhalb von 10 Minuten bei 22-25°C mit 5 ml rohem Chlorameisensäureester von Acetonoxim versetzt. Nach einstündigem Nachrühren ist das Ausgangsmaterial im Dünnschichtchromatogramm nicht mehr nachweisbar. Man dampft die Dichlormethanlösung zusammen mit 9 g Kieselgel ein und reinigt das Gemisch an einer Kieselgelsäule mit Ethylacetat als Eluiermittel und erhält 0,9 g des Farbstoffes der Formel 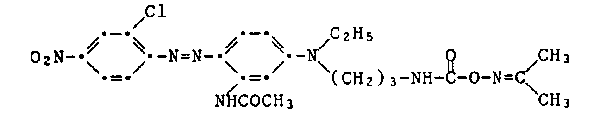 als braunes Pulver von Schmelzpunkt 167-168°, dessen Kernresonanzspektrum die Struktur bestätigt.

Beispiele 6-9: In analoger Weise werden aus der im Beispiel 5 beschriebenen Kupplungskomponente, den in Spalte 1 der folgenden Tabelle verzeichneten Diazokomponenten und den Chlorameisensäureestern der in Spalte 2 aufgeführten Ketoxime die folgenden Farbstoffe hergestellt:

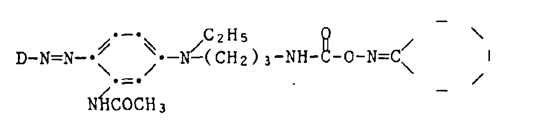

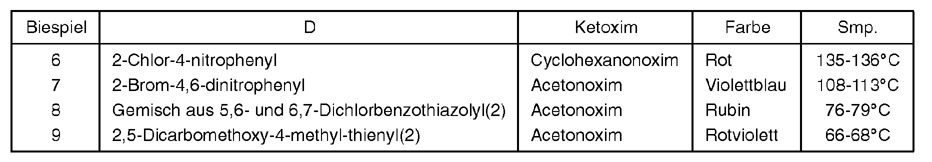

Beispiel 10: Eine Mischung gleicher Teile der in den Beispielen 1, 2 und 3 beschriebenen Farbstoffe wird durch Sandmahlung mit der doppelten Menge eines handelsüblichen Dispergators vom Typ Dinaphthylmethandisulfonat in bekannter Weise in eine 5-prozentige wässrige Dispersion übergeführt. Mit dieser Formulierung wird in üblicher Weise eine bezüglich Farbstoff 1-prozentige Carrierfärbung (95°C, Carrier auf der Basis von Trichlorbenzol und Biphenyl) auf Polyethylenterephthalatgewebe erstellt und reduktiv gereinigt. Das tiefrote Gewebe ist sehr gut reib-und waschecht.

Erhitzt man es aber zur Prüfung der Thermomigrierechtheit 45 Sekunden auf 160°C, so wird die Reibechtheit sehr schlecht und in üblichen Waschtests bei 60°C werden von den Begleitgeweben vor allem Celluloseacetat und Polyamid sehr starkangeblutet. Dieses Bild wird noch erheblich verschlechtert, wenn das Gewebe vordem Thermomigrationstest mit einem Textil-Weichmacher vom Typ Distearyl-diethylentriamin ausgerüstet wird.

Wird das gefärbte Gewebe dagegen vor der reduktiven Reinigung 60 Sekunden auf 210°C erhitzt, so bleibt der Thermomigrationstest mit und ohne Weichmacher praktisch ohne Einfluss auf die Reib- und Waschechtheiten und aus dem Gewebe lässt sich selbst durch einstündiges Extrahieren mit Dimethylformamid bei 95°C nur noch wenig Farbstoff extrahieren.

Beispiel 11: Mit der im Beispiel 10 beschriebenen 5-prozentigen Farbstofformulierung wird im HT-Verfahren bei 130°C in üblicher Weise eine bezüglich Farbstoff 1-prozentige Färbung erstellt und reduktiv gereinigt.

Die in der im Beispiel 10 beschriebenen Weise geprüfte Thermomigrationsechtheit erweist sich als sehr gut. Gleich verhält sich eine HT-Färbung mit einer 5-prozentigen Dispersion des im Beispiel 1 beschriebenen Farbstoffes.

Beispiel 12: Eine mit einer 5-prozentigen Dispersion des Farbstoffes von Beispiel 1 durch Foulardieren und Thermofixieren bei 200° hergestellte starke rote Färbung erweist sich in den beschriebenen Tests als sehr gut thermomigrierecht.

Beispiel 13: 12,0 g wasserfreies e-Caprolactam werden in 30 ml Toluol gelöst und bei Raumtemperatur langsam zugetropft zu einer Vorlage von 17,8 g Toluylen-2,4-diisocyanat und 0,05 g Diazabicyclooctan. Nach 22 Stunden Rühren bei Raumtemperatur und 6 Stunden Rühren bei 80°C wird die Lösung mehrere Tage bei Raumtemperatur stehen gelassen. Der sich ausbildende Niederschlag wird abfiltriert und getrocknet. Man erhält 6,1 g farblose Kristalle der Formel 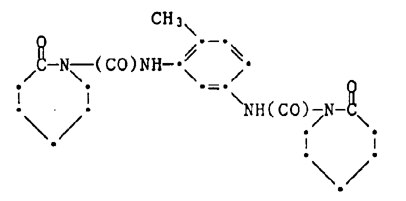 und vom Schmelzpunkt 164 - 166°C.

Die Mutterlauge enthält ca. 29 g Produkt der Formel 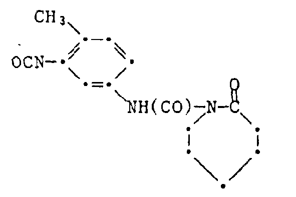 als Toluollösung.

Von dieser Lösung nimmt man 1/4 und destilliert das Lösungsmittel ab.

Nach Zugabe von 50 ml wasserfreiem Dimethylformamid zum Rückstand wird 6,3 g Farbstoff der Formel 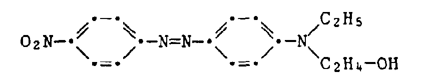 zugefügt. Während 48 Stunden wird bei Raumtemperatur gerührt und anschliessend ausgegossen auf 500 g Eiswasser. Nach Filtrieren, Waschen und Trocknen erhält man 11,0 g Rohprodukt. Zur Reinigung wird suspendiert in 80 ml Methanol und 3 Stunden bei Rückflusstemperatur gerührt. Filtrieren (heiss), Waschen und Trocknen liefert braunrote Kristalle der Formel 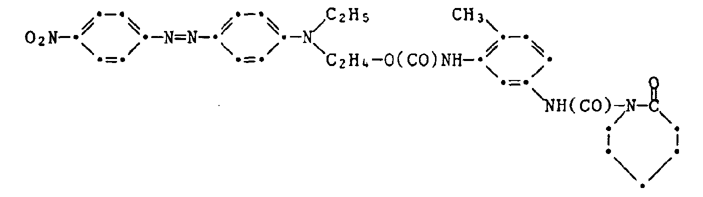 und vom Schmelzpunkt 169 - 173°C. Der Farbstoff färbt Polyestermaterial in scharlachroten Farbtönen.

Beispiel 14: Eine Toluollösung enthaltend 29 g des im Beispiel 13 beschriebenen Monoisocyanats wird bei Raumtemperatur zugetropft zu 14,1 g N-Ethyl-N-hydroxyethylanilin. Nach 6 Stunden Rühren bei 80°C wird das Lösungsmittel abdestilliert. Die zurückbleibende farblose Masse entspricht der Formel 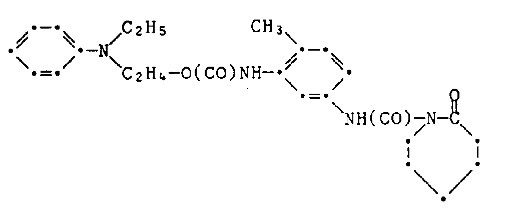 und kann ohne weitere Reinigung als Kupplungskomponente verwendet werden.

Beispiel 15: 3,3 g 2-Cyan-4-nitroanilin werden auf die übliche Weise in Schwefelsäure diazotiert und bei 0 - 10°C gekuppelt auf eine Vorlage, enthaltend 0,02 mol der Kupplungskomponente aus Beispiel 14, 50 ml Essigsäure und 50 g Eis. Nach Beendigung der Kupplung werden 300 ml Wasser zugegeben. Nach 1 Stunden Rühren bei Raumtemperatur wird abfiltriert und gewaschen. Zur Reinigung wird das Rohprodukt in 100 ml Methanol angeschlämmt und 2 Stunden bei Rückflusstemperatur gerührt. Danach wird heiss abfiltriert und mit Methanol gewaschen Nach dem Trocknen bei 40°C erhält man 10,2 g schwarzgrünes Pulver der Formel 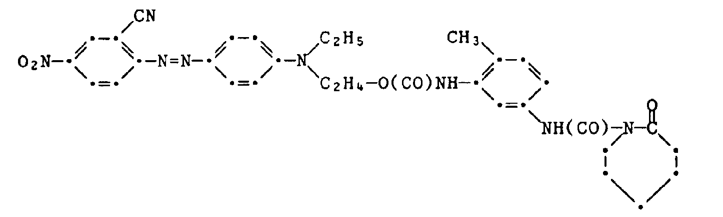 vom Schmelzpunkt 125 - 130°C. Der Farbstoff färbt Polyestermaterial in rubinroten Farbtönen.

Beispiel 16: Zu 8,9 g Toluylen-2,4-diisocyanat, 0,05 g Diazabicyclooctan und 10 ml Dimethylformamid werden bei 10°C 8,3 g N-Ethyl-N-hydroxyethylanilin langsam zugetropft Nach 6 Stunden Rühren bei Raumtemperatur werden 5,7 g ε-Caprolactam zugegeben. Es wird weitere 6 Stunden bei 80°C gerührt.

Das Reaktionsgemisch, welches das Zwischenprodukt der Formel 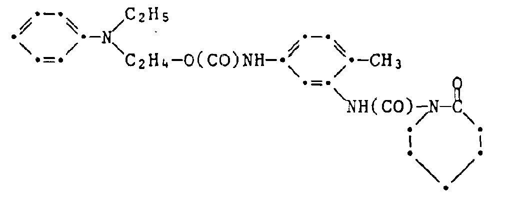 enthält, kann in dieser Form als Kupplungskomponente verwendet werden.

Beispiel 17: 4,6 g 2,4-Dinitroanilin werden auf die übliche Art in Schwefelsäure diazotiert und bei 0 - 10°C gekuppelt auf eine Vorlage, enthaltend ca. 7,2 g der im Beispiel 16 beschriebenen Kupplungskomponente in 100 ml 80 %iger Essigsäure. Nach Verdünnen der Reaktionsmasse mit 400 g Eiswasser wird filtriert, gewaschen und getrocknet. Man erhält 12,7 g schwarze Kristalle der Formel 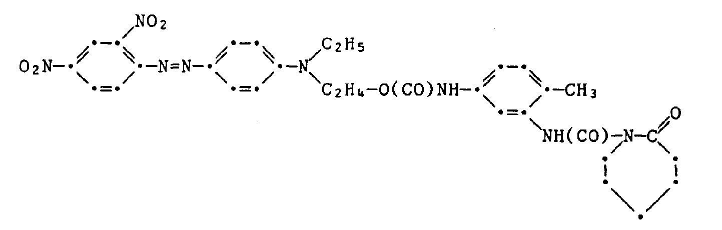 vom Schmelzpunkt 185 - 190°C.

Der Farbstoff färbt Polyestermaterial in rubinroten Farbtönen.

Beispiel 18: 6,3 g Farbstoff der Formel 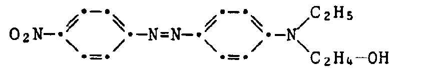 und 0,05 g Diazabicyclooctan werden in 50 ml wasserfreiem Dimethylformamid gelöst. Bei 10°C werden langsam 7,0 g Toluylen-2,4-diisocyanat zugetropft. Nach 8 Stunden Rühren bei Raumtemperatur werden 5,1 g Acetonoxim zugegeben. Nach Rühren über Nacht wird das Reaktionsgemisch ausgegossen auf 500 g Eiswasser Nach Filtrieren, Waschen und Trocknen erhält man 10,8 g oranges Pulver (Produktegemisch), dessen Dünnschichtchromatogramm auf Kieselgel mit Chloroform/Aceton 4:1 zwei Hauptzonen aufweist. Die vordere Zone mit einem R_{f}-Wert von 0,9 enthält die Substanz der Formel

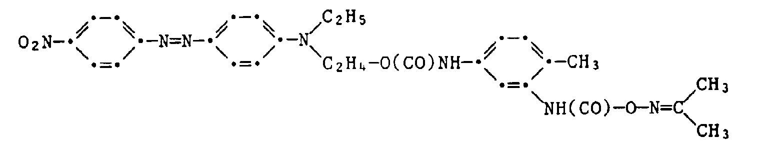

Das Rohprodukt zeigt ein Absorptionsmaximum bei 478 nm (in Ethanol) und färbt Polyestermaterial in scharlachroten Farbtönen.

Beispiel 19: Vom im Beispiel 18 beschriebenen Farbstoff wird eine 1-prozentige Lösung in N-Methylpyrrolidon hergestellt. Damit wird im HT-Verfahren eine bezüglich Farbstoff 0,5 prozentige Färbung von Polyester erstellt.

Die Thermomigrierechtheit (Prüfung analog Bsp. 10) erweist sich als sehr gut, wenn das gefärbte Gewebe vor der reduktiven Reinigung 1 Minute auf 230°C erhitzt wird.

Beispiel 20: 19,2 g p-Nitroanilin werden auf übliche Weise in Salzsäure diazotiert und bei 0 - 10°C gekuppelt auf eine Vorlage, enthaltend 34,6 g 3-(N-Cyanethyl-N-hydroxyethyl)amino-acetanilid in 300 ml Eisessig und 400 g Eiswasser. Mit 30 %iger Natronlauge wird bei 0 - 10°C auf pH 3 gestellt. Filtrieren, Waschen und Trocknen liefert 50,1 g g Rohprodukt. Zur Reinigung wird suspendiert in 300 ml Tertiärbutyl-methylether und 2 Stunden bei Rückflusstemperatur gerührt. Nach Filtrieren (heiss), Waschen und Trocknen erhält man 47,8 g dunkelrote Kristalle der Formel 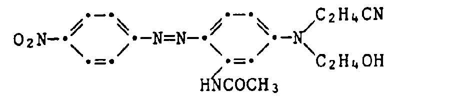 vom Schmelzpunkt 164 - 165°C.

1,6 g dieses Farbstoffs und 0,05 g Diazabicyclooctan werden gelöst in 20 ml wasserfreiem Dimethylformamid. Dazu werden bei Raumtemperatur langsam 0,9 g Toluylen-2,4-diisocyanat zugetropft. Nach 48 Stunden Rühren bei Raumtemperatur werden 1,4 g wasserfreies Caprolactam zugegeben.

Nach 12 Stunden Rühren bei 80°C wird auf 300 g Eiswasser ausgegossen. Filtrieren, Waschen und Trocknen liefert 2,5 g braunrotes Pulver (Produktegemisch) vom Schmelzpunkt 110 - 150°C. Das Dünnschichtchromatogramm auf Kieselgel mit Essigester zeigt zwei Hauptzonen mit R_{f}-Werten von 0,75 und 0,6.

Das Produktegemisch färbt Polyestermaterial in scharlachroten Farbtönen.

1,2 g des vorstehend beschriebenen Reaktionsgemisches werden mit Essigester chromatographiert an einer Säule von 230 g Kieselgel. Von der Fraktion mit R_{f} = 0,75 erhält man 0,2 g braunrotes Pulver der Formel 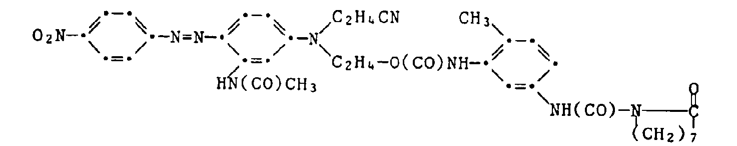 und vom Schmelzpunkt 85 - 105°C.

Der Farbstoff färbt Polyestermaterial in scharlachroten Farbtönen.

Beispiel 21: Von dem im Beispiel 20 beschriebenen Produktegemisch wird eine 5-prozentige wässrige Dispersion hergestellt (vgl. Beispiel 10). Davon wird im Thermosol-Verfahren eine Färbung von Polyester erstellt (10 g/l Farbstoff, Flottenaufnahme 50 %, Fixiertemperatur 230°C).

Man erhält eine sehr gut thermomigrierechte Färbung (Prüfung analog Bsp. 10).

Gleich gute Resultate erhält man, wenn anstelle des Produktegemisches aus Beispiel 20 die gereinigte Farbstoff-Fraktion aus diesem Beispiel eingesetzt wird.

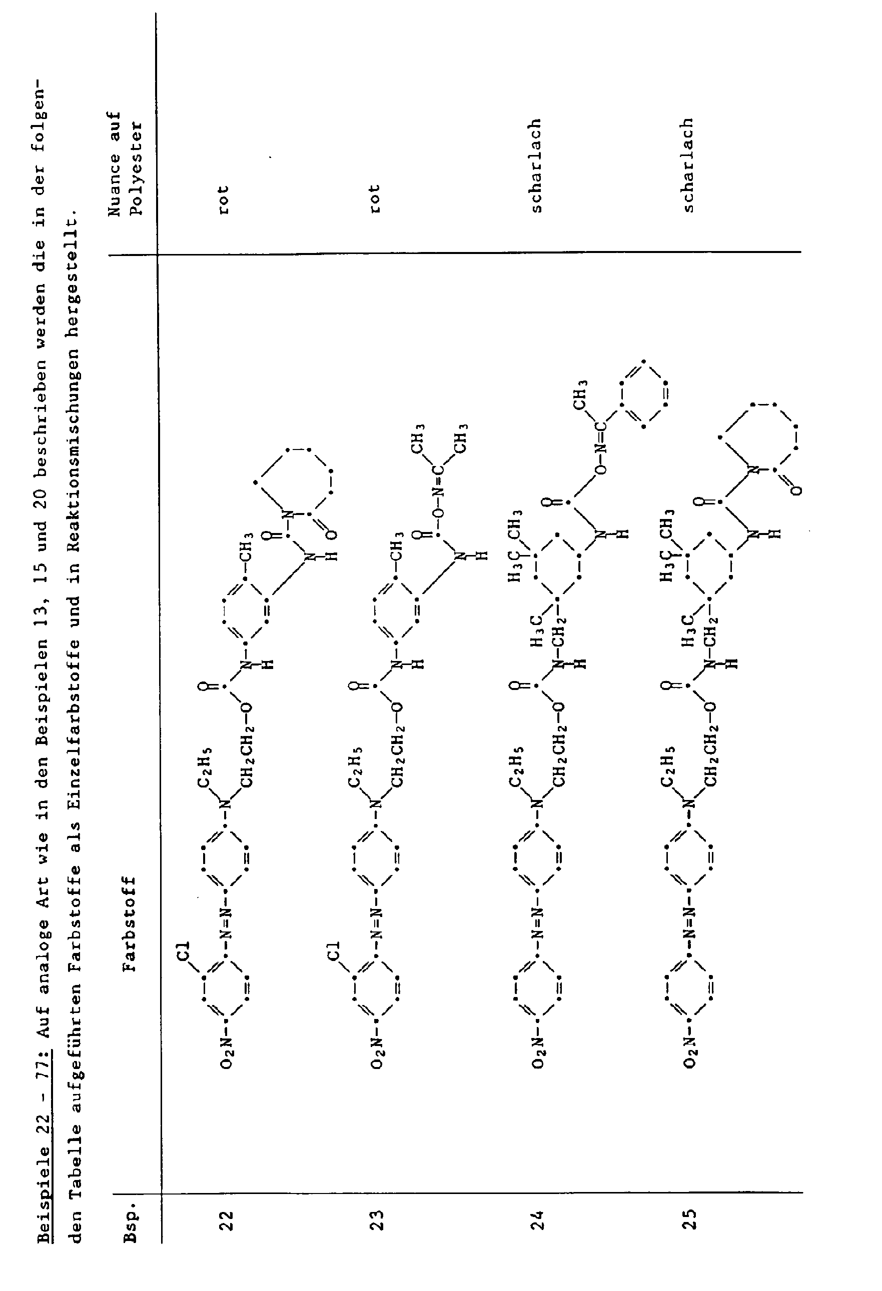

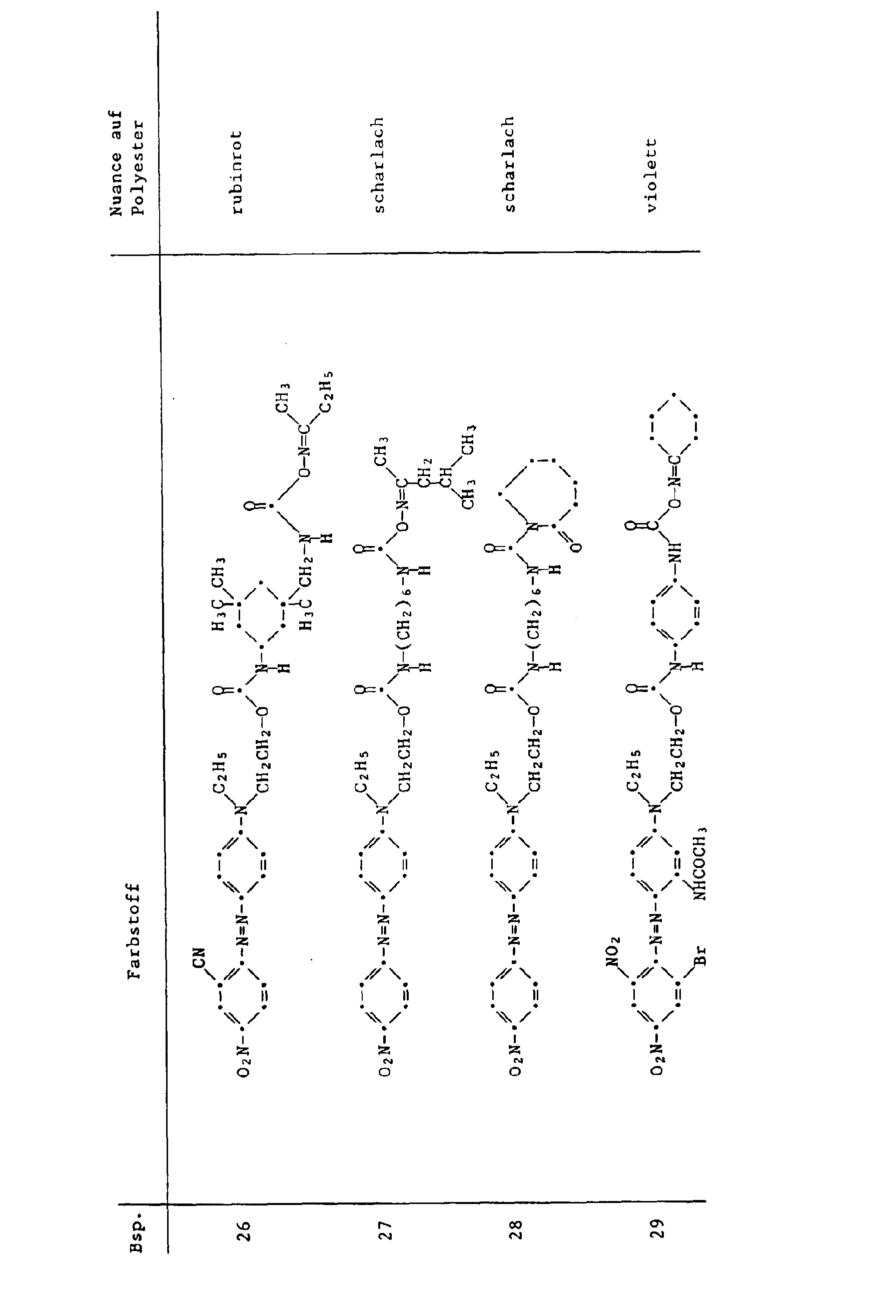

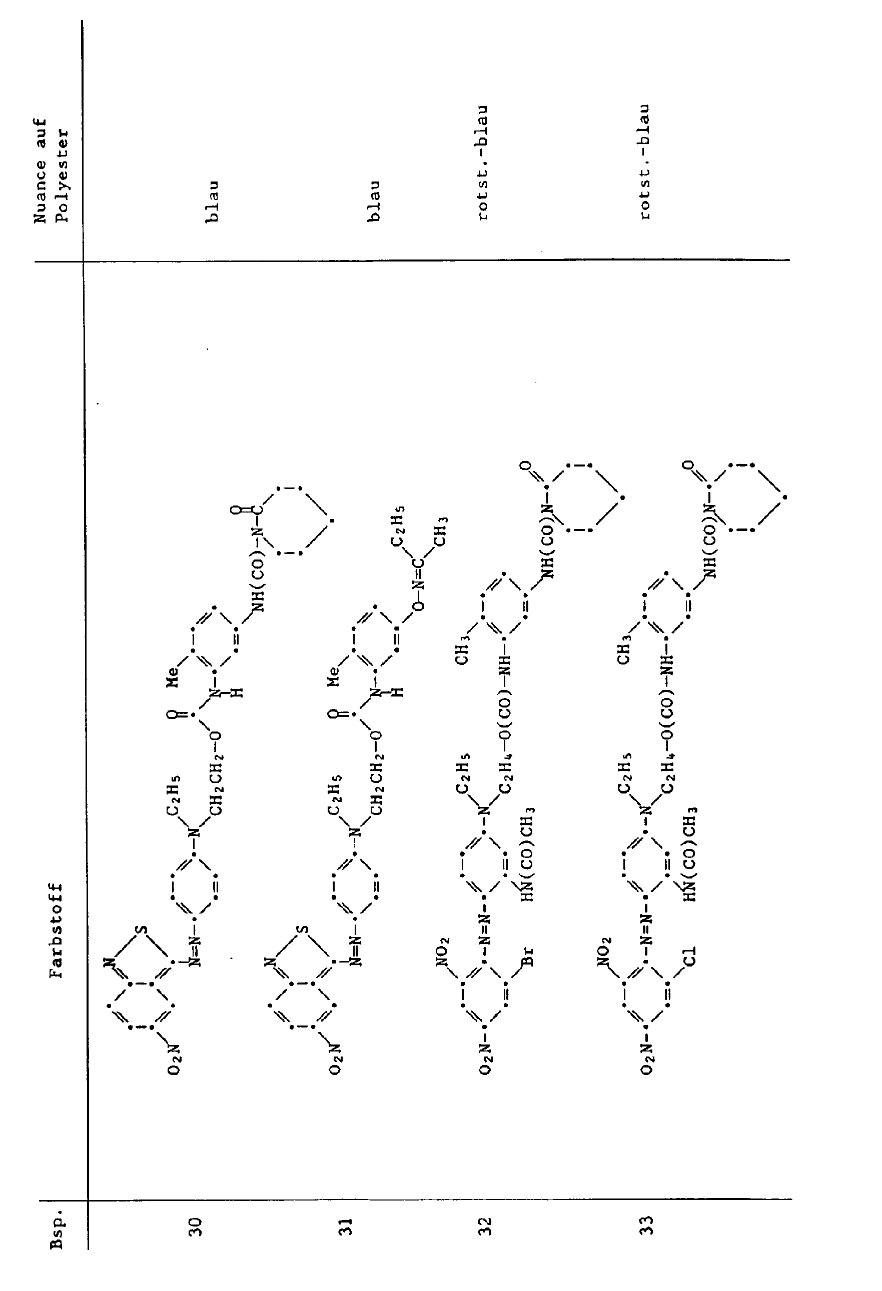

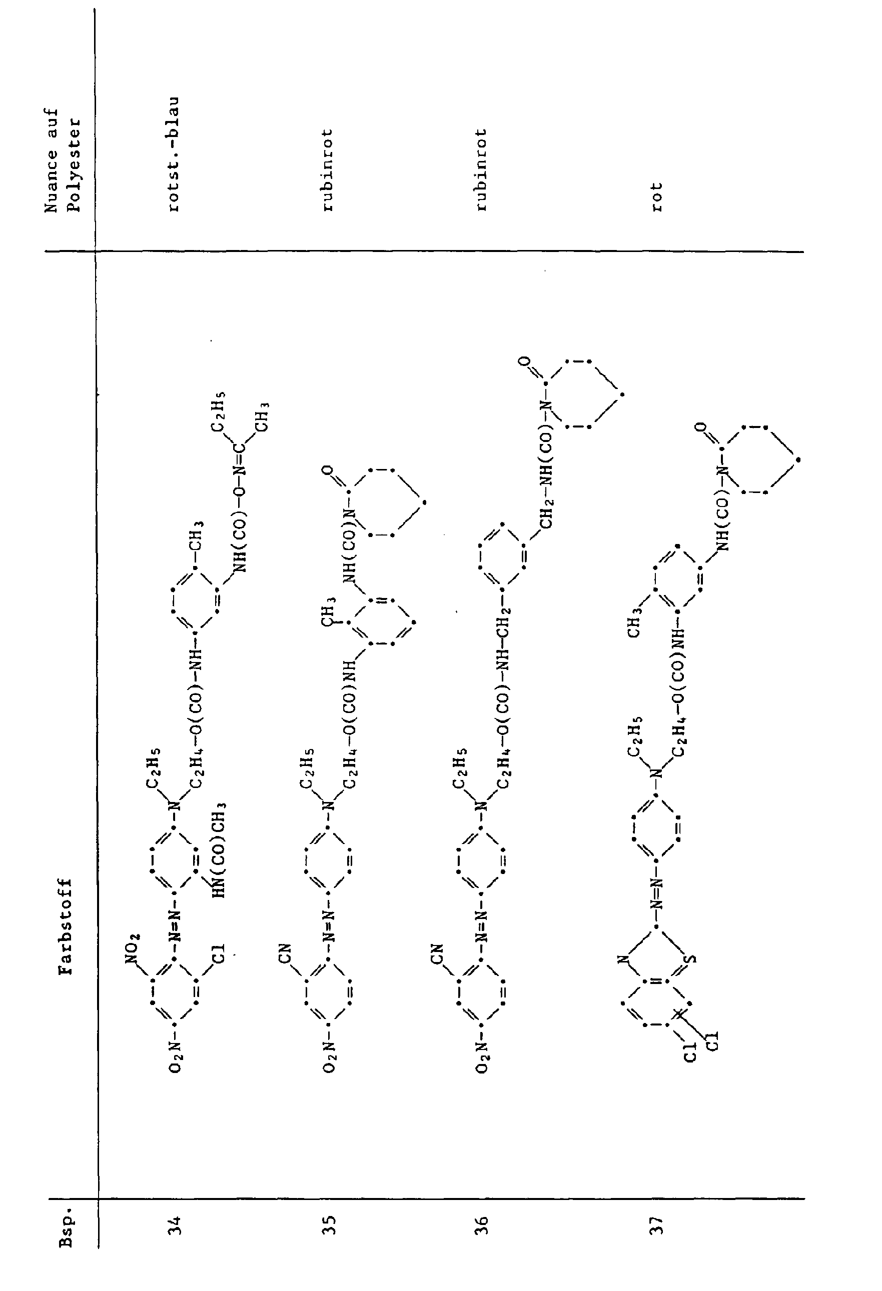

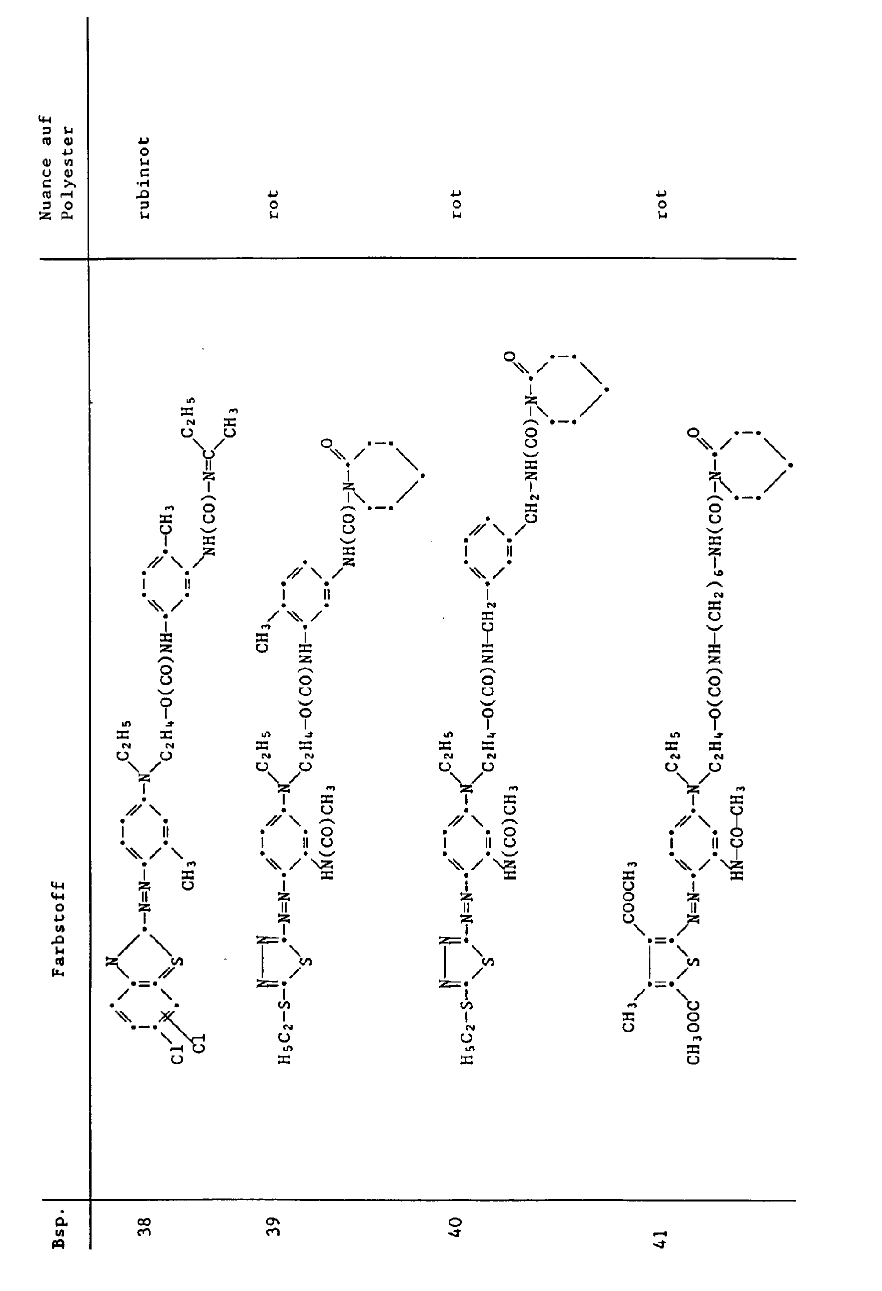

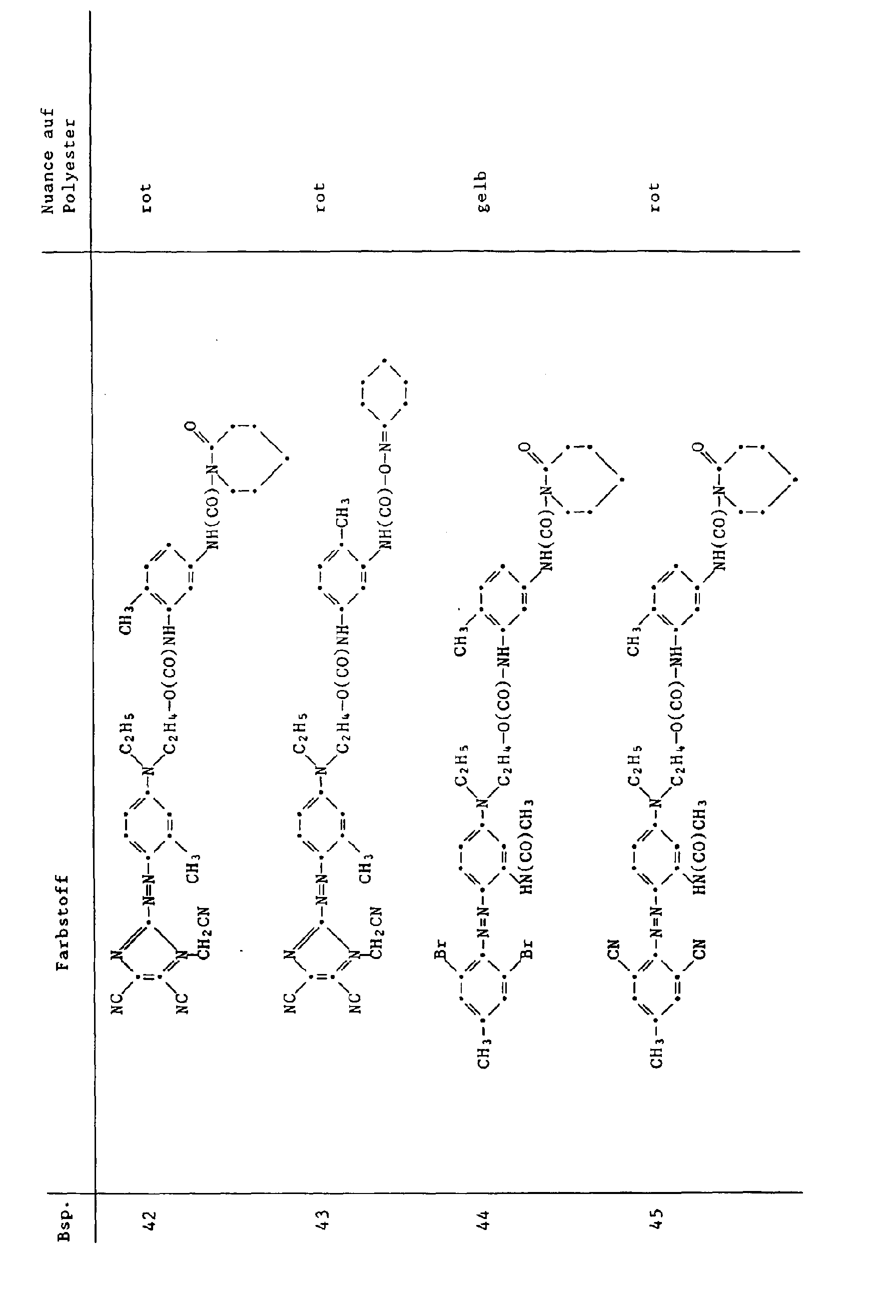

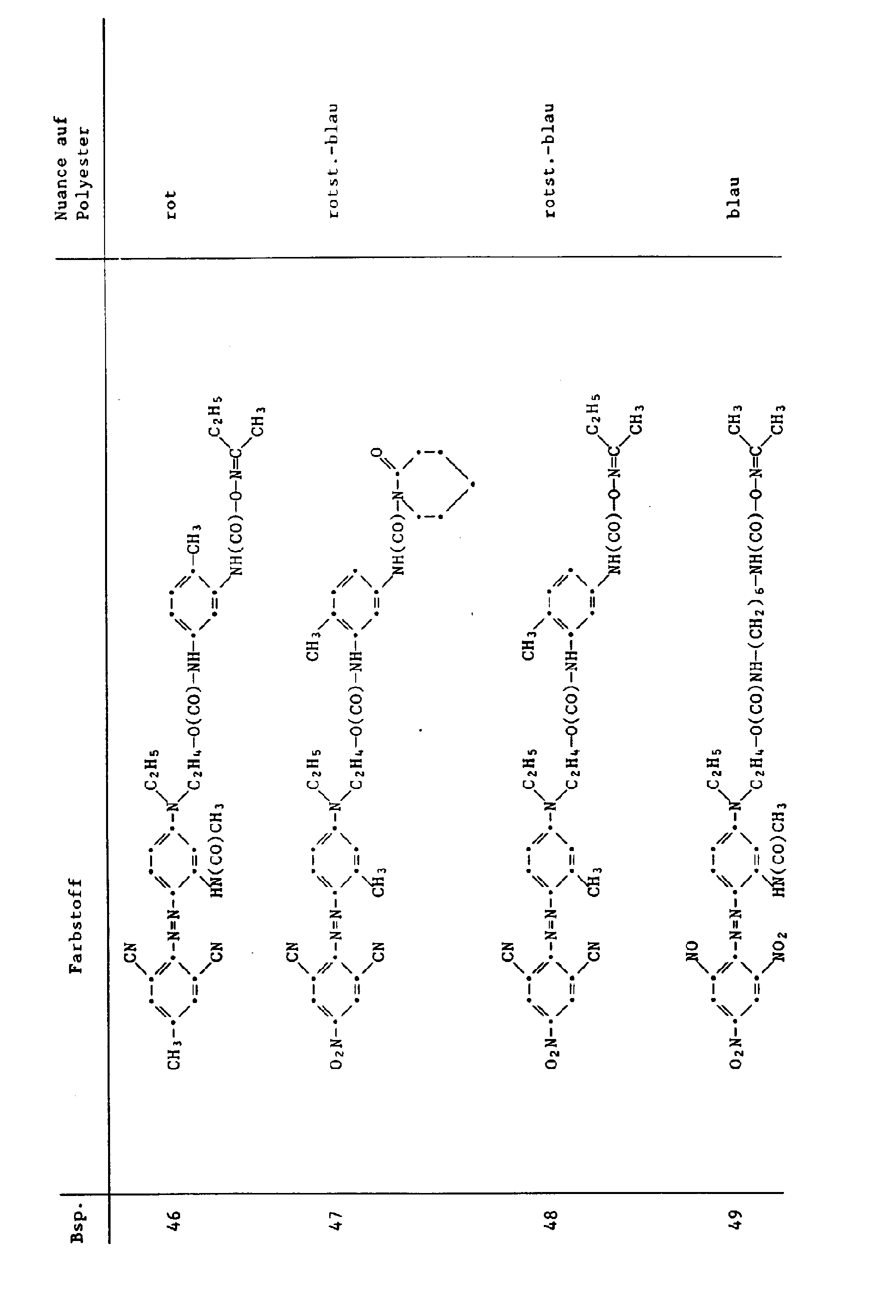

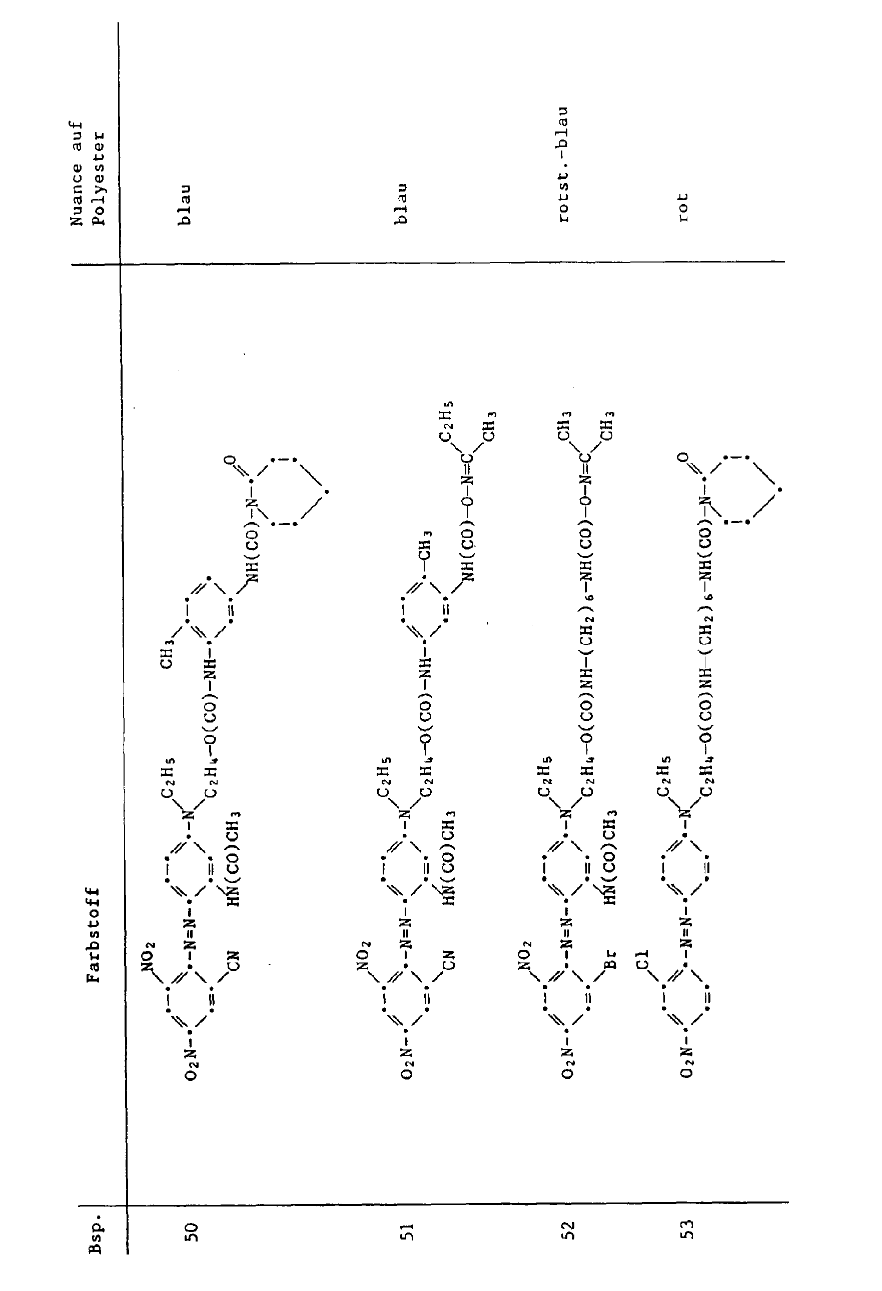

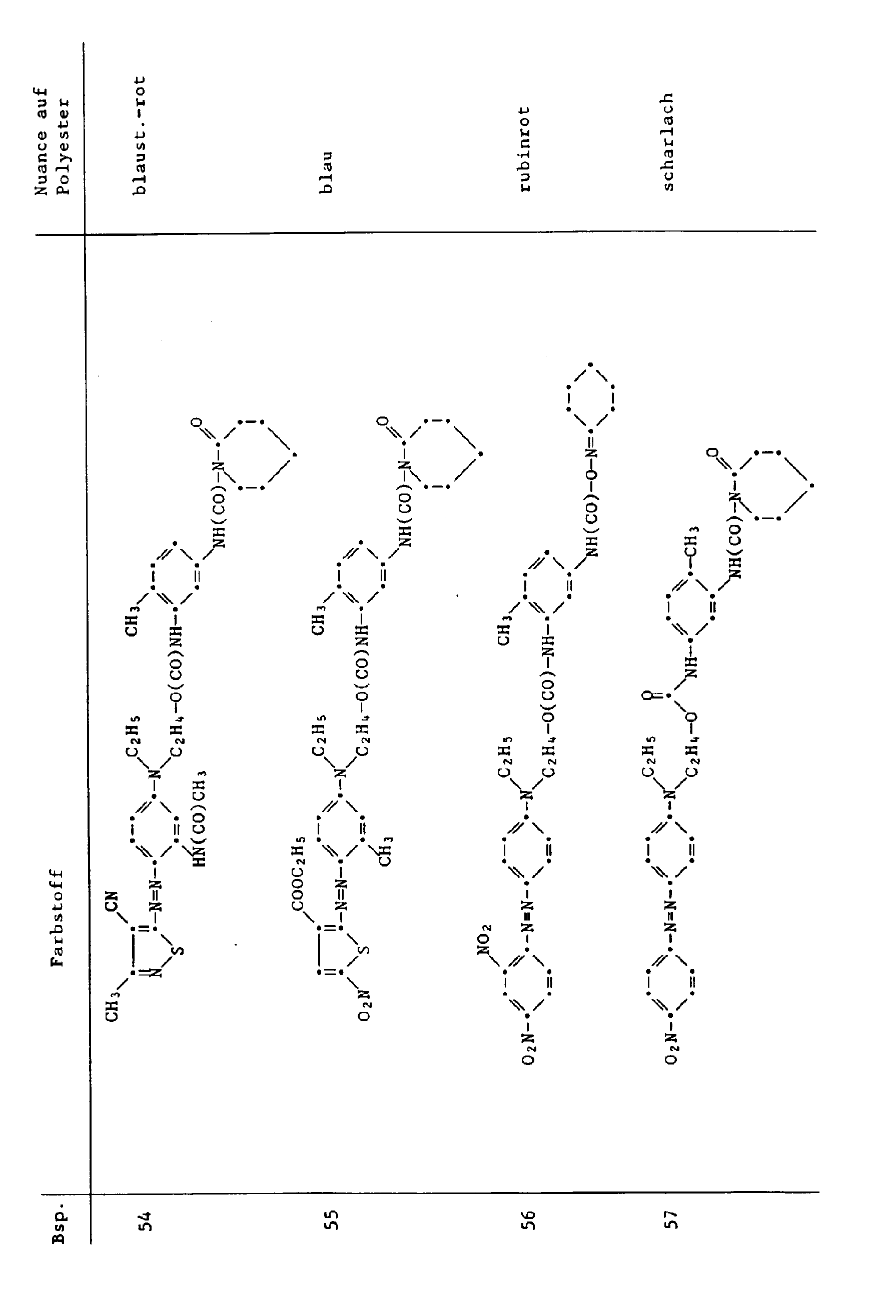

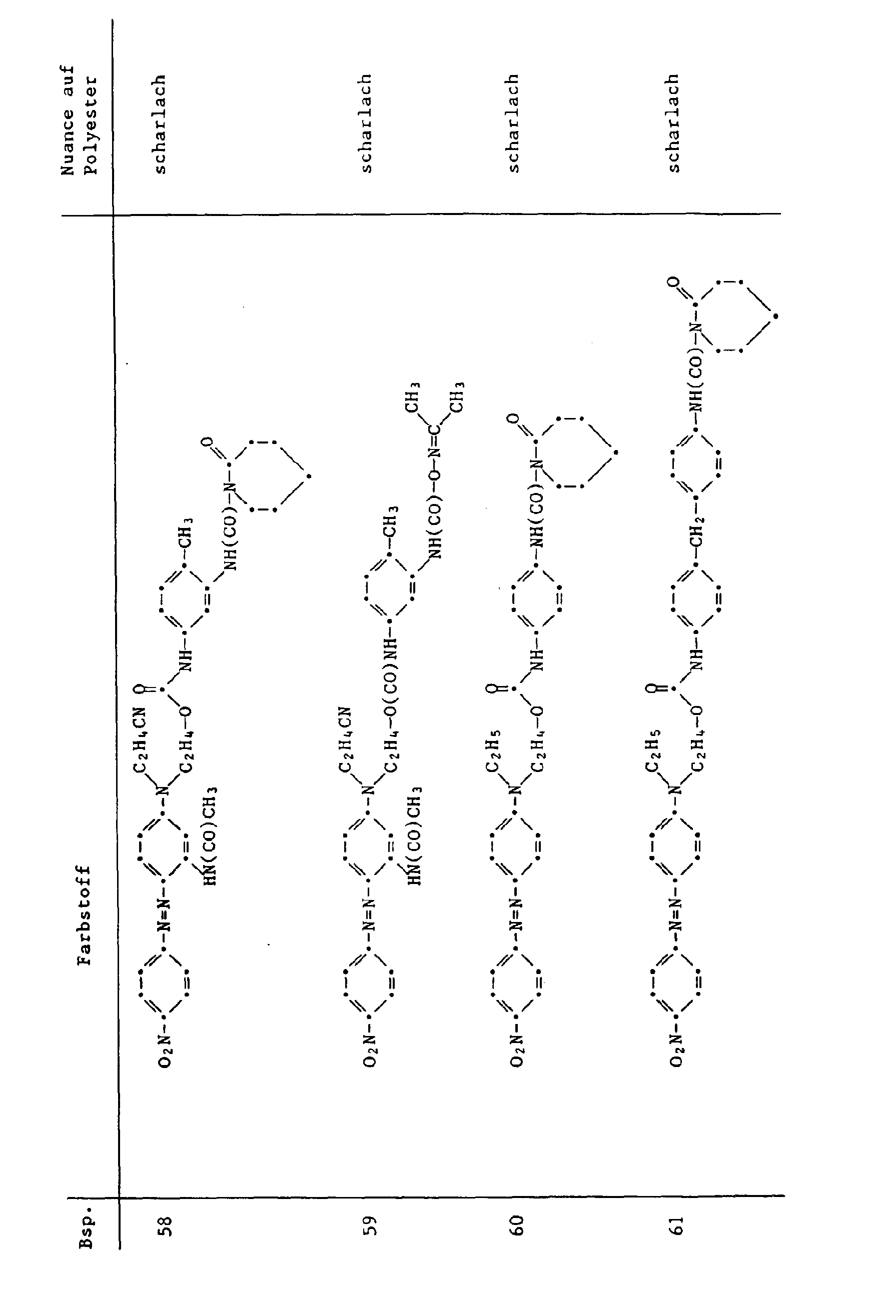

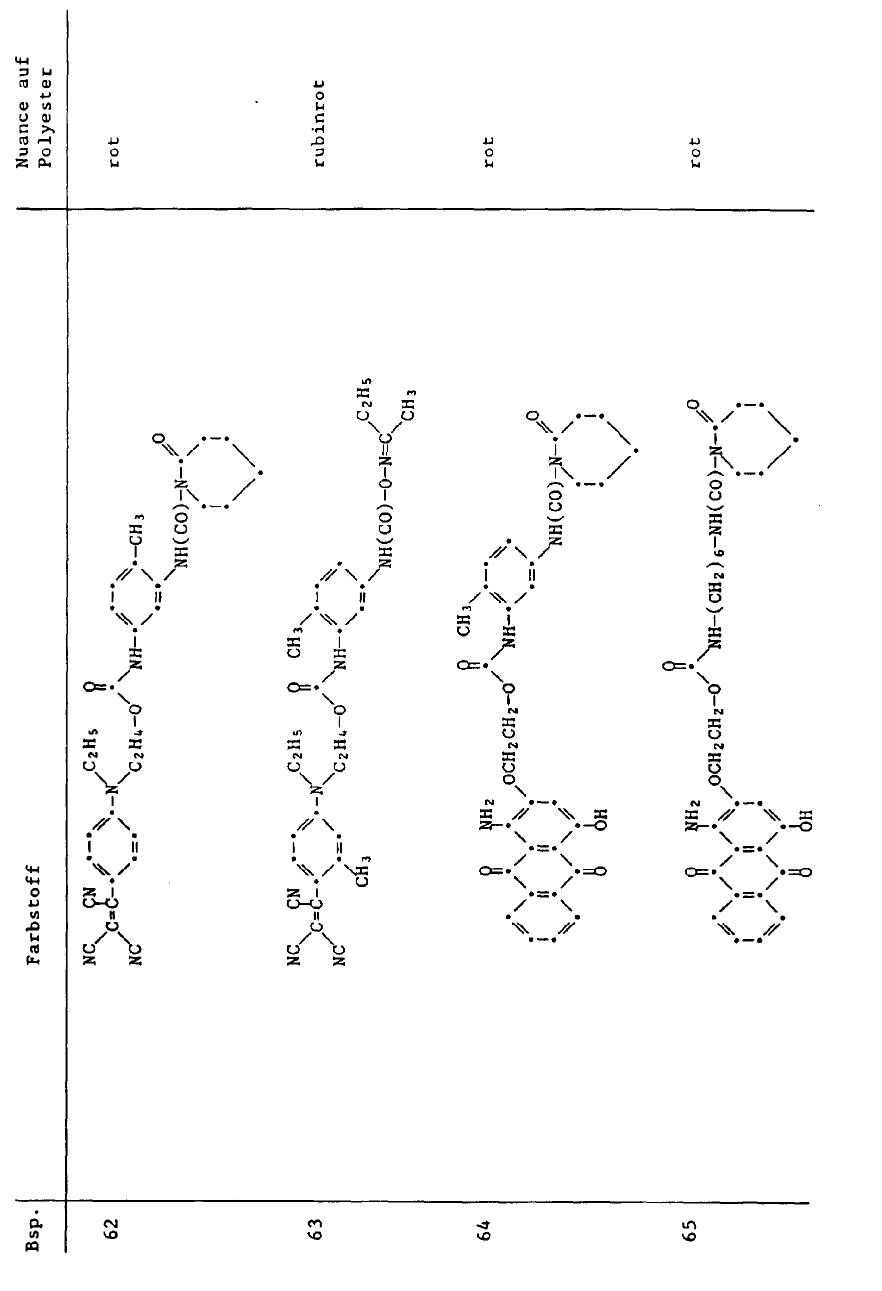

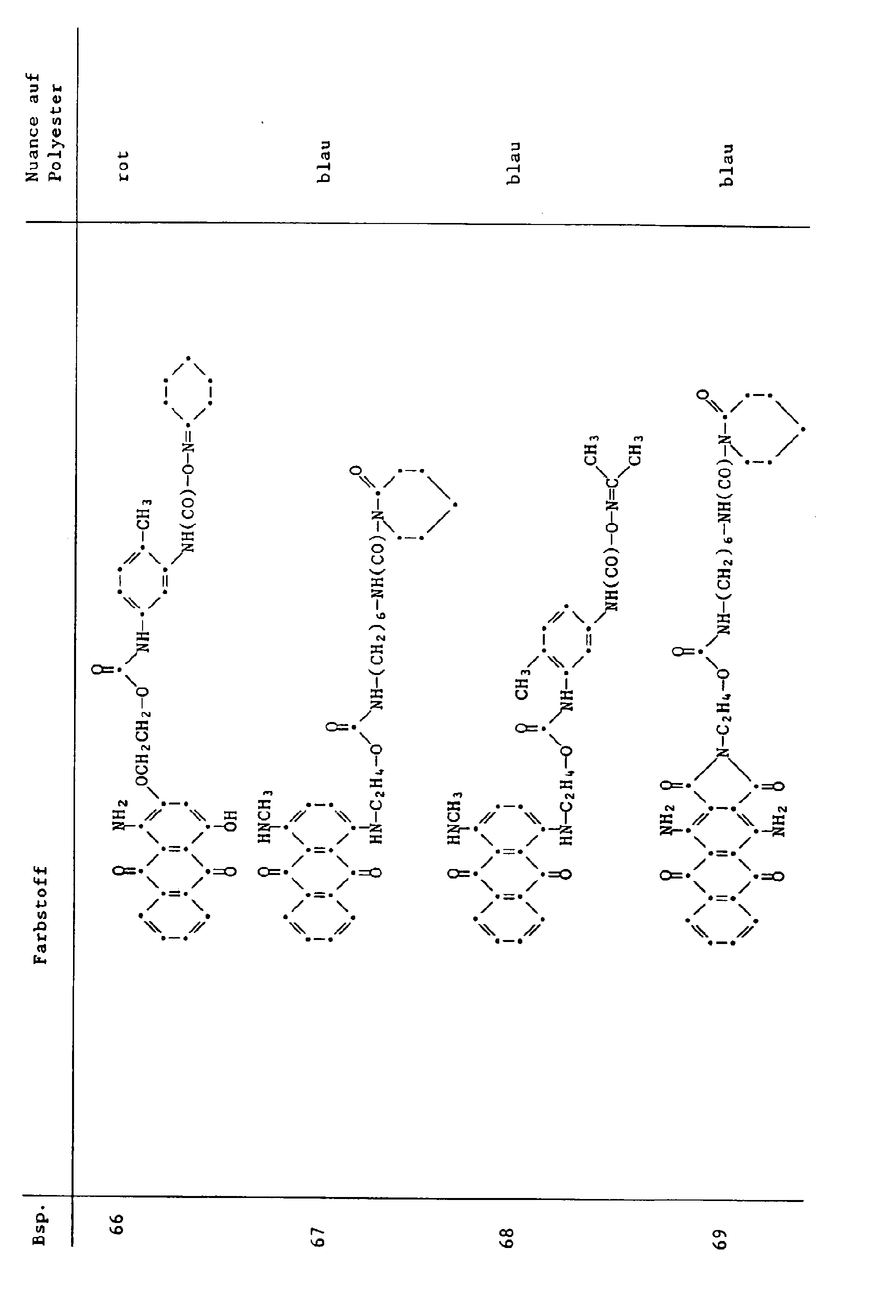

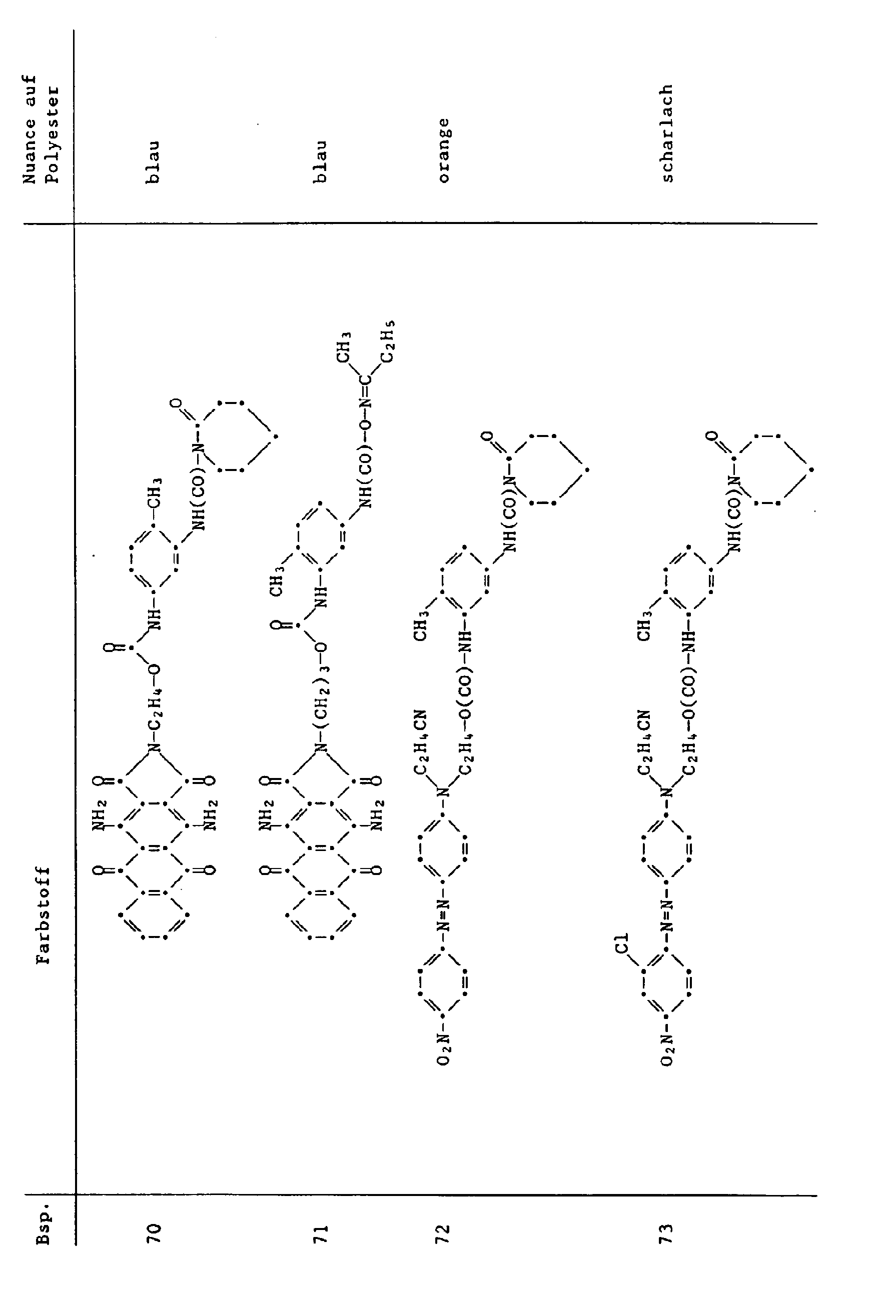

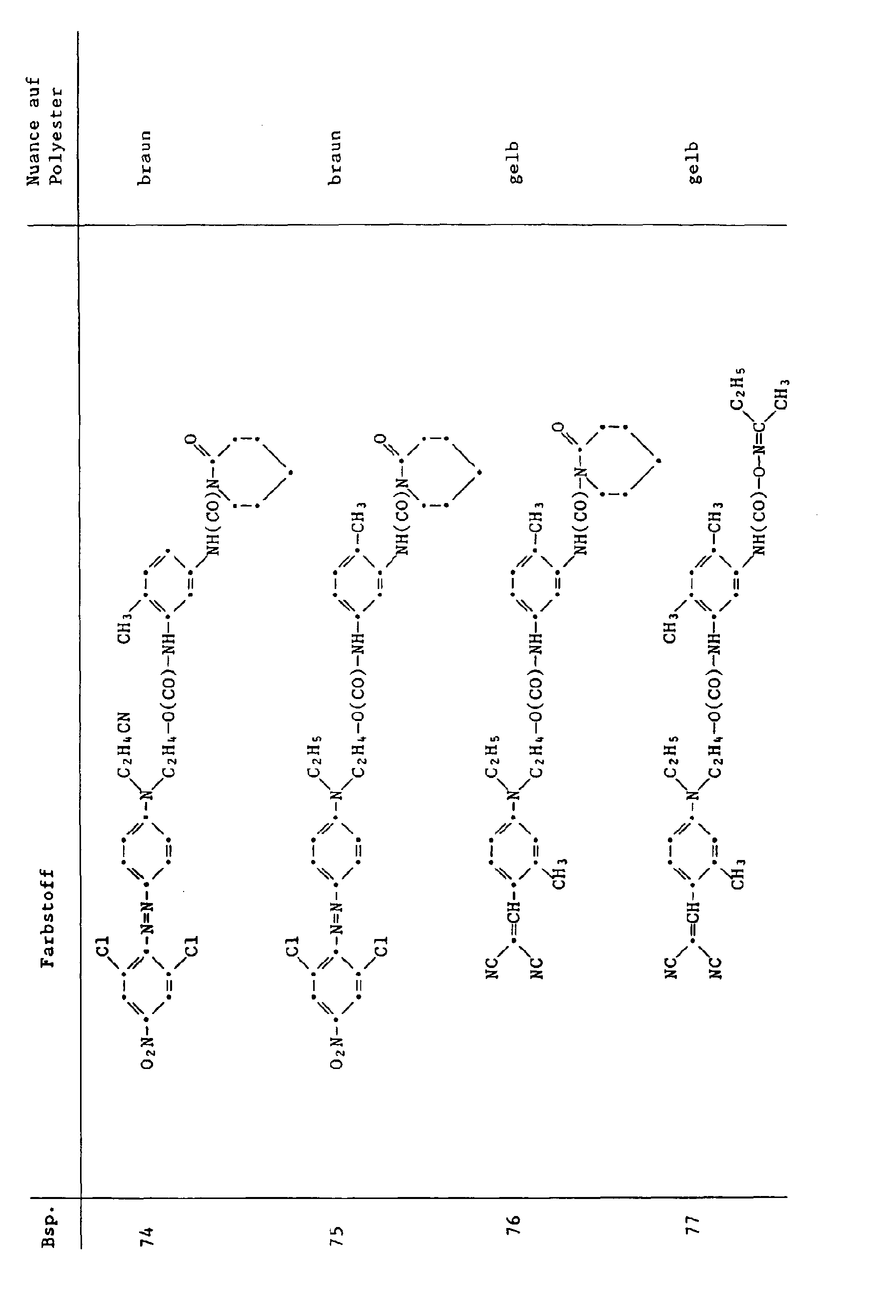

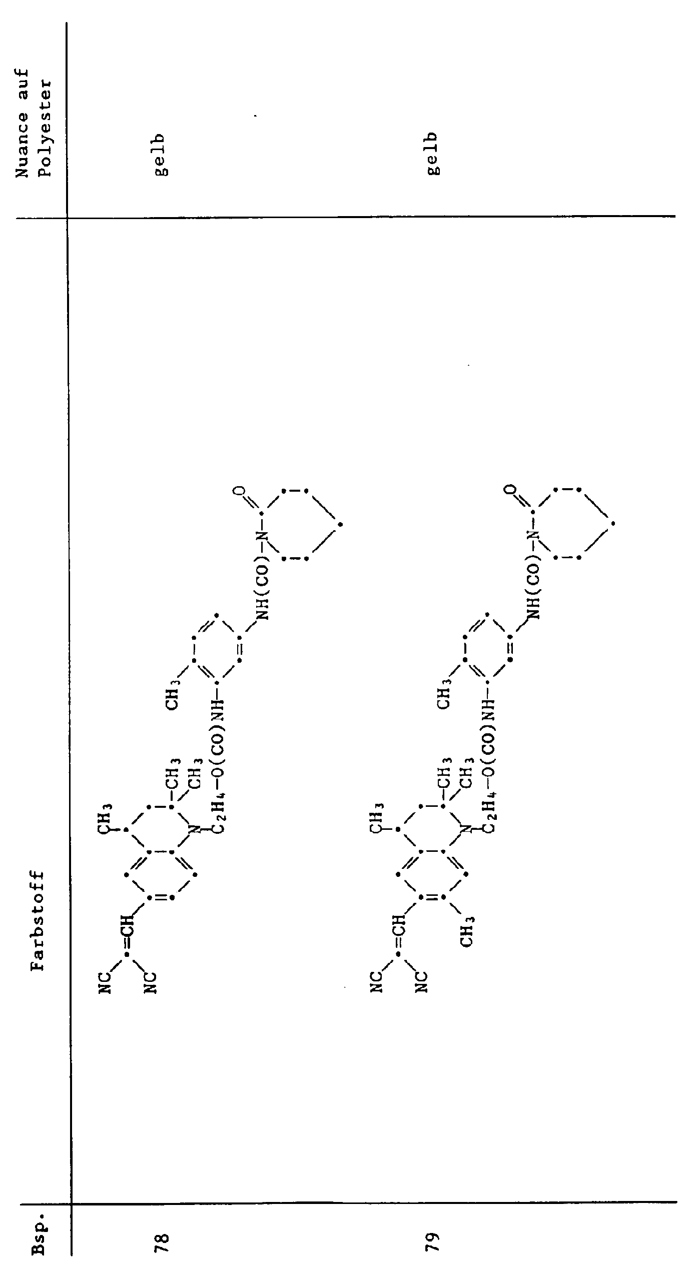

Beispiele 80 - 82: 70,5 g N-Cyanethyl-N-ethylanilin vom Gehalt 98,7 % werden in der im Beispiel 5 beschriebenen Weise in wasserfreiem Ethanol in Gegenwart von Ammoniak mit Raneynickel hydriert. Nach 2,5 Stunden kommt die Wasserstoffaufnahme zum Stillstand. Das nach dem Abfiltrieren des Katalysators und Einengen der Lösung erhaltene rohe ölige Amin der Formel (64 g = 90 % d.Th.). 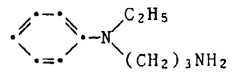 wird ohne Reinigung weiterverwendet. Man erhält daraus durch Kupplung mit der äquivalenten Menge aus 2-Chlor-4-nitroanilin hergestellter Diazoniumsalzlösung in der in Beispiel 5 beschriebenen Weise den rohen Aminofarbstoff der Formel 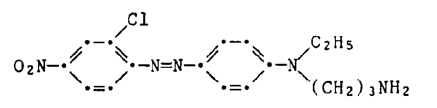 als braunes Pulver vom Schmelzpunkt 205 - 208°C.

Durch Umsetzung dieses Aminopropylfarbstoffes mit den Chlorameisensäureestern der in Spalte 2 der folgenden Tabelle aufgeführten Ketoxime werden die folgenden roten Farbstoffe erhalten:

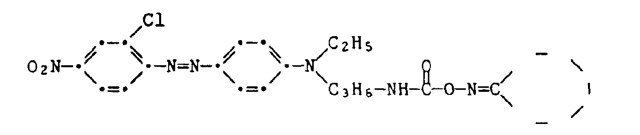

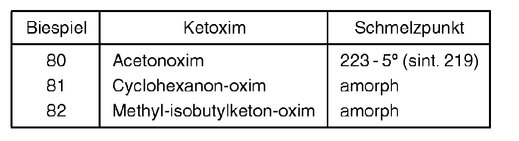

Beispiel 83: 21,3 g 2-Brom-3-cyan-4,6-diaminopyridin und 60 g 1,2-Diaminoethan werden in einem Autoklaven 2 Stunden bei 125° und 2 bar gerührt und das überschüssige Diaminoethan im Vakuum abgedampft. Man erhält 32,8 g rohen Eindampfrückstand, der neben Diaminoethanhydrobromid die Verbindung der Formel 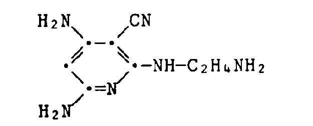 teilweise als Hydrobromid enthält. 4 g des Rückstandes werden mit einer aus 1,83 g 2,4-Dinitroanilin hergestellten Diazoniumsalzlösung in 80 ml 80-prozentiger Essigsäure während 2 Stunden bei 0 - 5° gekuppelt, der Farbstoff abgenutscht, in Wasser aufgeschlämmt und mit Natriumhydroxid auf pH 11 - 12 gestellt, abgenutscht, mit Wasser gewaschen und getrocknet. Man erhält 3,0 g des roten Aminoethylfarbstoffs der Formel 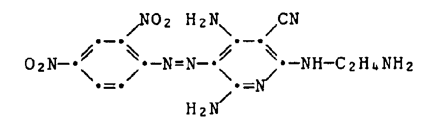 der bis 230°C nicht schmilzt.

1,5 g (- 0,004 Mol) davon werden in 20 ml Dichlormethan suspendiert und mit 0,6 ml (0,0044 Mol) Triethylamin versetzt. Dann tropft man innerhalb von ca. 20 Minuten 0,0088 Mol Chlorameisensäureester von Acetonoxim zu und lässt 1,5 Stunden bei Raumtemperatur rühren. Man filtriert nicht umgesetzten Aminoethylfarbstoff ab, vernichtet den Chlorameisensäureester mit wenig Ammoniak und dampft im Vakuum ein.

Chromatographie an Kieselgel mit Ethylacetat als Eluiermittel liefert den roten Farbstoff der Formel 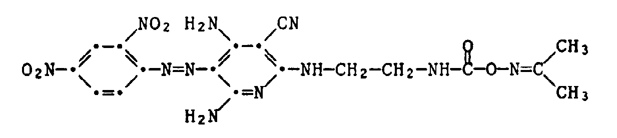 als braunes Pulver vom Schmelzpunkt 205 - 206°.

Beispiele 84-98: Auf analoge Art wie in den Beispielen 5 bis 9 und 80 bis 83 beschrieben werden die in der folgenden Tabelle beschriebenen Farbstoffe hergestellt.

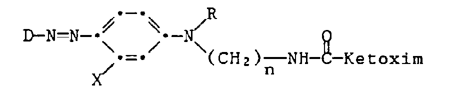

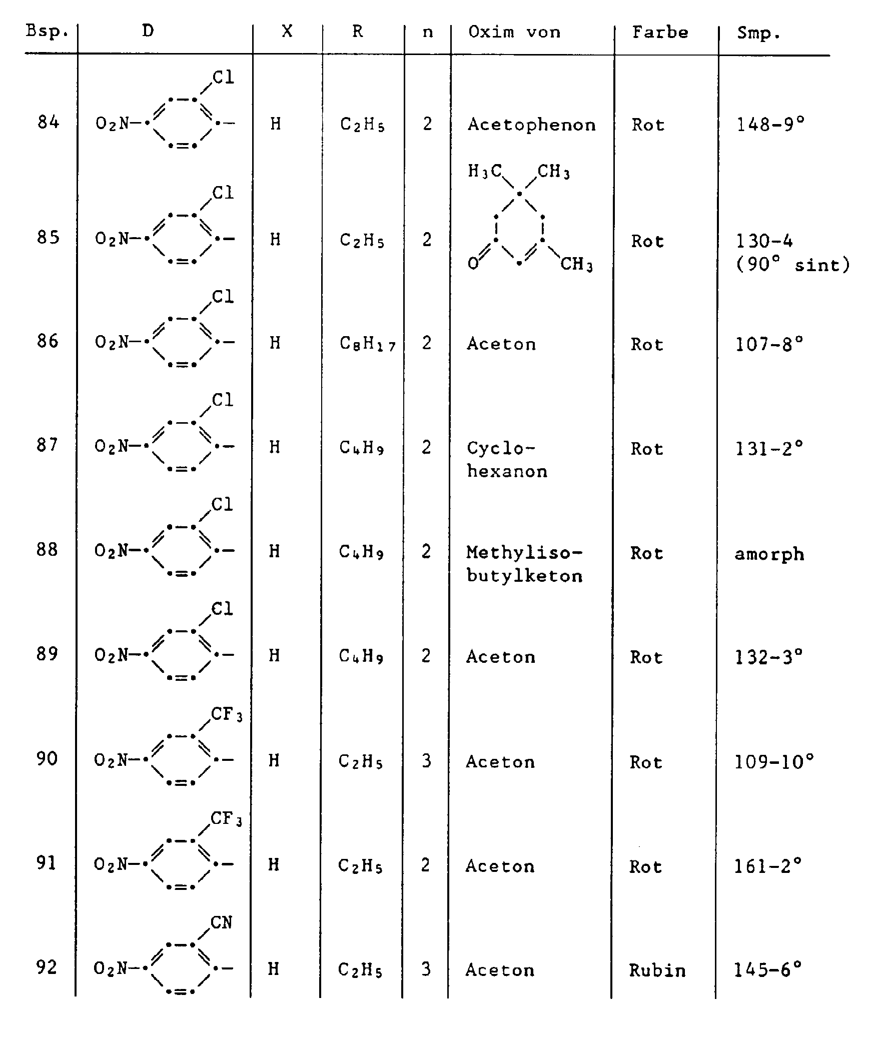

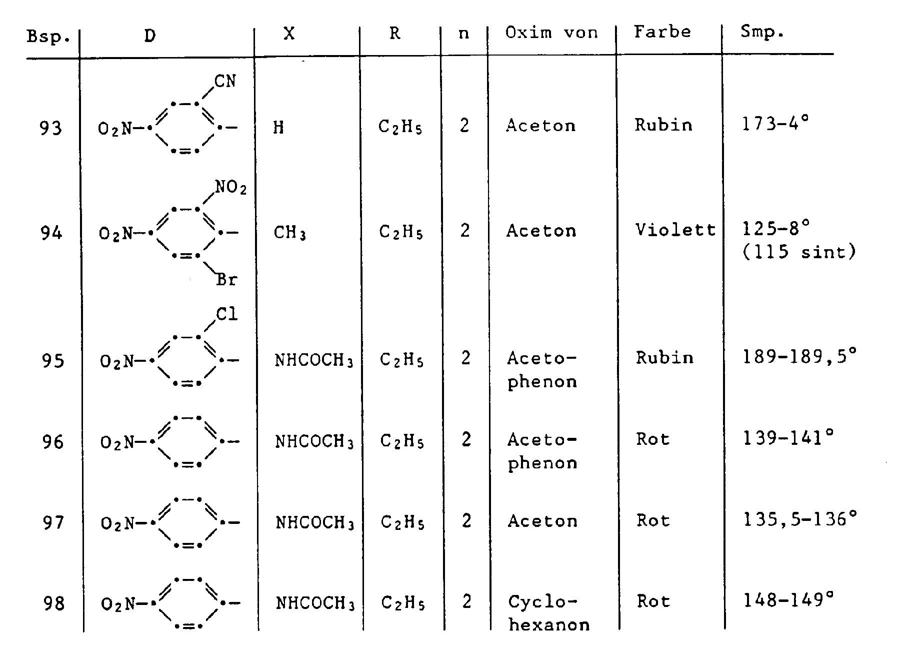

Beispiel 99: 86 g der Kupplungskomponente 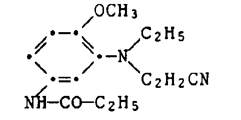 mit einem Gehalt von 85 % werden in 280 ml Ethanol und 87 g flüssigem Ammoniak mit Hilfe von 12,6 g Raneynickel bei 150 bar innerhalb von 51/2 Stunden bei 70 -75°C hydriert. Nach dem Abfiltrieren und Eindampfen der Hydrierlösung erhält man 85 g einer gelbbraunen, viskosen Masse. Diese wird in ca. 500 ml 1 N Salzsäure gelöst, die Lösung mit Natriumhydroxid auf pH 6,5 bis 7 gestellt und durch Ausschütteln mit Ethylacetat von unverändetem Edukt befreit.

Dann wird mit Natriumhydroxid auf pH 11,5 gestellt und mit n-Butanol ausgeschüttelt. Nach dem Waschen, Trocknen und Eindampfen der Butanolphasen erhält man 54 g der Aminopropylverbindung der Formel 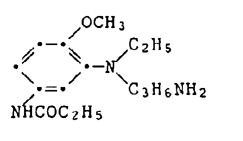 als braune, amorphe Masse.

6,2 g (0,022 Mol) davon werden in 20 ml Ethanol gelöst und bei 0 - 5°C in 200 ml 1 N Schwefelsäure eingetragen. Dazu lässt man die aus 0,02 Mol 2-Brom-4,6-dinitroanilin hergestellte Diazoniumsalzlösung bei 0 - 5°C innerhalb von 30 Minuten zutropfen und hält dabei den pH-Wert durch Zugabe von Natriumhydroxid zwischen 2 und 3. Anschliessend stellt man auf pH 11,5 und nutscht, wäscht und trocknet den Aminofarbstoff der Formel 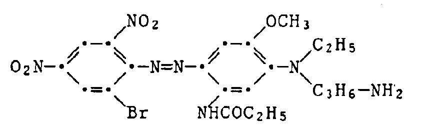 der bei ca. 140°C schmilzt.

Durch Umsetzung mit überschüssigem Chlorameisensäureester von Acetonoxim in trockenem Pyridin erhält man daraus den Farbstoff der Formel 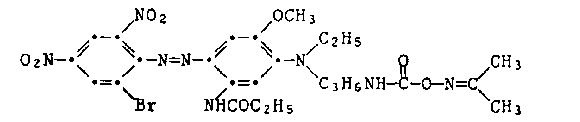 vom Schmelzpunkt 100 - 102°C.

Dieser färbt Textilmaterial aus Polyester in blauen Tönen.

Beispiel 100: 0,45 g des Bromdinitrofarbstoffes aus Beispiel 99 werden in 15 ml Dimethylformamid mit 0,07 g Kupferacetatmonohydrat und 0,07 g Natriumnitrit während 3 Stunden bei Raumtemperatur gerührt Dann wird mit viel Wasser verdünnt, der Niederschlag genutscht, gewaschen und getrocknet. Man erhält 0,33 g des Farbstoffes der Formel 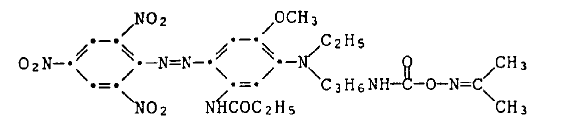 als braunes Pulver vom Schmelzpunkt 150 - 155°C, welcher Textilmaterial aus Polyester in grünstichig blauen Tönen färbt.

Beispiel 101: 13 g 3-Ethylaminoacetanilid und 9,5 ml Chloracetonitril werden mit 7,5 g Natriumcarbonatpulver während 3 Stunden bei 70 - 80°C gerührt, die Mischung in 80 ml Aceton aufgenommen, heiss von anorganischem Material abfiltriert und aus dem Filtrat durch Fällen mit Wasser, Abfiltrieren und Trocknen 14,8 g der Cyanmethylverbindung der Formel 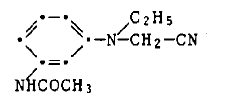 in Form eines hellbraunen Pulvers vom Schmelzpunkt 119 - 120°C isoliert.

Durch Hydrieren in 100 ml Tetrahydrofuran mit Rhodium auf Aluminiumoxid bei 35°C und 3 bar während 20 Stunden erhält man nach üblicher Aufarbeitung 10,3 g der Kupplungskomponente der Formel 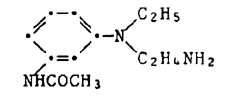 als rotbraune, amorphe Masse. Diese wird in 200 ml Eisessig gelöst und mit der Diazoniumsalzlösung der aequivalenten Menge 2-Brom-4,6-dinitroanilin bei 0 bis 10°C unter Einhaltung eines pH-Wertes von 2 bis 3 gekuppelt. Nach dem Abnutschen, Aufschlämmen in Wasser, Freisetzen der Base mit Natriumhydroxidlösung, erneutem Abnutschen, Waschen und Trocknen erhält man 9,2 g des Farbstoffes der Formel

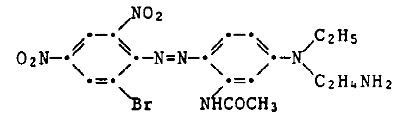

Umsetzung mit dem Chlorameisensäureester von Cyclohexanonoxim und säulenchromatographische Reinigung an Kieselgel mit Ethylacetat als Eluiermittel liefert den Farbstoff der Formel 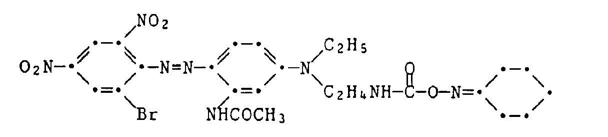 vom Schmelzpunkt 98 - 99°C (94 sint.).

Dieser färbt Textilmaterial aus Polyester in violetten Tönen.

Beispiel 102: 11,3 g wasserfreies e-Caprolactam und 0,05 g Diazabicyclooctan werden zusammen auf 80°C erwärmt. Zur Schmelze werden bei gutem Rühren innert 30 Minuten 10,5 g Chlorethylisocyanat zugetropft. Das Gemisch wird 7 Stunden bei 80°C gerührt. Nach Abkühlen auf Raumtemperatur wird die farbloses Schmelze weitere 3 Stunden unter leichtem Vakuum gerührt. Ueber Nacht bei 5°C erhält man 21,4 g farblose, leicht klebrige Kristalle der Formel 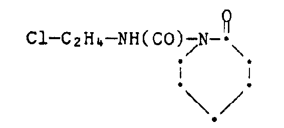 und vom Schmelzpunkt 34 - 37°C.

Die so erhaltenen Kristalle werden versetzt mit 10,9 g N-Ethylanilin, 0,8 g Kaliumiodid und 10,6 g Natriumcarbonat. Das Gemisch wird über Nacht bei 100°C gerührt. Nach Zugabe von 2 g Bernsteinsäureanhydrid wird eine weitere Stunde bei 80°C gerührt. Durch Extrahieren mit Methylenchlorid erhält man 24 g Kupplungskomponente der Formel 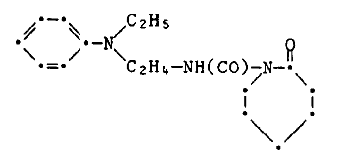 als braunes Oel. Das Reaktionsgemisch kann aber auch ohne Extraktion direkt in 200 ml 80 % Essigsäure aufgenommen und zum Kuppeln eingesetzt werden.

Beispiele 103 - 107: Auf analoge Art wie in Beispiel 102 beschrieben werden auch die folgenden Kupplungskomponenten erhalten.

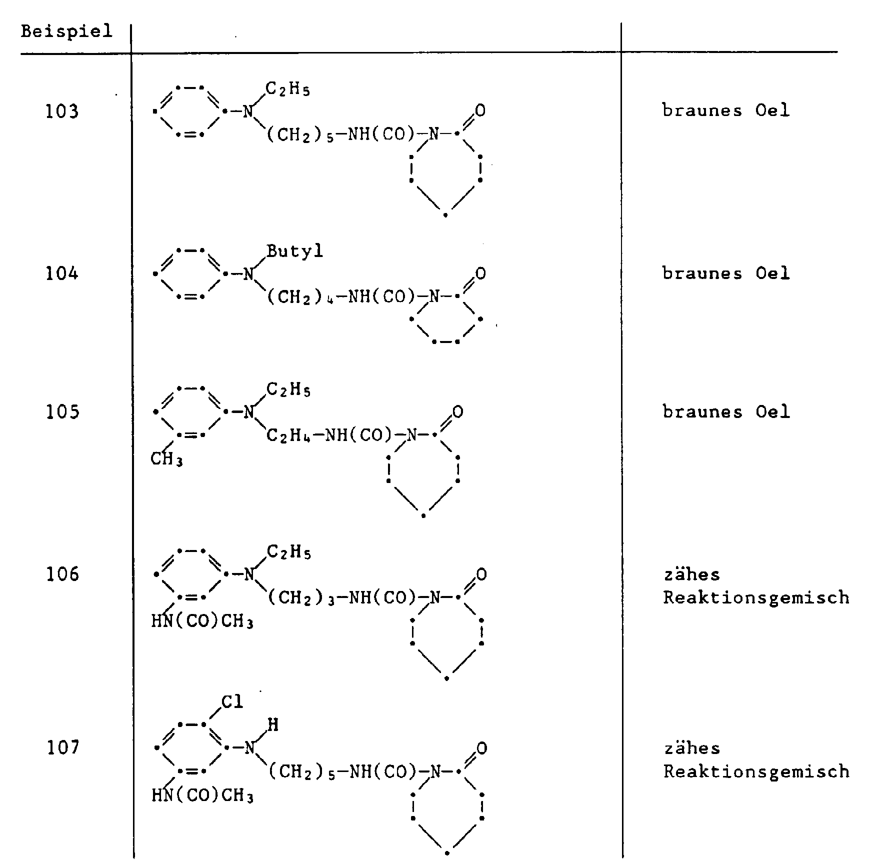

Beispiel 108: 8,6 g 2-Chlor-4-nitroanilin werden in 95 g 7 %iger Nitrosylschwefelsäure gelöst und 2 Stunden bei Raumtemperatur gerührt. Danach wird die Lösung auf eine Vorlage von 200 g Eis und 2,5 g Harnstoff ausgegossen. Die gelbliche Diazoniumsalzlösung wird innert 5 Minuten bei 0 - 5°C zugetropft zu einer Vorlage von 15,2 g der Kupplungskomponente aus Beispiel 102, 130 g 80 %iger Essigsäure und 200 g Eiswasser. Durch Zugabe von 30 %iger Natronlauge und 600 g Eis wird bei 0 - 10°C auf pH 3 eingestellt. Nach einstündigem Rühren wird abfiltriert, gewaschen und an der Luft getrocknet. Man erhält 23,2 g Rohprodukt. Zur Reinigung wird das Rohprodukt in Aceton angeschlämmt. Nach 3 Stunden Rühren bei Rückflusstemperatur wird die Suspension über Nacht bei Raumtemperatur gerührt und dann abfiltriert. Man erhält 15,3 g schwarzgrüne Kristalle der Formel 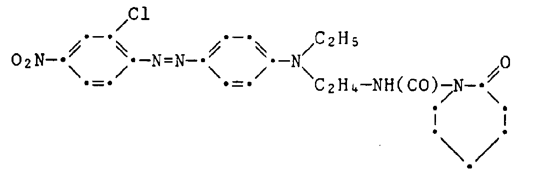 und vom Schmelzpunkt 132 - 134°C. Der Farbstoff färbt Polyestermaterial in roten Farbtönen.

Beispiel 109: 0,11 g Caprolactam in 10 ml Toluol werden mit 0,15 g Hexachlor-dimethylcarbonat in Gegenwart von 0,16 ml N,N-Diethylanilin während 4 Stunden bei Rückflusstemperatur gerührt. Dann wird die auf Raumtemperatur gekühlte hellbraune Lösung mit 0,2 ml Triethylamin und 0,35 g des im Beispiel 1 beschriebenen Aminoethylfarbstoffes versetzt und 1 Stunde bei Raumtemperatur, dann eine Stunde bei 90° gerührt. Als Hauptkomponente des Reaktionsgemisches erhält man den Farbstoff der Formel

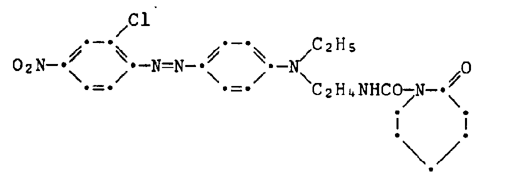

Beispiel 110 Auf analoge Art wie im Beispiel 10 beschrieben wird vom Farbstoff aus Beispiel 108 eine 5-prozentige wässrige Dispersion hergestellt. Damit wird im HT-Verfahren eine bezüglich Farbstoff 0,5-prozentige Färbung von Polyester erstellt.

Wird das gefärbte Gewebe vor oder nach der reduktiven Reinigung 60 Sekunden auf 220°C erhitzt, so erweist sich die Thermomigrierechtheit (Prüfung analog Beispiel 10) als sehr gut.

Wird mit derselben Farbstoff-Dispersion eine Thermosol-Färbung von Polyester vorgenommen (10 g/I Farbstoff, Flottenaufnahme 50 %, Fixiertemperatur 220°), so resultiert daraus eine ebenfalls sehr thermomigrierechte Färbung.

Beispiel 111: Ersetzt man in Beispiel 102 das ε-Caprolactam durch die äquivalente Menge 6-Valerolactam und das N-Ethylanilin durch die äquivalente Menge N-Methylanilin und setzt man gleich zu Beginn zusätzlich 20 ml Dimethylformamid als Lösungsmittel zu, so erhält man die Kupplungskomponente der Formel 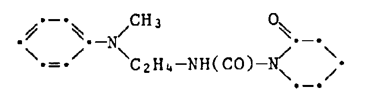 als salzhaltige Lösung in Dimethylformamid.

Wird die Hälfte dieser Lösung (0,05 mol) mit 100 ml 80 % Essigsäure vermischt und wie im Beispiel 108 angegeben mit 0,05 mol diazotiertem 2-Chlor-4-nitroanilin gekuppelt, so erhält man 15,5 g weinrotes, öliges Produkt Reinigung durch Säulenchromatographie an Kieselgel mit Essigester ergibt ein schwarzes Pulver der Formel 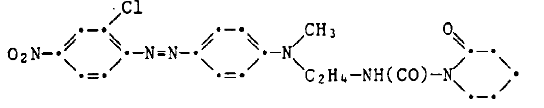 und vom Schmelzpunkt 166 - 169°C. Der Farbstoff färbt Polyestermaterial in roten Farbtönen.

Beispiel 112: Ersetzt man in Beispiel 108 das Chlornitroanilin durch die äquivalente Menge 2-Cyan-4-nitro-anilin, so erhält man 7,7 g Rohprodukt. Zur Reinigung wird das Rohprodukt zu 50 ml Tertiärbutyl-methylether gegeben und 3 Stunden bei Rückflusstemperatur gerührt. Nach Abkühlen auf Raumtemperatur wird abfiltriert und getrocknet. Man erhält 7,0 g schwarzbraune Kristalle der Formel 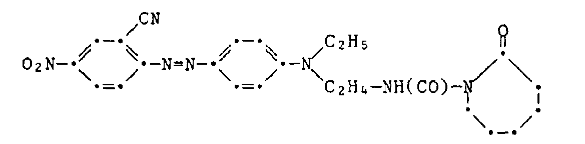 und vom Schmelzpunkt 137 - 139°C. Der Farbstoff färbt Polyestermaterial in rubinroten Farbtönen.

Beispiel 113: Auf analoge Art wie im Beispiel 102 beschrieben werden 9,6 g Brompentylisocyanat zunächst mit 5,7 g e-Caprolactam und anschliessend mit 7,2 g 3-(N-Ethyl)-amino-acetanilid umgesetzt zum Zwischenprodukt der Formel 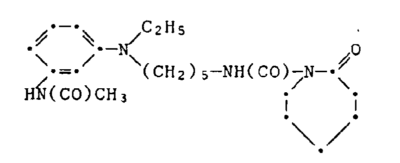 welches ohne Reinigung weiter verwendet wird.

10,5 g 6-Brom-2,4-dinitro-anilin werden auf die übliche Weise in Schwefelsäure diazotiert und bei 0 - 10°C gekuppelt auf eine Lösung der oben beschriebenen Kupplungskomponente in 200 ml 50 %iger Essigsäure. Das Reaktionsgemisch wird mit 600 g Eiswasser versetzt und mehrere Stunden gerührt. Danach wird die Wasserphase dekantiert und verworfen. Dieser Waschvorgang wird noch zweimal wiederholt. Trocknen an der Luft ergibt 21,8 g Produkt der Formel 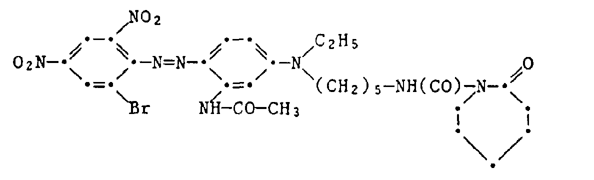 und vom Schmelzpunkt 80 - 85°C. Der Farbstoff färbt Polyestermaterial in rotstichig-blauen Farbtönen.

Beispiel 114: 16,9 g des im Beispiel 113 beschriebenen Farbstoffs, 110 ml Dimethylformamid und 3,3 g Kupfer(I) cyanid werden zusammengegeben und 12 Stunden bei 50°C gerührt. Das Reaktionsgemisch wird ausgegossen auf eine Vorlage von 600 ml Eiswasser und 13 g (0,03 mol) Eisen(lll)chlorid. Nach 2 Stunden Rühren wird filtriert, gewaschen und getrocknet. Man erhält 10,4 g Rohprodukt. Zur Reinigung wird suspendiert in 150 ml Methanol und 3 Stunden bei Rückflusstemperatur gerührt. Abkühlen auf 0 - 5°C, Filtrieren und Trocken an der Luft liefert schwarze Kristalle der Formel 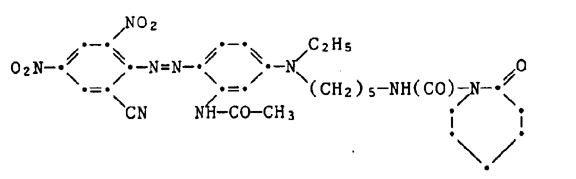 und vom Schmelzpunkt 120 - 125°C. Der Farbstoff färbt Polyestermaterial in blauen Farbtönen.

Beispiel 115: 16,2 g der gemäss Beispiel 102 erhaltenen Kupplungskomponente werden in 20 ml Dimethylformamid gelöst und mit 5,8 g Tetracyanethylen versetzt. Ueber Nacht wird bei 40°C gerührt. Nach Abkühlen auf 0 - 5°C werden 180 ml 70 %iges Methanol zugetropft. Nach 2 Stunden Rühren bei 0 - 5cC wird abfiltriert und gewaschen. Der Rückstand wird suspendiert in 50 ml Methanol und 2 Stunden bei Rückflusstemperatur gerührt. Abkühlen lassen, 4 Stunden bei 0 - 5°C Rühren, Abfiltrieren, Waschen und Trocknen bei 40°C liefert 7,5 g schwarze Kristalle der Formel 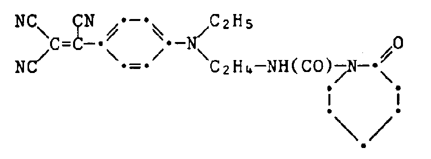 und vom Schmelzpunkt 112 - 115°C. Der Farbstoff färbt Polyestermaterial in roten Farbtönen.

Beispiel 116 - 120: Auf analoge Art wie im Beispiel 115 beschrieben erhält man folgende Tricyanvinyl-Farbstoffe:

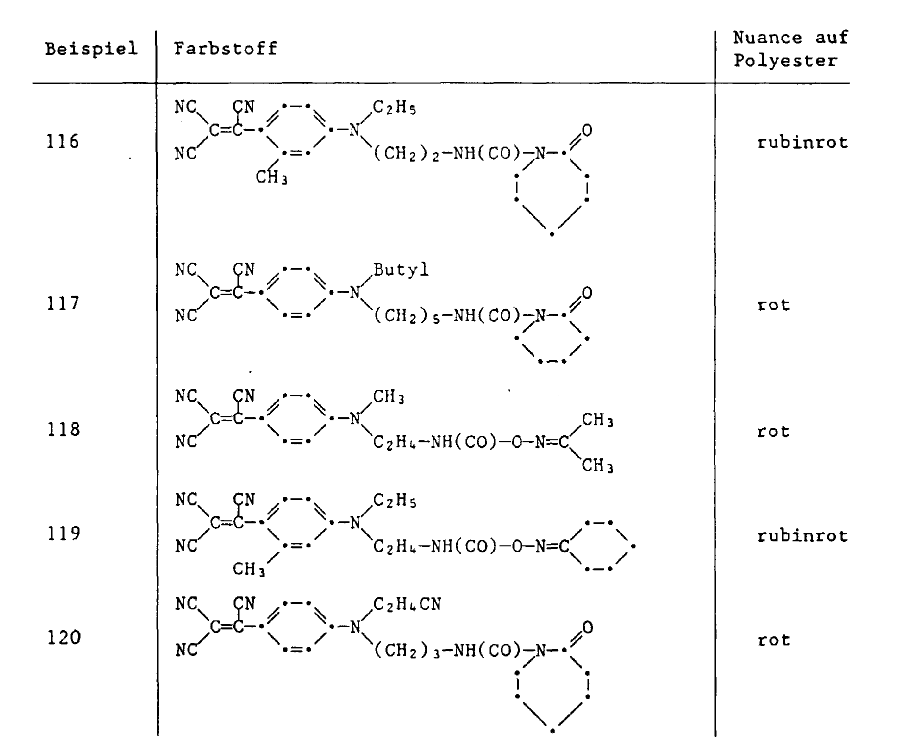

Beispiel 121: 0,8 g der im Beispiel 102 beschriebenen Additionsverbindung aus Chlorethylisocyanat und ε-Caprolactam, 10 ml Dimethylformamid, 1,1 g des Natriumsalzes von 1,4-Diamino-3-cyan-2-tetrazolyl-anthrachinon und 0,1 g Kaliumiodid werden über Nacht bei 80°C gerührt. Bei 0 - 5°C werden 50 ml Methanol zugetropft. Nach weiteren 2 Stunden Rühren bei 0 - 5°C wird abfiltriert. Nach dem Waschen mit kaltem Methanol und mit Wasser wird der Filtrierrückstand suspendiert in 20 ml Methanol und 2 Stunden bei Rückflusstemperatur gerührt. Nach dem Abkühlen wird abfiltriert, gewaschen und getrocknet bei 40°C. Man erhält 0,8 g blaues Pulver der Formel 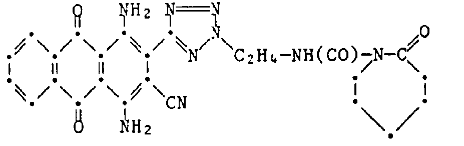 und vom Schmelzpunkt 123 - 133°C. Der Farbstoff färbt Polyestermaterial in blauen Tönen.

Beispiele 122 - 132: Auf analoge Art wie im Beispiel 121 oder im Beispiel 83 beschrieben erhält man folgende Anthrachinon-Farbstoffe:

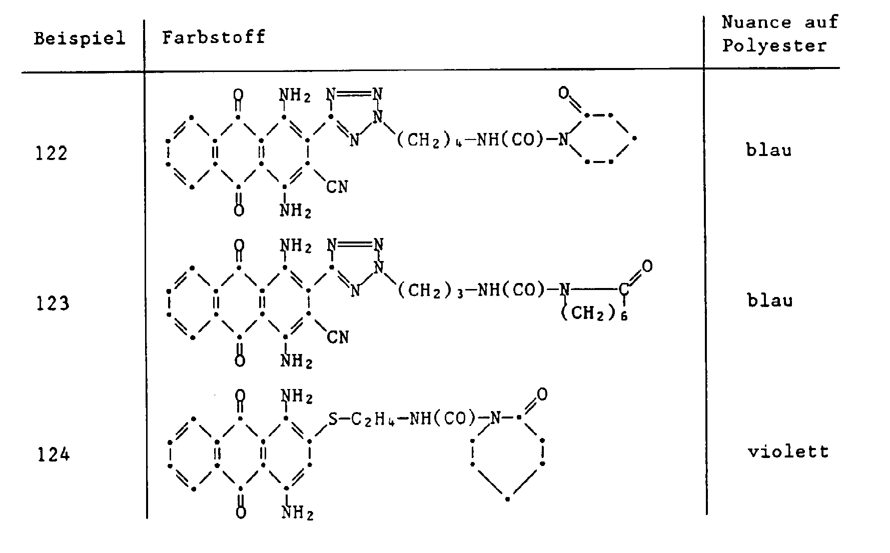

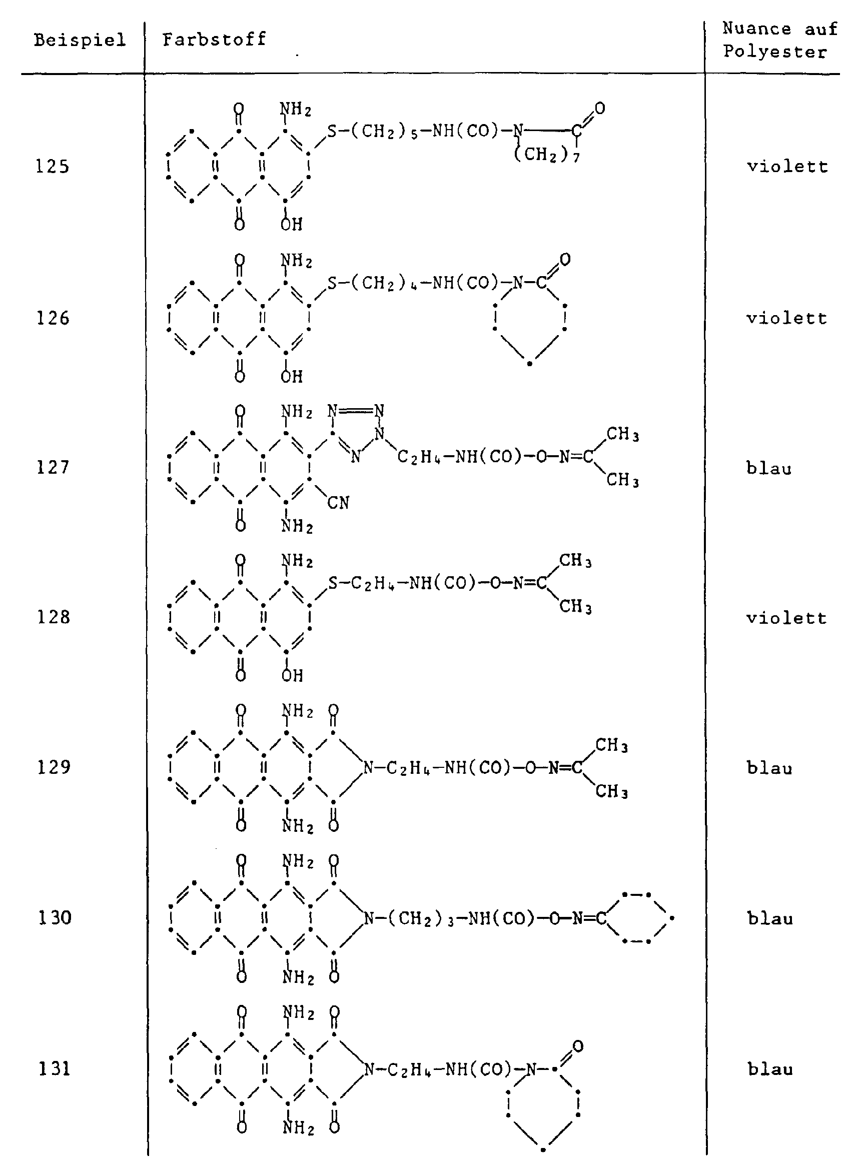

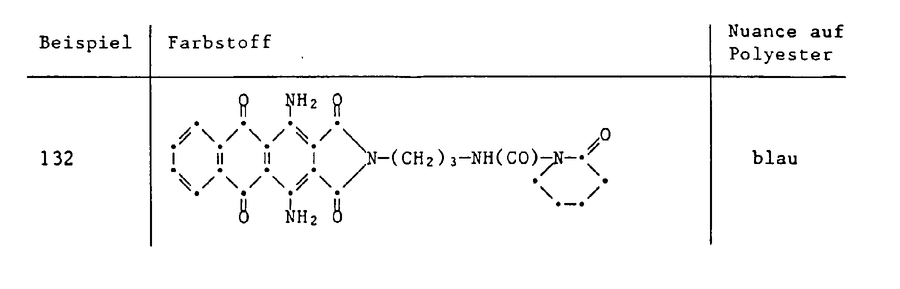

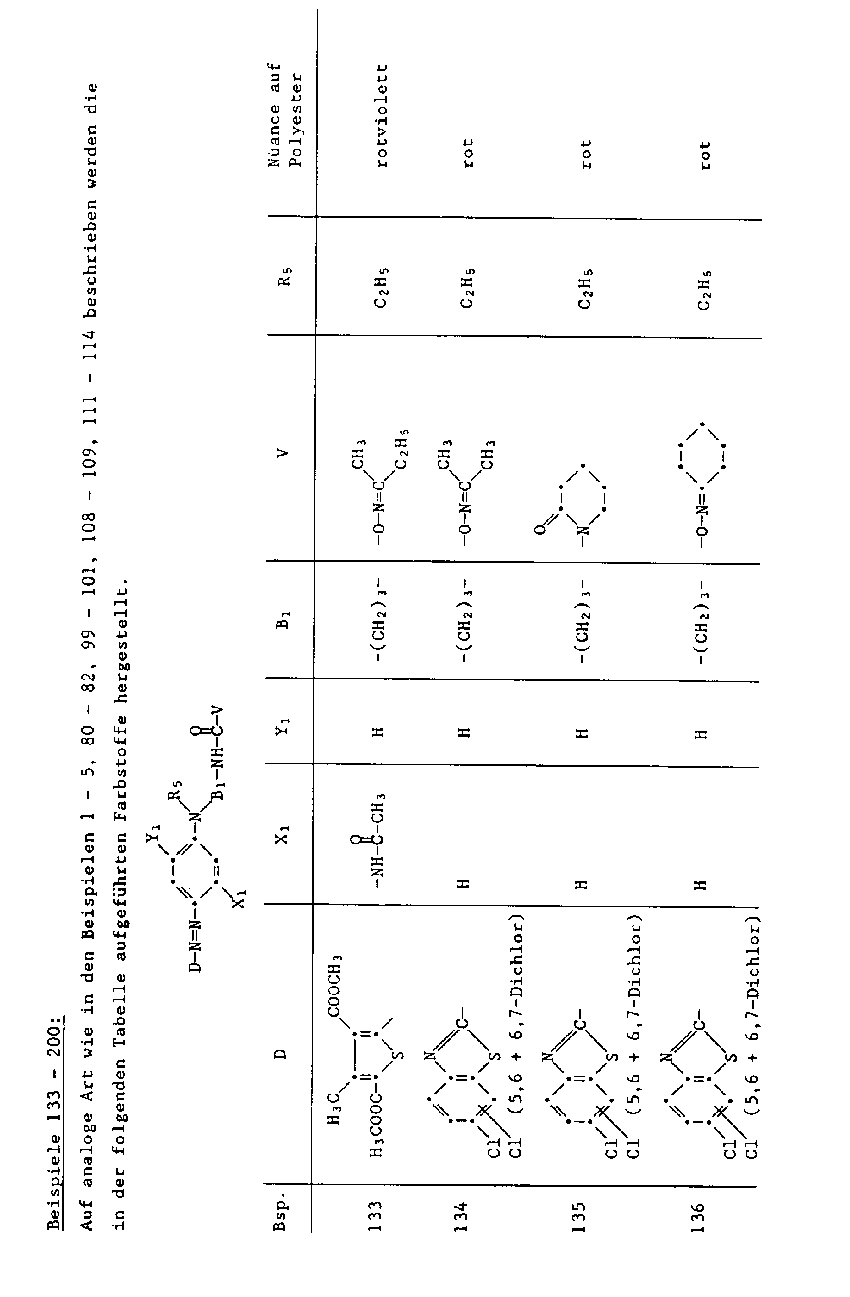

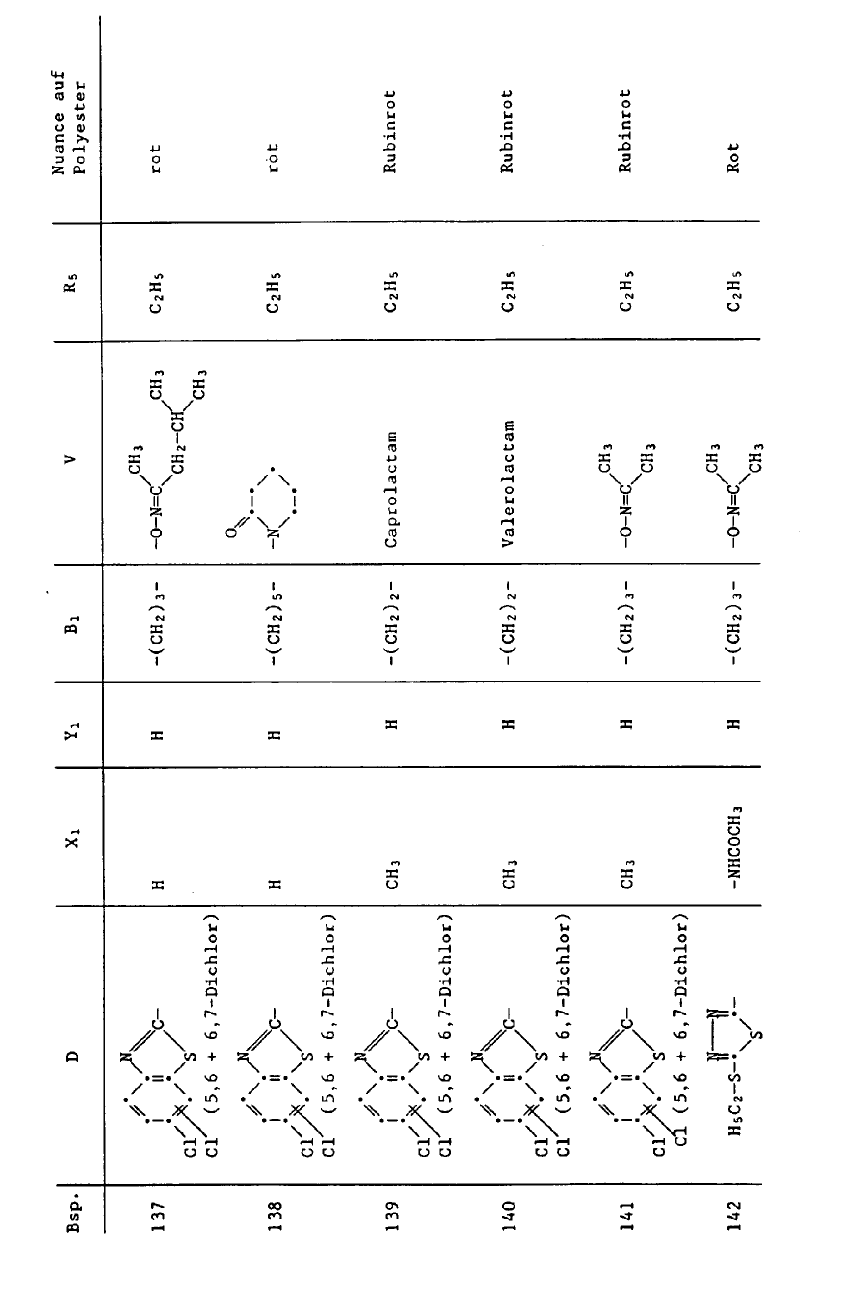

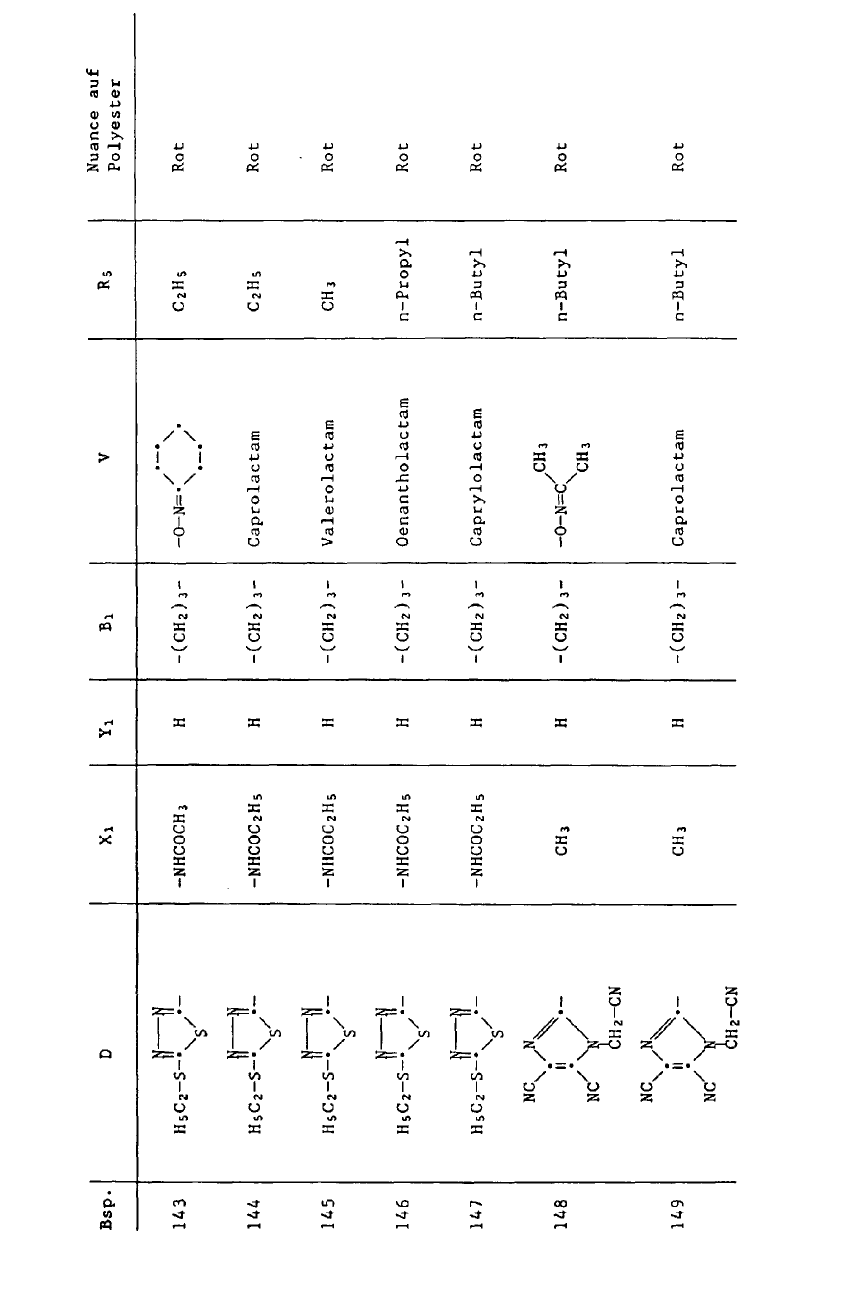

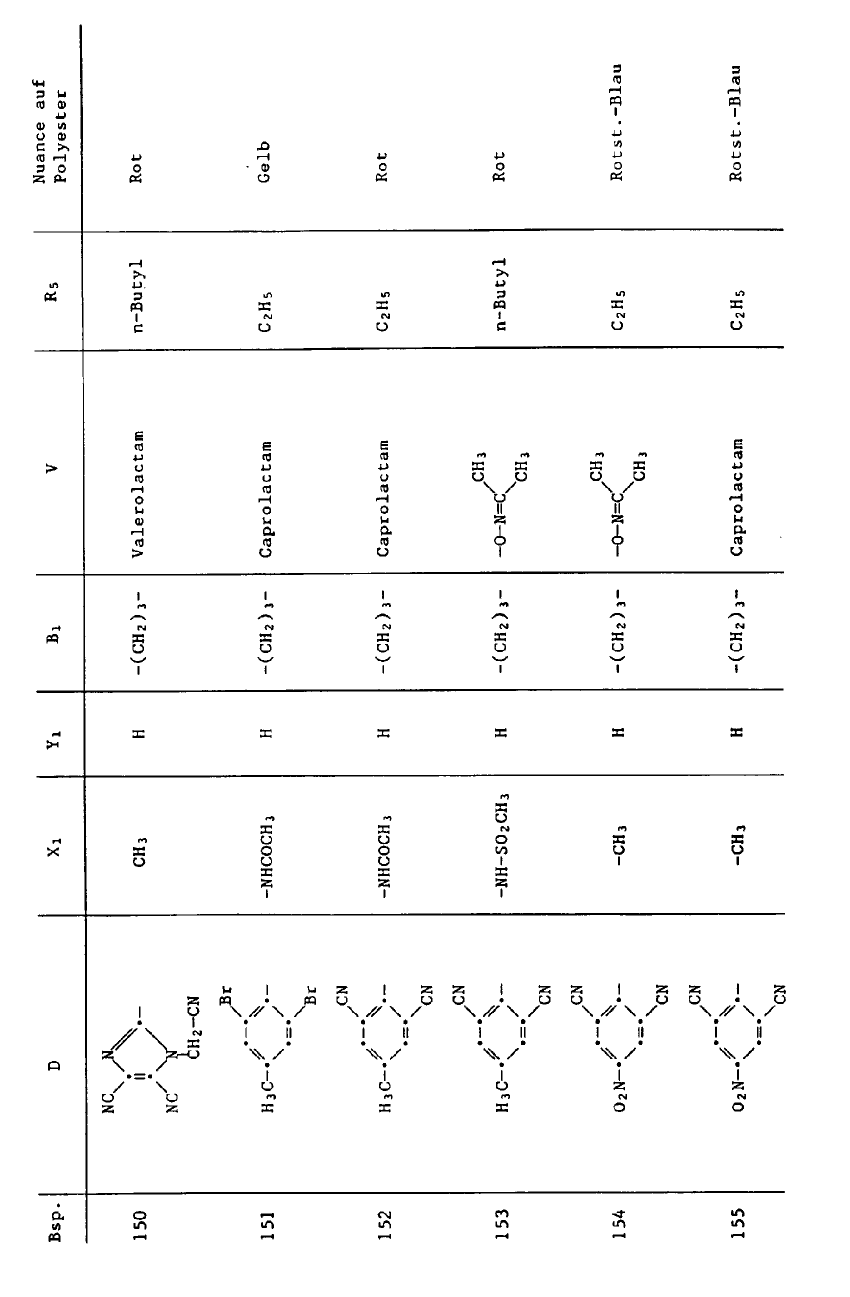

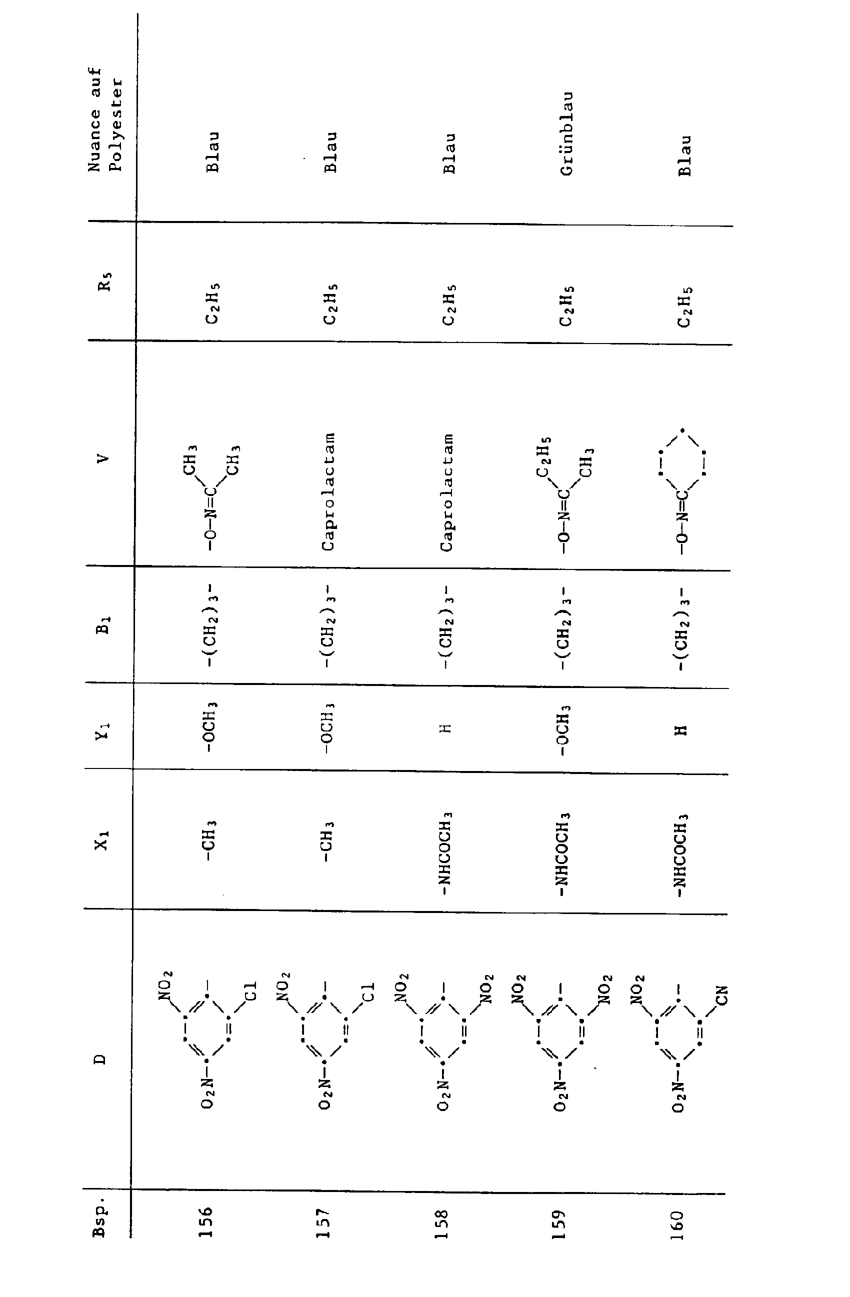

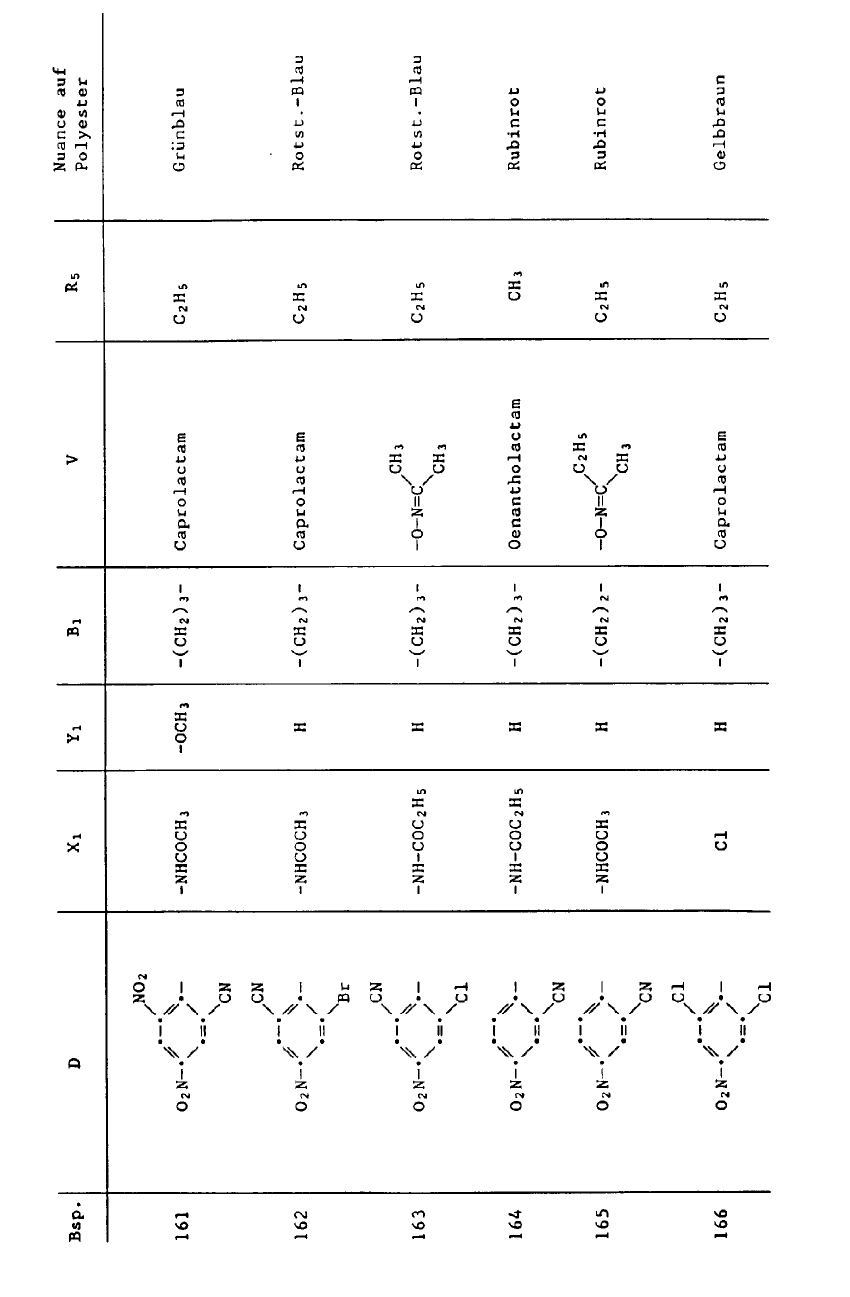

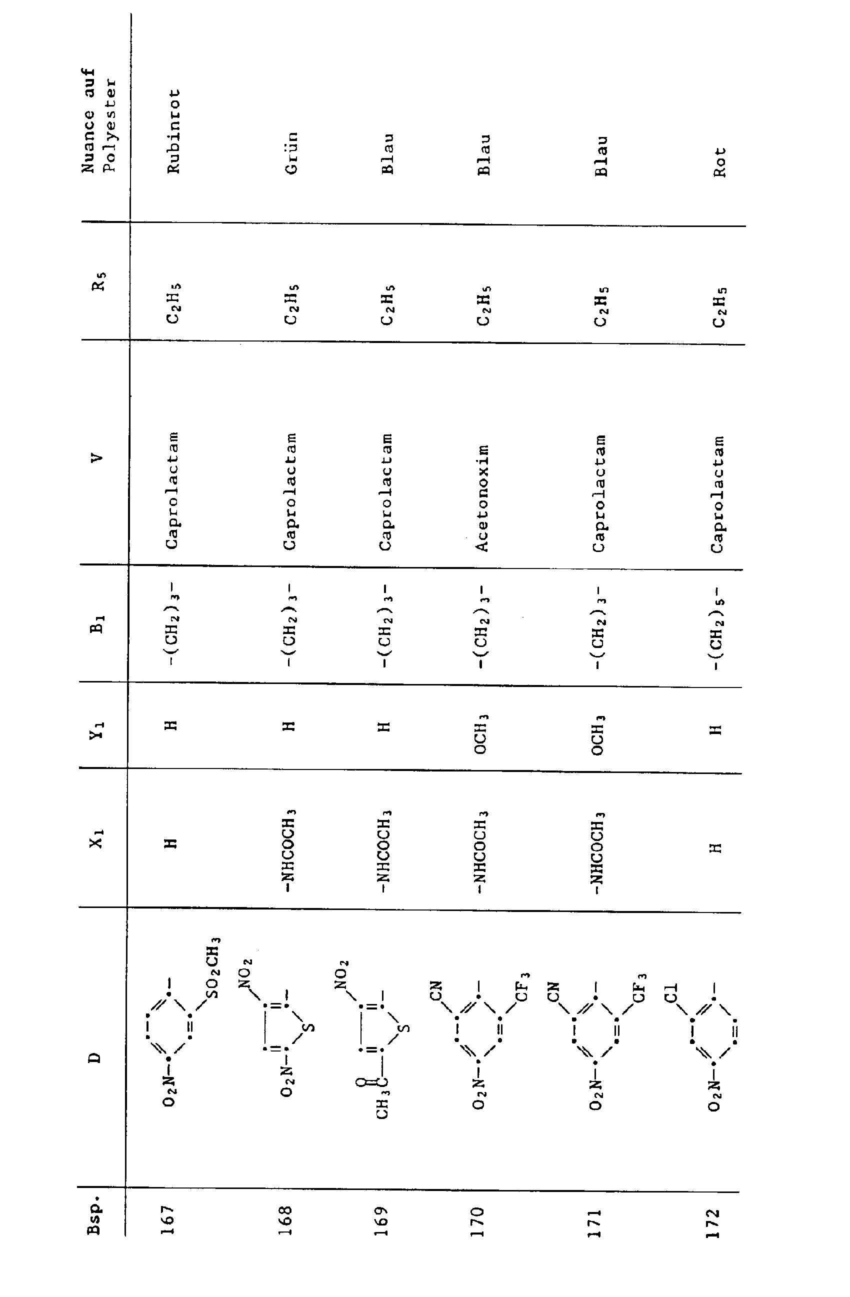

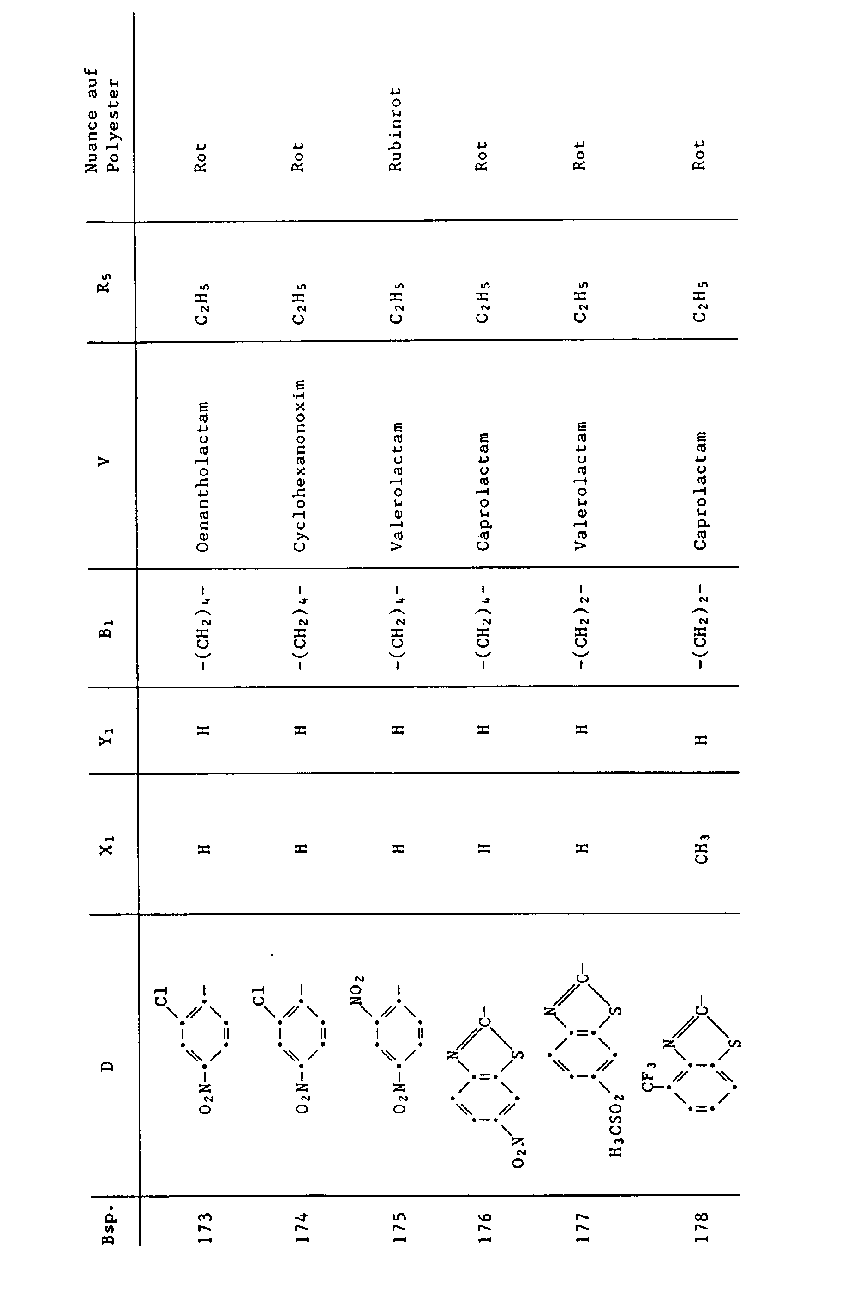

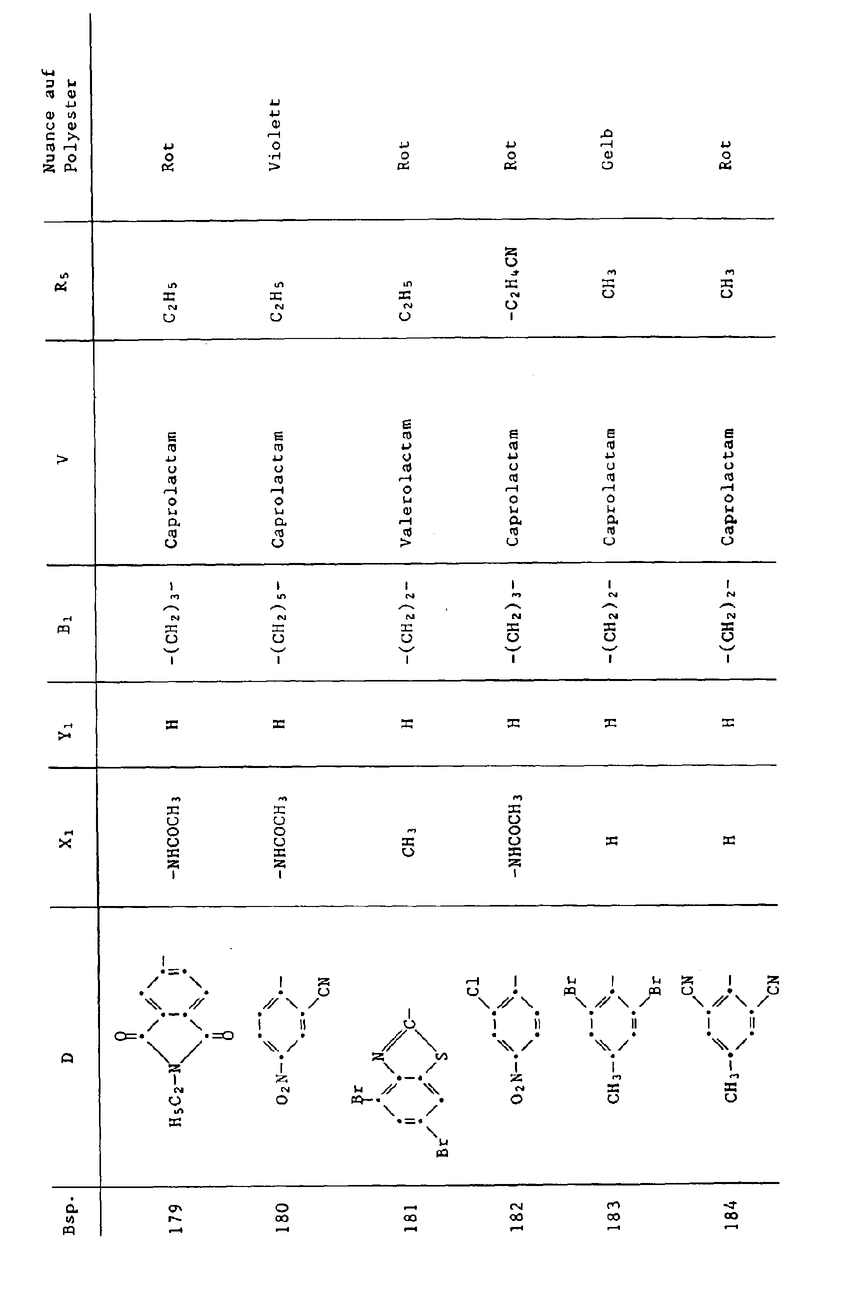

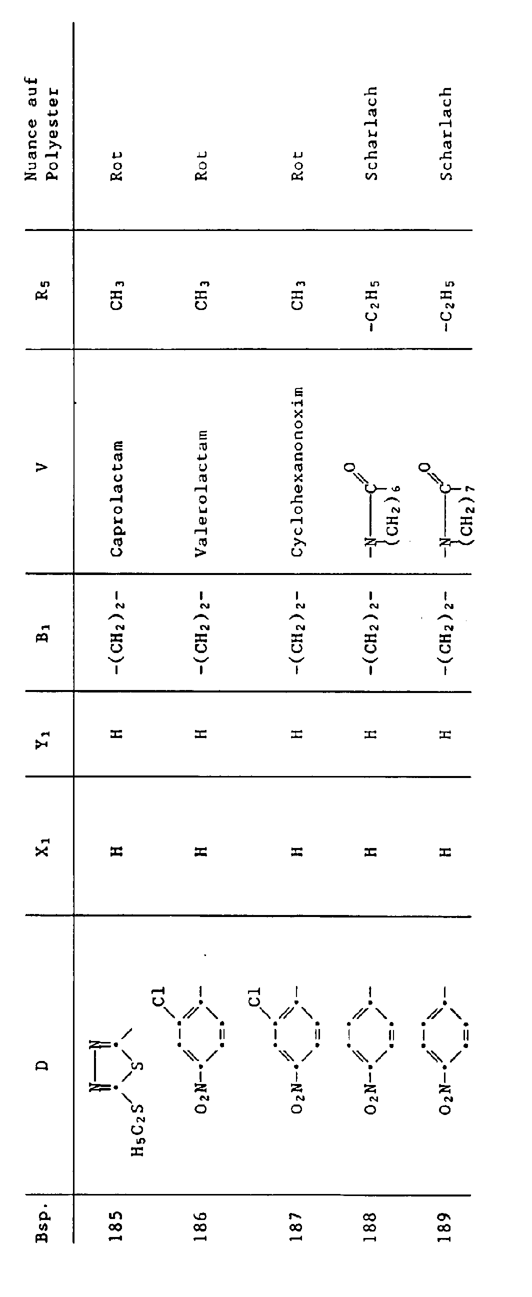

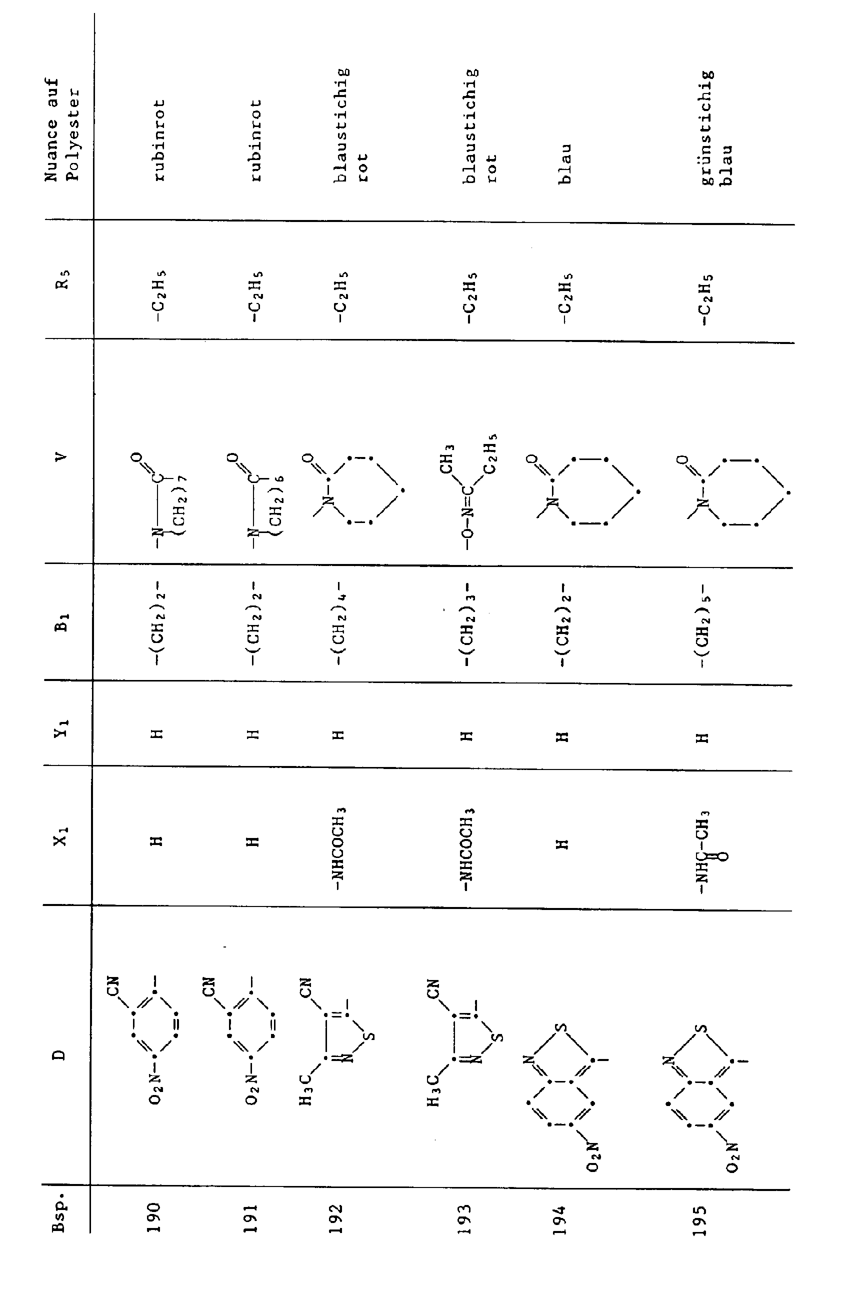

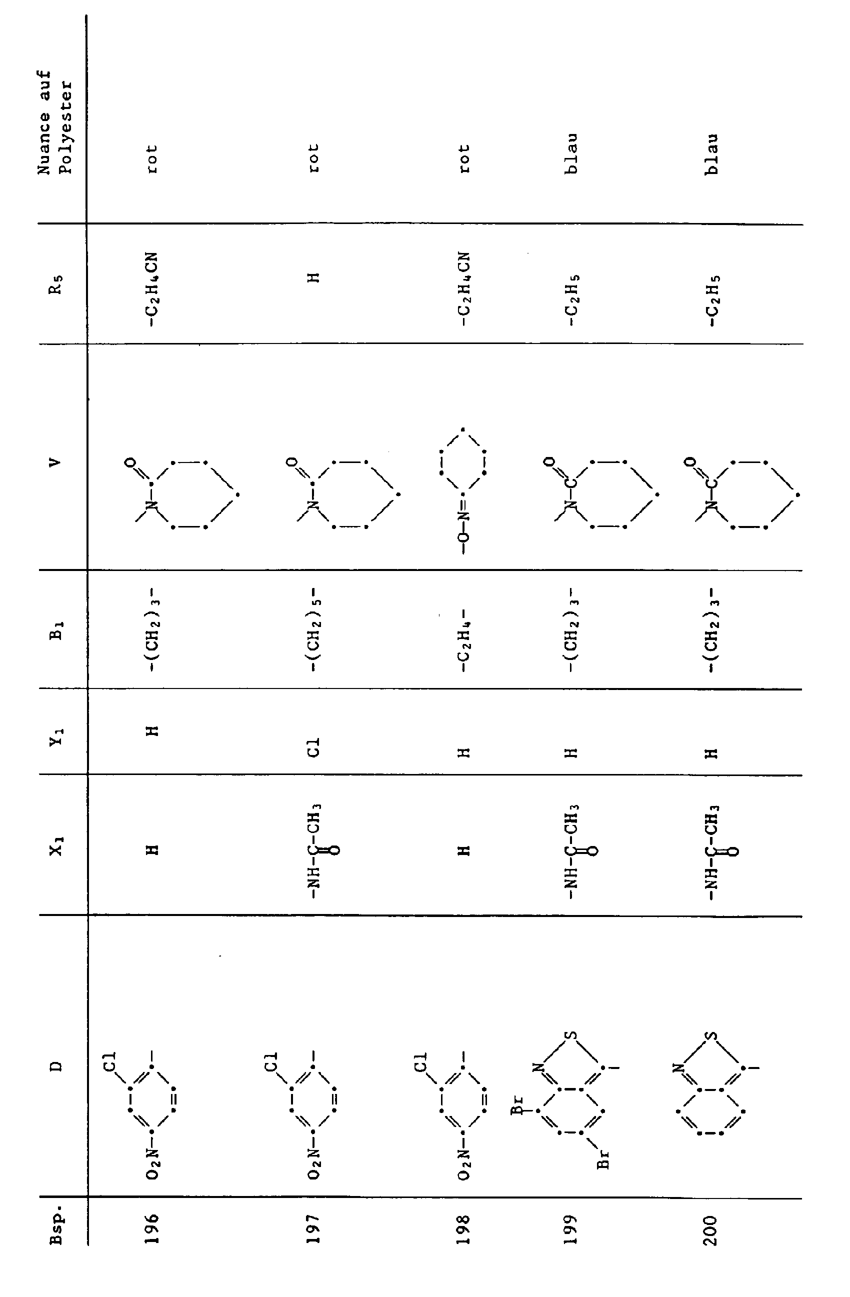

## Patentansprüche (Patentansprüche für folgende(n) Vertragsstaat(en) : BE, CH, DE, FR, GB, IT, LI)

1. Dispersionsfarbstoffe der Formel 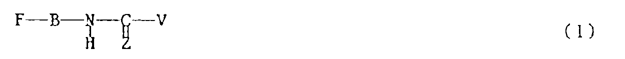 worin
F den Rest eines von wasserlöslichmachenden Gruppen freien Farbstoffes,
B ein Brückenglied oder eine direkte Bindung,
Z 0 oder S und
V den Rest einer Gruppe H-V bedeutet, wobei HV ein Oxim der Formel 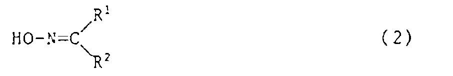 oder ein Lactam der Formel 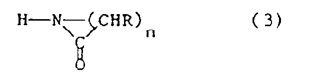 ist, worin
R¹ und R² unabhängig voneinander gegebenenfalls substituiertes Alkyl oder Aryl bedeuten oder R¹ und R² zusammen mit dem sie verbindenden C-Atom einen cycloaliphatischen Ring bilden,
R Wasserstoff oder Alkyl und
n eine ganze Zahl von 4 bis 11 bedeutet.

2. Dispersionsfarbstoffe gemäss Anspruch 1, dadurch gekennzeichnet, dass F ein Farbstoffrest aus einer der folgenden Farbstoffklassen ist: Nitrofarbstoffe, Nitrodiphenylaminfarbstoffe, Methinfarbstoffe, Chinolinfarbstoffe, Aminonaphthochinonfarbstoffe, Cumarinfarbstoffe und insbesondere Anthrachinonfarbstoffe, Tricyanvinylfarbstoffe und Azofarbstoffe, wie Monoazo und Disazofarbstoffe.

3. Dispersionsfarbstoffe gemäss einem der Ansprüche 1 oder 2, dadurch gekennzeichnet, dass es sich bei B um eine geradkettige oder verzweigte Alkylengruppe mit 2 bis 8 C-Atomen oder um eine der folgenden Gruppen handelt: 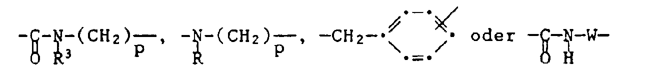 wobei R³ Wasserstoff oder C₁-Cₑ-Alkyl, p eine ganze Zahl von 1 bis 8 und W einen zweiwertigen organischen Rest bedeutet.

4. Dispersionsfarbstoffe gemäss einem der Ansprüche 1 bis 3, dadurch gekennzeichnet, dass Z Sauerstoff bedeutet.

5. Dispersionsfarbstoffe gemäss einem der Ansprüche 1 bis 4, dadurch gekennzeichnet, dass R¹ und R² unabhängig voneinander je C₁-C₁₂-Alkyl, Phenyl, Naphthyl oder Pyridyl bedeuten oder R¹ und R² zusammen mit dem sie verbindenden C-Atom Cyclopentyl, Cyclohexyl oder Cycloheptyl bedeuten.

6. Dispersionsfarbstoffe gemäss einem der Ansprüche 1 bis 4, dadurch gekennzeichnet, dass R Methyl oder Wasserstoff und n 4, 5 oder 6 bedeutet.

7. Dispersionsfarbstoffe der Formel 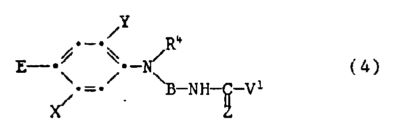 worin
E D-N=N- oder 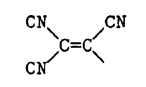 bedeutet, wobei D der Rest einer carbocyclischen oder heterocyclischen Diazokomponente ist,
X Wasserstoff, C₁-C₄-Alkyl, C₁-C₄-Alkoxy, Acylamino, Halogen, C₁-C₄-Alkylsulfonylamino oder eine Gruppe der Formel -NH-CO-NHQ, worin Q Wasserstoff, C₁-C₄-Alkyl oder Phenyl ist,
Y Wasserstoff, Halogen, C₁-C₄-Alkyl, C₁-C₄-Alkoxy, C₁-C₄-Alkoxy-C₁-C₄-alkyl oder C₁-C₄-Alkoxy-C₂-C₄-alkoxy,
R⁴ C₁-C₁₂-Alkyl, C₂-C₆-Alkenyl oder Phenyl, oder Y und R⁴, zusammen mit dem Stickstoffatom und den beiden sie verbindenden C-Atomen, einen 5- oder 6-gliedrigen Ring bilden,
B einen Rest der Formel 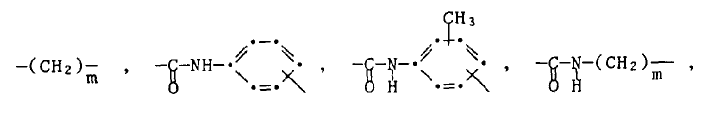 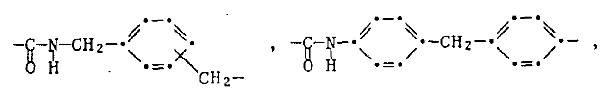 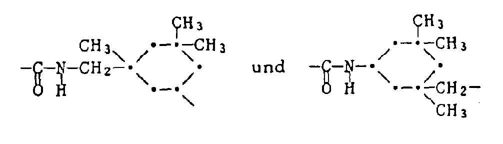 wobei
m eine ganze Zahl von 2 bis 6 bedeutet,
Z O oder S und
_{V}1 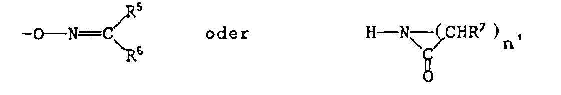 bedeutet, wobei
R⁵ und R⁶ unabhängig voneinander C₁-C₆-Alkyl bedeuten oder R⁵ und R⁶ zusammen mit dem sie verbindenden C-Atom einen Cyclopentyl-, Cyclohexyl- oder Cycloheptylrest bilden,
_{R}⁷ Methyl oder Wasserstoff und
n' 4, 5, 6 oder 7 ist.

8. Dispersionsfarbstoffe gemäss Anspruch 7, worin E D-N=N- bedeutet.

9. Dispersionsfarbstoffe gemäss Anspruch 7 und 8, worin D ein Benzthiazolyl- oder Benzisothiazolylrest ist, welcher unsubstituiert ist oder ein- oder zweimal durch Brom, Nitro oder Chlor substituiert ist oder ein Phenylrest, der ein-oder zweimal substituiert ist durch Nitro, Chlor, Cyan, Methyl- oder Ethylsulfonyl oder Phenylazo.

10. Dispersionsfarbstoffe gemäss einem der Ansprüche 7 bis 9, worin X Wasserstoff, Methyl, Methoxy, Chlor, Brom, Acetylamino oder Ureido ist.

11. Dispersionsfarbstoffe gemäss einem der Ansprüche 7 bis 10, worin Y Chlor, Methyl, Methoxy, Methoxyethyl, Methoxyethoxy oder vor allem Wasserstoff ist.

12. Dispersionsfarbstoffe der Formeln 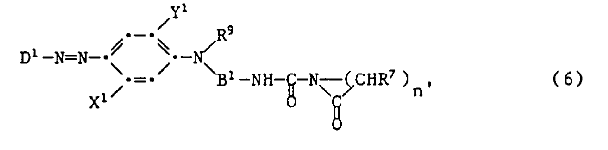 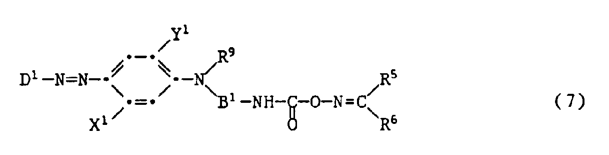 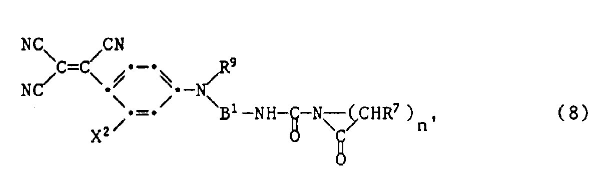 oder 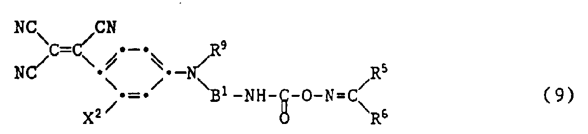 worin
D¹ einen Benzthiazolylrest, welcher unsubstituiert ist oder ein- oder zweimal durch Chlor substituiert ist oder einen Phenylrest, der ein- oder zweimal durch Nitro, Chlor, Cyan, Methyl- oder Ethylsulfonyl oder Phenylazo substituiert ist,
X¹ Wasserstoff, Methyl, Methoxy, Chlor, Brom oder Acetylamino,
Y¹ Chlor, Methyl, Methoxy, Methoxyethyl, Methoxyethoxy oder Wasserstoff,
X² Wasserstoff, Methyl, Methoxy, Chlor oder Brom,
R⁹ C₁-C₄-Alkyl, welches unsubstituiert ist oder durch Hydroxy, Cyan, C₁-C₄-Alkoxy oder Phenyl substituiert ist,
B¹ einen Rest der Formel 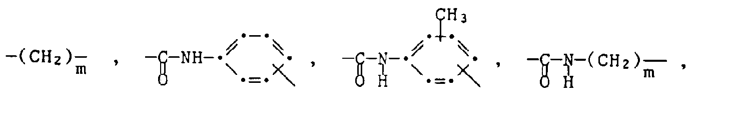 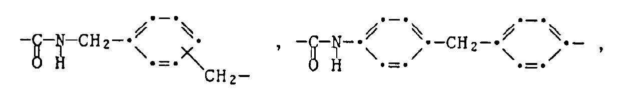 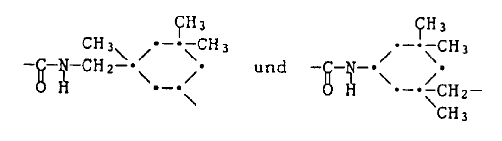 wobei
m eine ganze Zahl von 2 bis 6 bedeutet,
R⁵ und R⁶ unabhängig voneinander C₁-C₆-Alkyl oder R⁵ und R⁶ bilden zusammen mit dem sie verbindenden C-Atom einen Cyclopentyl-, Cyclohexyl- oder Cycloheptylrest,
R⁷ Methyl oder Wasserstoff und
n' 4, 5, 6 oder 7 bedeuten.

13. Verfahren zur Herstellung der Farbstoffe gemäss Anspruch 7, dadurch gekennzeichnet, dass man ein diazotiertes Amin der Formel 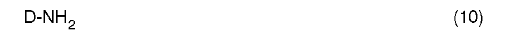
oder Tetracyanethylen mit einer Verbindung der Formel 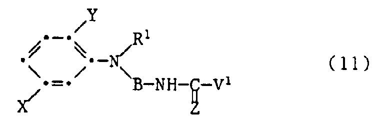 umsetzt

14. Verfahren zur Herstellung der Farbstoffe der Formel (4) gemäss Anspruch 7, worin V¹ einen Oximrest darstellt, dadurch gekennzeichnet, dass man eine Verbindung der Formel 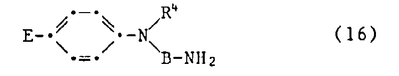 mit einem Halogenameisensäureester eines Oxims der Formel 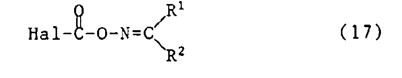 umsetzt

15. Verfahren zur Herstellung von Farbstoffen der Formel 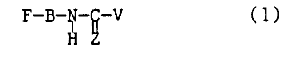 oder 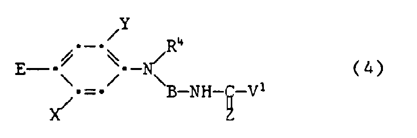 worin Bein Brückenglied der Formel 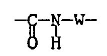 darstellt, wobei W einen zweiwertigen organischen Rest bedeutet, und worin die übrigen Symbole die in den Ansprüchen 1 und 7 angegebenen Bedeutungen aufweisen, dadurch gekennzeichnet, dass man ein Diisocyanat der Formel 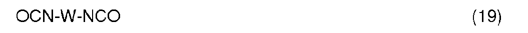 in beliebiger Reihenfolge mit einem Farbstoff, enthaltend eine reaktionsfähige -OH- oder 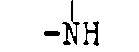 Gruppe, und mit einem Oxim der Formel (2) oder einem Lactam der Formel (3) umsetzt.

16. Verbindungen der Formel 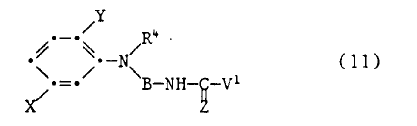 worin X, Y, R⁴, B, Z und V¹ die im Anspruch 7 angegebene Bedeutung aufweisen.

17. Verfahren zur Herstellung der Verbindungen gemäss Anspruch 16, dadurch gekennzeichnet, dass man Halogenalkylisocyanat oder Halogenalkylisothiocyanat der Formel 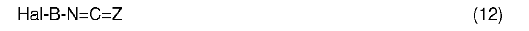 mit einem Oxim oder Lactam der Formel 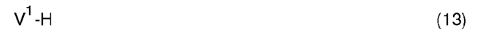 umsetzt und das erhaltene Reaktionsprodukt der Formel 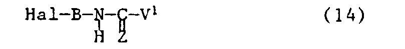 mit einer Verbindung der Formel 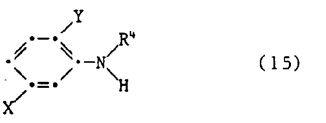 zur Reaktion bringt.

18. Verwendung der Dispersionsfarbstoffe gemäss einem der Ansprüche 1 bis 12 zum Färben oder Bedrucken von halbsynthetischem oder synthetischem hydrophobem Fasermaterial, insbesondere Textilmaterial.

19. Verwendung nach Anspruch 18 zum Färben oder Bedrucken von Textilmaterial aus Polyesterfasern.

20. Verfahren zum Färben oder Bedrucken von halbsynthetischem oder synthetischem hydrophobem Fasermaterial, insbesondere Textilmaterial, dadurch gekennzeichnet, dass man eine oder mehrere der in den Ansprüchen 1 bis 12 definierten Verbindungen auf das Material aufbringt oder diesem einverleibt und anschliessend eine thermische Nachbehandlung während 30 bis 500 Sekunden bei 100 bis 240°C durchführt.

21. Verfahren gemäss Anspruch 20, dadurch gekennzeichnet, dass die thermische Nachbehandlung während 45 bis 200 Sekunden bei 180 bis 220°C durchgeführt wird.

22. Das gemäss Anspruch 20 oder 21 gefärbte oder bedruckte Material.

## Patentansprüche (Patentansprüche für folgende(n) Vertragsstaat(en) : ES)

1. Verfahren zur Herstellung der Dispersionsfarbstoffe der Formel 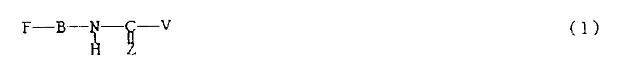 worin
F den Rest eines von wasserlöslichmachenden Gruppen freien Farbstoffes,
B ein Brückenglied oder eine direkte Bindung,
Z 0 oder S und
V den Rest einer Gruppe H-V bedeutet, wobei HV ein Oxim der Formel 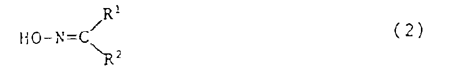 oder ein Lactam der Formel 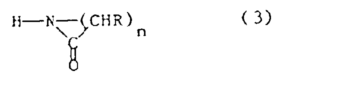 ist, worin
R¹ und R² unabhängig voneinander gegebenenfalls substituiertes Alkyl oder Aryl bedeuten oder R¹ und R² zusammen mit dem sie verbindenden C-Atom einen cycloaliphatischen Ring bilden,
R Wasserstoff oder Alkyl und
n eine ganze Zahl von 4 bis 11 bedeutet.
dadurch gekennzeichnet, dass man einen Farbstoff F-H mit einer Verbindung der Formel 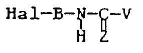
worin F; B, Z und V die unter der Formel (1) angegebene Bedeutungen haben und Hal Chlor oder Brom bedeutet, umsetzt.

2. Verfahren gemäss Anspruch 1, dadurch gekennzeichnet, dass F ein Farbstoffrest aus einer der folgenden Farbstoffklassen ist: Nitrofarbstoffe, Nitrodiphenylaminfarbstoffe, Methinfarbstoffe, Chinolinfarbstoffe, Aminonaphthochinonfarbstoffe, Cumarinfarbstoffe und insbesondere Anthrachinonfarbstoffe, Tricyanvinylfarbstoffe und Azofarbstoffe, wie Monoazo und Disazofarbstoffe.

3. Verfahren gemäss einem der Ansprüche 1 oder 2, dadurch gekennzeichnet, dass es sich bei B um eine geradkettige oder verzweigte Alkylengruppe mit 2 bis 8 C-Atomen oder um eine der folgenden Gruppen handelt: 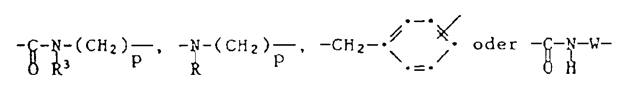 wobei R³ Wasserstoff oder C₁-C₆-Alkyl, p eine ganze Zahl von 1 bis 8 und W einen zweiwertigen organischen Rest bedeutet.

4. Verfahren gemäss einem der Ansprüche 1 bis 3, dadurch gekennzeichnet, dass Z Sauerstoff bedeutet.

5. Verfahren gemäss einem der Ansprüche 1 bis 4, dadurch gekennzeichnet, dass R¹ und R² unabhängig voneinander je C₁-C₁₂-Alkyl, Phenyl, Naphthyl oder Pyridyl bedeuten oder R¹ und R² zusammen mit dem sie verbindenden C-Atom Cyclopentyl, Cyclohexyl oder Cycloheptyl bedeuten.

6. Verfahren gemäss einem der Ansprüche 1 bis 4, dadurch gekennzeichnet, dass R Methyl oder Wasserstoff und n 4, 5 oder 6 bedeutet.

7. Verfahren zur Herstellung der Dispersionsfarbstoffe der Formel 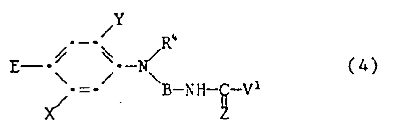 worin
E D-N=N- oder 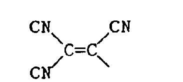 bedeutet, wobei D der Rest einer carbocyclischen oder heterocyclischen Diazokomponente ist,
X Wasserstoff, G₁-C₄-Alkyl, C₁-C₄-Alkoxy, Acylamino, Halogen, C₁-C₄-Alkylsulfonylamino oder eine Gruppe der Formel -NH-CO-NHQ, worin Q Wasserstoff, C₁-C₄-Alkyl oder Phenyl ist,
Y Wasserstoff, Halogen, C₁-C₄-Alkyl, C₁-C₄₋Alkoxy, C₁-C₄₋Alkoxy-C₁-C₄-alkyl oder C₁-C₄-Alkoxy-C₂-C₄-alkoxy,
R⁴ C₁-C₁₂-Alkyl, C₂-C₆-Alkenyl oder Phenyl, oder Y und R⁴, zusammen mit dem Stickstoffatom und den beiden sie verbindenden C-Atomen, einen 5- oder 6-gliedrigen Ring bilden,
B einen Rest der Formel 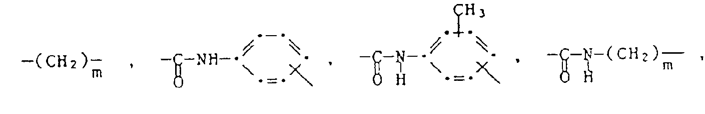 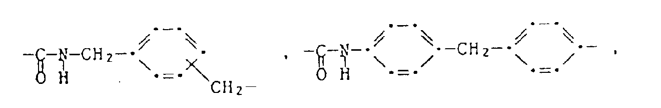 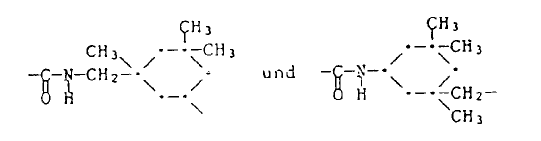 wobei
m eine ganze Zahl von 2 bis 6 bedeutet,
Z 0 oder S und
_{V}1 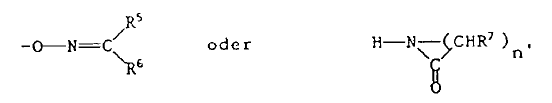 bedeutet, wobei
R⁵ und R⁶ unabhängig voneinander C₁-C₆-Alkyl bedeuten oder R⁵ und R⁶ zusammen mit dem sie verbindenden C-Atom einen Cyclopentyl-, Cyclohexyl- oder Cycloheptylrest bilden,
_{R}⁷ Methyl oder Wasserstoff und
n' 4, 5, 6 oder 7 ist;
dadurch gekennzeichnet, dass man ein diazotiertes Amin der Formel 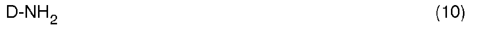 oder Tetracyanethylen mit einer Verbindung der Formel 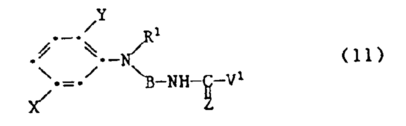 umsetzt.

8. Verfahren gemäss Anspruch 7, worin E D-N=N- bedeutet.

9. Verfahren gemäss Anspruch 7 und 8, worin D ein Benzthiazolyl- oder Benzisothiazolylrest ist, welcher unsubstituiert ist oder ein- oder zweimal durch Brom, Nitro oder Chlor substituiert ist oder ein Phenylrest, der ein- oder zweimal substituiert ist durch Nitro, Chlor, Cyan, Methyl- oder Ethylsulfonyl oder Phenylazo.

10. Verfahren gemäss einem der Anspruche 7 bis 9, worin X Wasserstoff, Methyl, Methoxy, Chlor, Brom, Acetylamino oder Ureido ist.

11. Verfahren gemäss einem der Ansprüche 7 bis 10, worin Y Chlor, Methyl, Methoxy, Methoxyethyl, Methoxyethoxy oder vor allem Wasserstoff ist.

12. Verfahren zur Herstellung der Farbstoffe der Formel (4) gemäss Anspruch 7, worin V¹ einen Oximrest darstellt, dadurch gekennzeichnet, dass man eine Verbindung der Formel 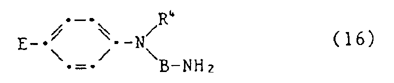 mit einem Halogenameisensäureester eines Oxims der Formel 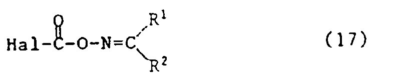 umsetzt.

13. Verfahren zur Herstellung von Farbstoffen der Formel 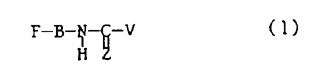 oder 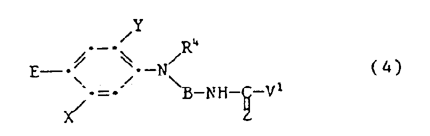 worin Bein Brückenglied der Formel 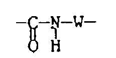 darstellt, wobei W einen zweiwertigen organischen Rest bedeutet, und worin die übrigen Symbole die in den Ansprüchen 1 und 7 angegebenen Bedeutungen aufweisen, dadurch gekennzeichnet, dass man ein Diisocyanat der Formel 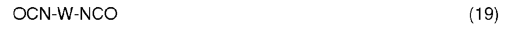 in beliebiger Reihenfolge mit einem Farbstoff, enthaltend eine reaktionsfähige -OH- oder 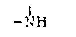 Gruppe, und mit einem Oxim der Formel (2) oder einem Lactam der Formel (3) umsetzt.

14. Verfahren gemäss einem der Ansprüche 7, 12 oder 13 zur Herstellung der Farbstoffe der Formeln 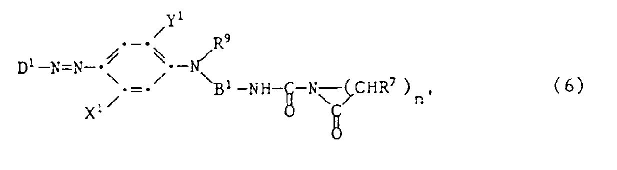 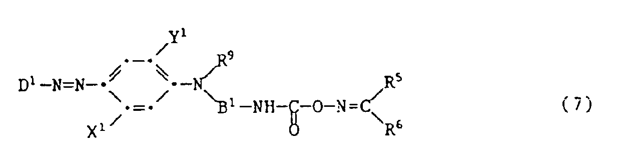 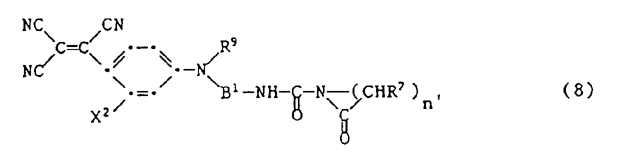 oder 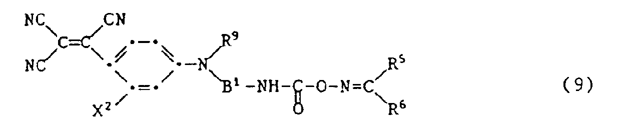 worin
D¹ einen Benzthiazolylrest, welcher unsubstituiert ist oder ein- oder zweimal durch Chlor substituiert ist oder einen Phenylrest, der ein- oder zweimal durch Nitro, Chlor, Cyan, Methyl- oder Ethylsulfonyl oder Phenylazo substituiert ist,
X¹ Wasserstoff, Methyl, Methoxy, Chlor, Brom oder Acetylamino,
Y¹ Chlor, Methyl, Methoxy, Methoxyethyl, Methoxyethoxy oder'Wasserstoff,
X² Wasserstoff, Methyl, Methoxy, Chlor oder Brom,
R⁹ C₁-C₄-Alkyl, welches unsubstituiert ist oder durch Hydroxy, Cyan, C₁-C₄-Alkoxy oder Phenyl substituiert ist,
B¹ einen Rest der Formel 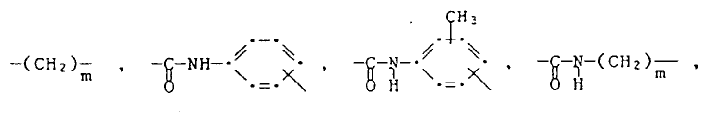 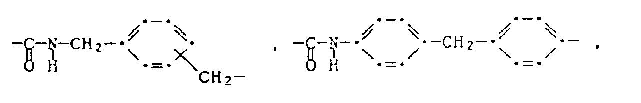 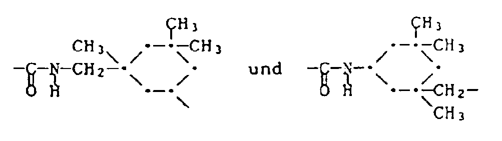 wobei
m eine ganze Zahl von 2 bis 6 bedeutet,
R⁵ und R⁶ unabhängig voneinander C₁-C₆-Alkyl oder R⁵ und R⁶ bilden zusammen mit dem sie verbindenden C-Atom einen Cyclopentyl-, Cyclohexyl- oder Cycloheptylrest,
R⁷ Methyl oder Wasserstoff und
n' 4, 5, 6 oder 7 bedeuten.

15. Verfahren zur Herstellung der Verbindungen der Formel 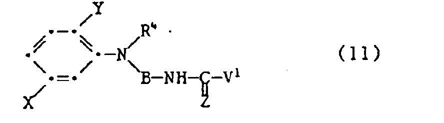 worin X, Y, R⁴, B, Z und V¹ die im Anspruch 7 angegebene Bedeutung aufweisen,
dadurch gekennzeichnet, dass man Halogenalkylisocyanat oder Halogenalkylisothiocyanat der Formel 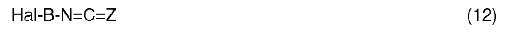 mit einem Oxim oder Lactam der Formel 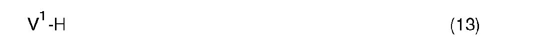 umsetzt und das erhaltene Reaktionsprodukt der Formel 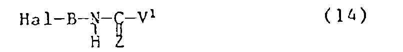 mit einer Verbindung der Formel 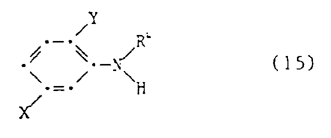 zur Reaktion bringt.

16. Verwendung der Dispersionsfarbstoffe gemäss einem der Ansprüche 1 bis 12 zum Färben oder Bedrucken von halbsynthetischem oder synthetischem hydrophobem Fasermaterial, insbesondere Textilmaterial.

17. Verwendung nach Anspruch 16 zum Färben oder Bedrucken von Textilmaterial aus Polyesterfasern.

18. Verfahren zum Färben oder Bedrucken von halbsynthetischem oder synthetischem hydrophobem Fasermaterial, insbesondere Textilmaterial, dadurch gekennzeichnet, dass man eine oder mehrere der in den Ansprüchen 1 bis 12 definierten Verbindungen auf das Material aufbringt oder diesem einverleibt und anschliessend eine thermische Nachbehandlung während 30 bis 500 Sekunden bei 100 bis 240°C durchführt.

19. Verfahren gemäss Anspruch 18, dadurch gekennzeichnet, dass die thermische Nachbehandlung während 45 bis 200 Sekunden bei 180 bis 220°C durchgeführt wird.

20. Das gemäss Anspruch 18 oder 19 gefärbte oder bedruckte Material.

## Claims (Claims for the following Contracting State(s) : BE, CH, DE, FR, GB, IT, LI)

1. A disperse dye of the formula 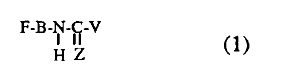 where
F is the radical of a dye which is free of water-solubilizing groups
B is a bridge member or a direct bond,
Z is O or S and
V is the radical of a group H-V, where H-V is an oxime of the formula 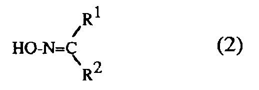
or a lactam of the formula 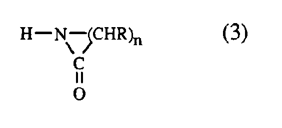 in which
R¹ and R² are each independently of the other substituted or unsubstituted alkyl or aryl, or R¹ and R² together with the carbon atom linking them form a cycloaliphatic ring,
R is hydrogen or alkyl and
n is an integer from 4 to 11.

2. A disperse dye according to claim 1, wherein F is a dye radical from one of the following dye classes: nitro dyes, nitrodiphenylamine dyes, methine dyes, quinoline dyes, aminonaphthoquinone dyes, coumarin dyes and in particular anthraquinone dyes, tricyanovinyl dyes and azo dyes, such as monoazo and disazo dyes.

3. A disperse dye according to either of claims 1 and 2, wherein B is a straight-chain or branched alkylene group of 2 to 8 carbon atoms or one of the following groups: 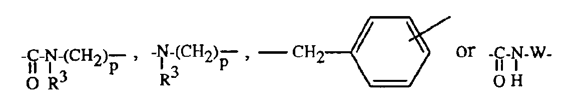 where R³ is hydrogen or C₁-C₆alkyl, p is an integer from 1 to 8 and W is a divalent organic radical.

4. A disperse dye according to any one of claims 1 to 3, wherein Z is oxygen.

5. A disperse dye according to any one of claims 1 to 4, wherein R¹ and R² are each independently of the other C₁-C₁₂alkyl, phenyl, naphthyl or pyridyl or R¹ and R² together with the carbon atom linking them are cyclopentyl, cyclohexyl or cycloheptyl.

6. A disperse dye according to any one of claims 1 to 4, wherein R is methyl or hydrogen and n is 4, 5 or 6.

7. A disperse dye of the formula 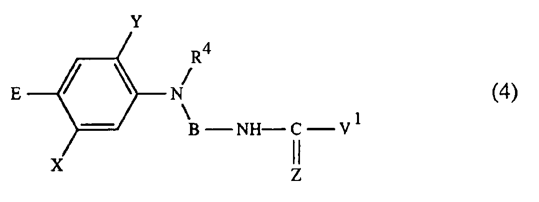 where
E is D-N=N- or 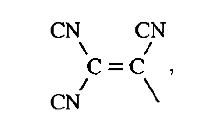
wherein D is the radical of a carbocyclic or heterocyclic diazo component,
X is hydrogen, C₁-C₄alkyl, C₁-C₄alkoxy, acylamino, halogen, C₁-C₄alkylsulfonylamino or a group of the formula
-NH-CO-NHQ, in which Q is hydrogen, C₁-C₄alkyl or phenyl,
Y is hydrogen, halogen, C₁-C₄alkyl, C₁-C₄alkoxy, C₁-C₄alkoxy-C₁-C₄alkyl or C₁-C₄alkoxy-C₂-C₄alkoxy.
R⁴ is C₁-C₁₂alkyl, C₂-C₆alkenyl or phenyl, or Y and R⁴ together with the nitrogen atom and the two carbon atoms linking them form a 5- or 6-membered ring,
B is a radical of the formula 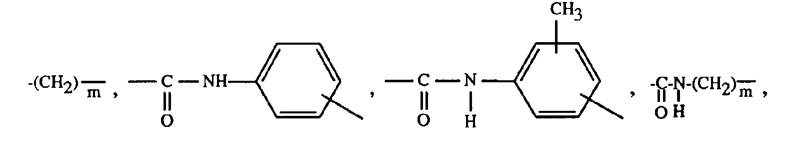 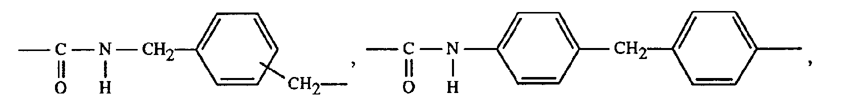 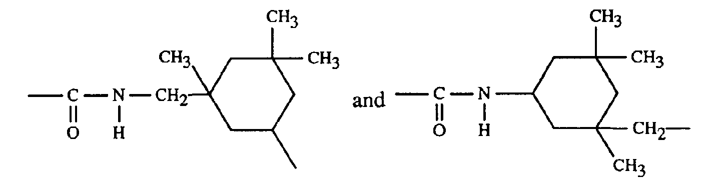
where m is an integer from 2 to 6,
Z is 0 or S and
V¹ is 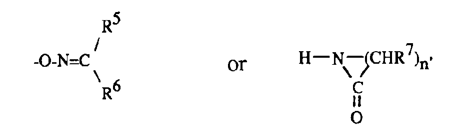
where R⁵ and R⁶ are each independently of the other C₁ -C₆alkyl or R⁵ and R⁶ together with the carbon atom linking them form a cyclopentyl, cyclohexyl or cycloheptyl radical,
R⁷ is methyl or hydrogen and
n' is 4, 5, 6 or 7.

8. A disperse dye according to claim 7, wherein E is D-N=N-.

9. A disperse dye according to either of claims 7 and 8, wherein D is a benzothiazolyl or benzisothiazolyl radical which is unsubstituted or monosubstituted or disubstituted by bromine, nitro or chlorine or is a phenyl radical which is monosubstituted or disubstituted by nitro, chlorine, cyano, methylsulfonyl or ethylsulfonyl or phenylazo.

10. A disperse dye according to any one of claims 7 to 9, wherein X is hydrogen, methyl, methoxy, chlorine, bromine, acetylamino or ureido.

11. A disperse dye according to any one of claims 7 to 10, wherein Y is chlorine, methyl, methoxy methoxyethyl, methoxyethoxy or in particular hydrogen.

12. A disperse dye of the formulae 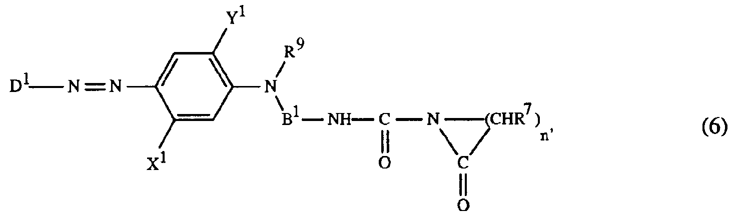 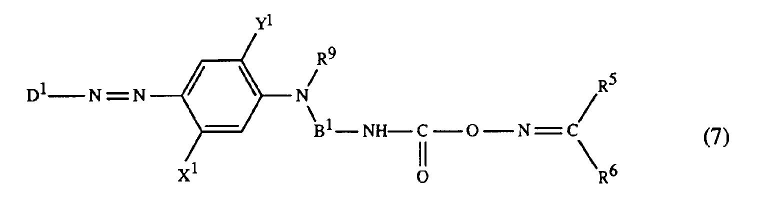 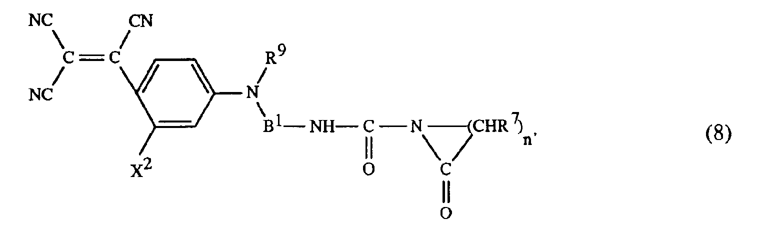 or 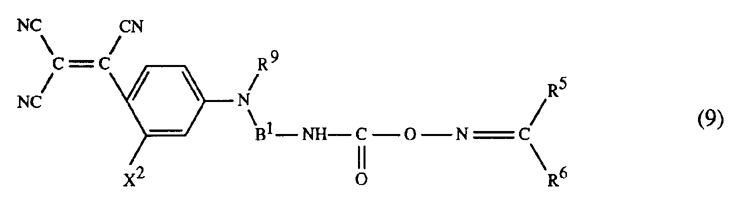 where
D¹ is a benzothiazolyl radical which is unsubstituted or monosubstituted or disubstituted by chlorine, or is a phenyl radical which is monosubstituted or disubstituted by nitro, chlorine, cyano, methylsulfonyl, ethylsulfonyl or phenylazo,
X¹ is hydrogen, methyl, methoxy, chlorine, bromine or acetylamino,
Y¹ is chlorine, methyl, methoxy, methoxyethyl, methoxyethoxy or hydrogen,
X² is hydrogen, methyl, methoxy, chlorine or bromine,
R⁹ is C₁-C₄alkyl which is unsubstituted or substituted by hydroxyl, cyano, C₁-C₄alkoxy or phenyl,
B¹ is a radical of the formula 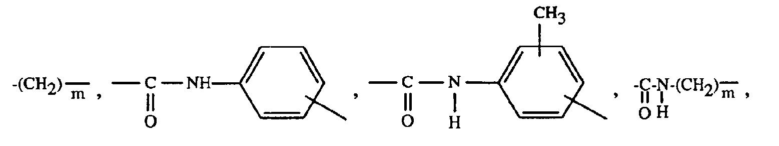 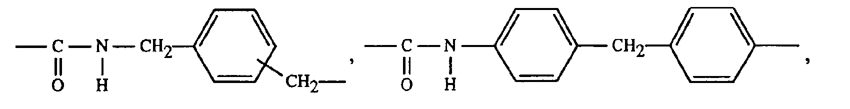 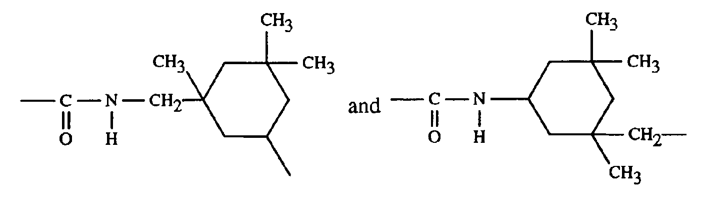
where m is an integer from 2 to 6,
R⁵ and R⁶ are each independently of the other C₁-C₆alkyl or R⁵ and R⁶ together with the carbon atom linking them form a cyclopentyl, cyclohexyl or cycloheptyl radical,
R⁷ is methyl or hydrogen, and
n' is 4, 5, 6 or 7.

13. A process for preparing a dye according to claim 7, which comprises reacting a diazotized amine of the formula 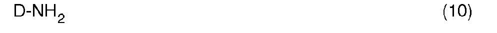 or tetracyanoethylene with a compound of the formula

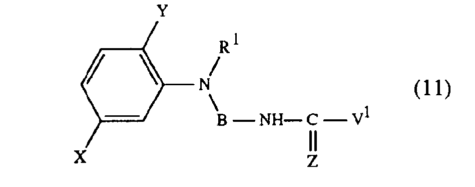

14. A process for preparing a dye of the formula (4) according to claim 7 in which V¹ is an oxime radical , which comprises reacting a compound of the formula 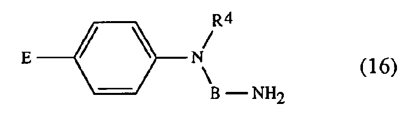 with a haloformic ester of an oxime of the formula

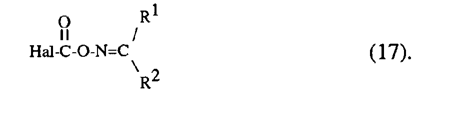

15. A process for preparing a dye of the formula 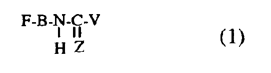 or 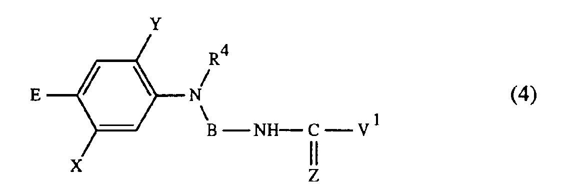 where B is a bridge member of the formula 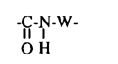 where W is a divalent organic radical and the other symbols are each as defined in claims 1 and 7, which comprises reacting a diisocyanate of the formula 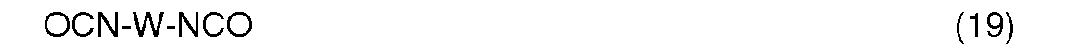 in any desired order with a dye which contains a reactive -OH or 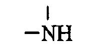 group and with an oxime of the formula (2) or a lactam of the formula (3).

16. A compound of the formula 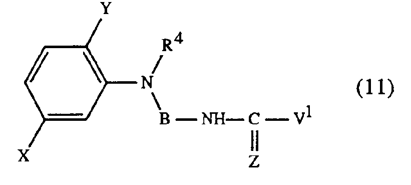 where X, Y, R⁴, B, Z and V¹ are each as defined in claim 7.

17. A process for preparing a compound according to claim 16, which comprises reacting a haloalkyl isocyanate or isothiocycnaate of the formula 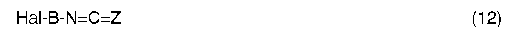 with an oxime or lactam of the formula 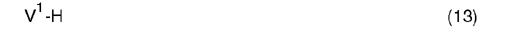 and reacting the resulting reaction product of the formula 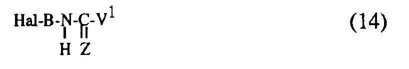 with a compound of the formula

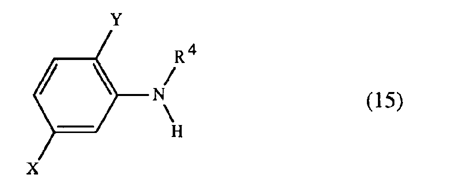

18. The use of a disperse dye according to any one of claims 1 to 12 for dyeing or printing cellulosic or synthetic hydrophobic fibre material, in particular textile material.

19. The use according to claim 18 for dyeing or printing textile material made of polyester fibres.

20. A process for dyeing and printing semisynthetic or synthetic hydrophobic fibre material, in particulartextile material, which comprises applying to or incorporating into the material one or more of the compounds defined in claims 1 to 12 and then carrying out a thermal aftertreatment at 100 to 240°C for 30 to 500 seconds.

21. A process according to claim 20, wherein the thermal aftertreatment is carried out at 180 to 220°C for 45 to 200 seconds.

22. A material dyed or printed according to claim 20 or 21.

## Claims (Claims for the following Contracting State(s) : ES)

1. A process for preparing a disperse dye of the formula 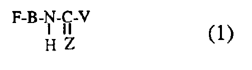 where
F is the radical of a dye which is free of water-solubilizing groups,
B is a bridge member or a direct bond,
Z is 0 or S and
V is the radical of a group H-V, where H-V is an oxime of the formula 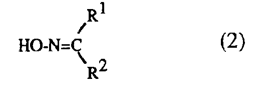
or a lactam of the formula 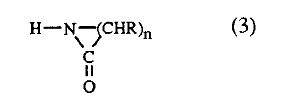 in which
R¹ and R² are each independently of the other substituted or unsubstituted alkyl or aryl, or R¹ and R² together with the carbon atom linking them form a cycloaliphatic ring,
R is hydrogen or alkyl and
n is an integer from 4 to 11,
which comprises reacting a dye F-H with a compounds of the formula 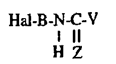
where F B, Z and V are each as defined under the formula (1) and Hal ic chlorine or bromine.

2. A process according to claim 1, wherein F is a dye radical from one of the following dye classes: nitro dyes, nitrodiphenylamine dyes, methine dyes, quinoline dyes, aminonaphthoquinone dyes, coumarin dyes and in particular anthraquinone dyes, tricyanovinyl dyes and azo dyes, such as monoazo and disazo dyes.

3. A process according to either of claims 1 and 2, wherein B is a straight-chain or branched alkylene group of 2 to 8 carbon atoms or one of the following groups: 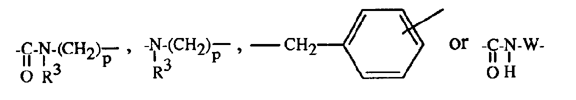 where R³ is hydrogen or C₁-C₆alkyl, p is an integer from 1 to 8 and W is a divalent organic radical.

4. A process according to any one of claims 1 to 3, wherein Z is oxygen.

5. A process according to any one of claims 1 to 4, wherein R¹ and R² are each independently of the other C₁-C₁₂alkyl, phenyl, naphthyl or pyridyl or R¹ and R² together with the carbon atom linking them are cyclopentyl, cyclohexyl or cycloheptyl.

6. A process according to any one of claims 1 to 4, wherein R is methyl or hydrogen and n is 4, 5 or 6.

7. A process for preparing a disperse dye of the formula 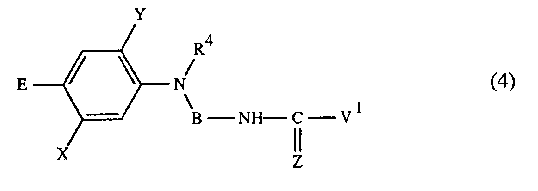 where
E is D-N=N- or 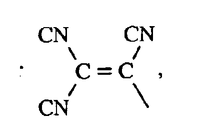
wherein D is the radical of a carbocyclic or heterocyclic diazo component,
X is hydrogen, C₁-C₄alkyl, C₁-C₄alkoxy, acylamino, halogen, Cᵢ-C₄alkylsulfonylamino or a group of the formula
-NH-CO-NHQ, in which Q is hydrogen, C₁-C₄alkyl or phenyl,
Y is hydrogen, halogen, C₁-C₄alkyl, C₁-C₄alkoxy, C₁-C₄alkoxy-C₁-C₄alkyl or C₁-C₄alkoxy-C₂-C₄alkoxy
R⁴ is C₁-C₁₂alkyl, C₂-C₆alkenyl or phenyl, or Y and R⁴ together with the nitrogen atom and the two carbon atoms linking them form a 5- or 6-membered ring,
B is a radical of the formula 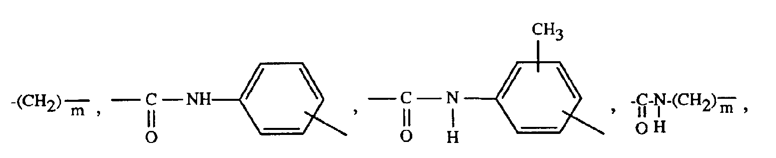 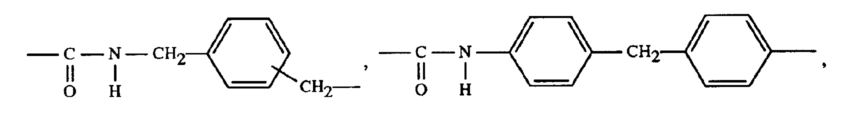 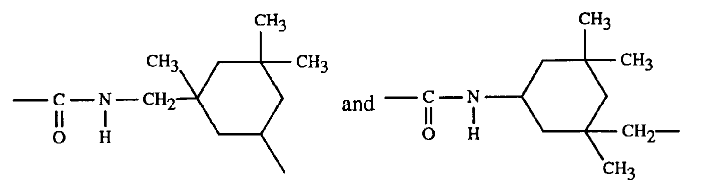
where m is an integer from 2 to 6,
Z is O or S and
V¹ is 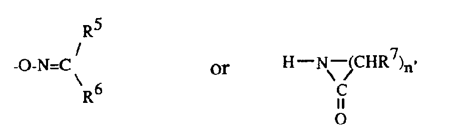
where R⁵ and R⁶ are each independently of the other C₁-Cₛalkyl or R⁵ and R⁶ together with the carbon atom linking them form a cyclopentyl, cyclohexyl or cycloheptyl radical,
R⁷ is methyl or hydrogen and
n' is 4, 5, 6 or 7,
which comprises reacting a diazotized amine of the formula 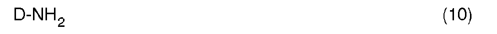 or tetracyanoethylene with a compound of the formula

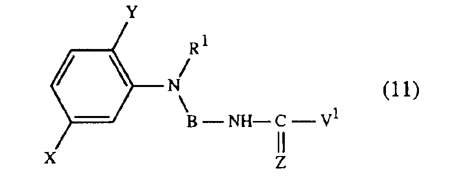

8. A process according to claim 7, wherein E is D-N=N-.

9. A process according to either of claims 7 and 8, wherein D is a benzothiazolyl or benzisothiazolyl radical which is unsubstituted or monosubstituted or disubstituted by bromine, nitro or chlorine or is a phenyl radical which is monosubstituted or disubstituted by nitro, chlorine, cyano, methylsulfonyl or ethylsulfonyl or phenylazo.

10. A process according to any one of claims 7 to 9, wherein X is hydrogen, methyl, methoxy chlorine, bromine, acetylamino or ureido.

11. A process according to any one of claims 7 to 10, wherein Y is chlorine, methyl, methoxy, methoxyethyl, methoxyethoxy or in particular hydrogen.

12. A process for preparing a dye of the formula (4) according to claim 7 in which V¹ is an oxime radical, which comprises reacting a compound of the formula 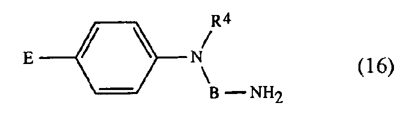 with a haloformic ester of an oxime of the formula

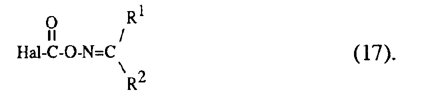

13. A process for preparing a dye of the formula 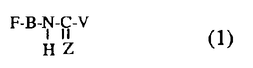 or 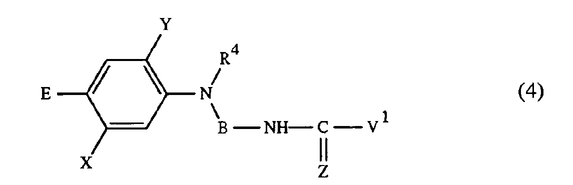 where B is a bridge member of the formula 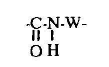 where W is a divalent organic radical and the other symbols are each as defined in claims 1 and 7, which comprises reacting a diisocyanate of the formula 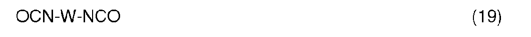 in any desired order with a dye which contains a reactive -OH or 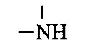 group and with an oxime of the formula (2) or a lactam of the formula (3).

14. A process according to any one of claims 7, 12, or 13 for preparing a dye of the formulae 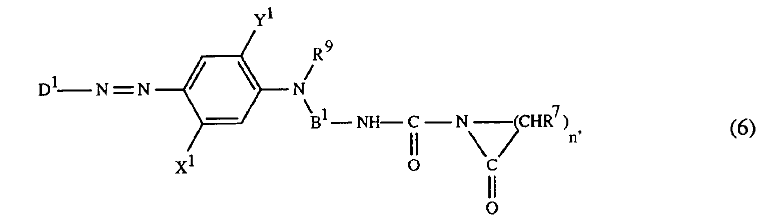 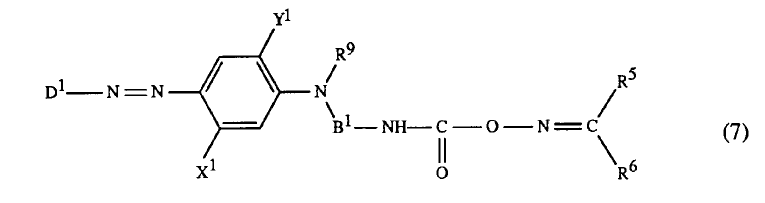 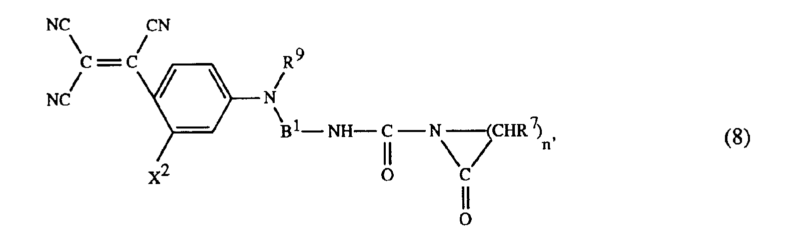 or 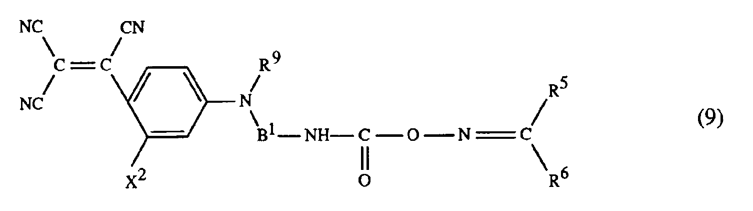 where
D¹ is a benzothiazolyl radical which is unsubstituted or monosubstituted or disubstituted by chlorine, or is a phenyl radical which is monosubstituted or disubstituted by nitro, chlorine, cyano, methylsulfonyl, ethylsulfonyl or phenylazo,
X¹ is hydrogen, methyl, methoxy, chlorine, bromine or acetylamino,
Y¹ is chlorine, methyl, methoxy, methoxyethyl, methoxyethoxy or hydrogen,
X² is hydrogen, methyl, methoxy, chlorine or bromine,
R⁹ is C₁-C₄alkyl which is unsubstituted or substituted by hydroxyl, cyano, C₁ -C₄alkoxy or phenyl,
B¹ is a radical of the formula 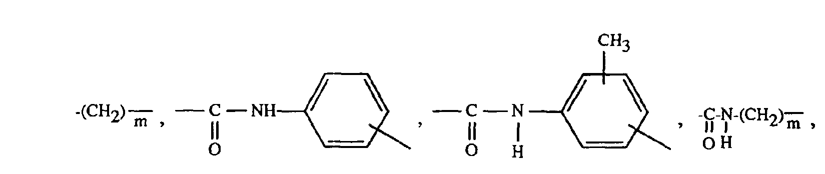 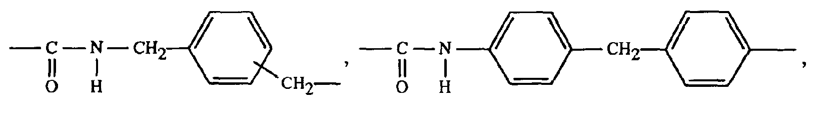 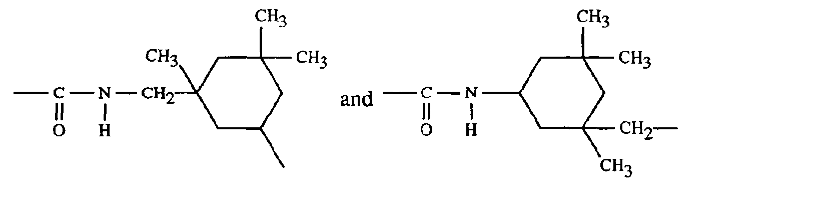
where m is an integer from 2 to 6,
R⁵ and R⁶ are each independently of the other C₁-C₆alkyl or R⁵ and R⁶ together with the carbon atom linking them form a cyclopentyl, cyclohexyl or cycloheptyl radical,
R⁷ is methyl or hydrogen, and
n' is 4, 5, 6 or 7.

15. A process for preparing a compound of the formula 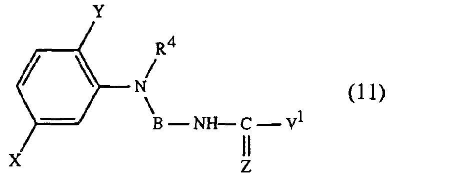
where X, Y, R⁴, B, Z and V¹ are each as defined in claim 7,
which comprises reacting a haloalkyl isocyanate or isothiocycnaate of the formula 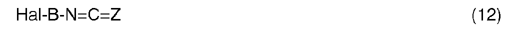
with an oxime or lactam of the formula 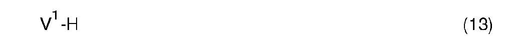
and reacting the resulting reaction product of the formula 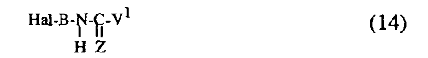
with a compound of the formula

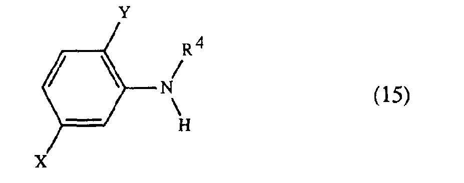

16. The use of a disperse dye according to any one of claims 1 to 12 for dyeing or printing cellulosic or synthetic hydrophobic fibre material, in particular textile material.

17. The use according to claim 16 for dyeing or printing textile material made of polyester fibres.

18. A process for dyeing and printing semisynthetic or synthetic hydrophobic fibre material, in particulartextile material, which comprises applying to or incorporating into the material one or more of the compounds defined in claims 1 to 12 and then carrying out a thermal aftertreatment at 100 to 240°C for 30 to 500 seconds.

19. A process according to claim 18, wherein the thermal aftertreatment is carried out at 180 to 220°C for 45 to 200 seconds.

20. A material dyed or printed according to claim 18 or 19.

## Revendications (Revendications pour l'(les) Etat(s) contractant(s) suivant(s) : BE, CH, DE, FR, GB, IT, LI)

1. Colorants de dispersion de formule 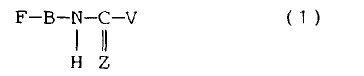 dans laquelle
F représente le radical d'un colorant exempt de groupes solubilisant dans l'eau,
B est un élément de pont ou une liaison directe,
Z est O ou S, et
V représente le radical d'un groupe H-V, où H-V est une oxime de formule 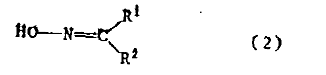 ou un lactame de formule 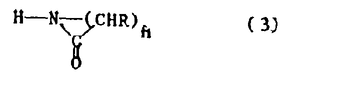 où,
R¹ et R² représentent, indépendamment l'un de l'autre, un alkyle ou un aryle, éventuellement substitutés, ou bien R¹ et R² forment avec l'atome de carbone qui les relie, un cycle cycloaliphatique,
R représente un atome d'hydrogène ou un groupe alkyle, et
n représente un nombre entier entre 4 et 11.

2. Colorants de dispersion selon la revendication 1, caractérisés en ce F représente un radical de colorant appartenant à l'une des classes de colorant suivantes: colorant nitro, colorants nitrodiphénylamine, colorant méthine, colorants quinoléine, colorants aminonaphtoquinone, colorants coumarine et, en particulier, colorants anthraquinone, colorants tricyanovinyle et colorants azo tels que colorants monoazo et diazo.

3. Colorants de dispersion selon l'une des revendications 1 ou 2, caractérisés en ce que B représente un groupe alcylène linéaire ou ramifié ayant de 2 à 8 atomes de carbone ou l'un des groupes suivants: 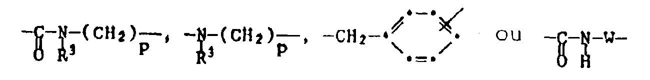 où R³ représente un atome d'hydrogène ou un alkyle en C₁-C₆ p un nombre entier compris entre 1 et 8 et W un radical organique bivalent.

4. Colorants de dispersion selon l'une des revendications 1 à 3, caractérisés en ce que Z représente un atome d'oxygène.

5. Colorants de dispersion selon l'une des revendications 1 à 4, caractérisés en ce que R¹ et R² représentent, indépendamment l'un de l'autre, un alkyle en C₁-C_{12'} un phényle, un naphtyle ou un pyridyle, ou bien R¹ et R² forment avec l'atome de carbone qui les relie, un groupe cyclopentyle, cyclohexyle ou cycloheptyle.

6. Colorants de dispersion selon l'une des revendications 1 à 4, caractérisés en ce que R représente un groupe méthyle ou un atome d'hydrogène et n représente 4, 5 ou 6.

7. Colorants de dispersion de formule 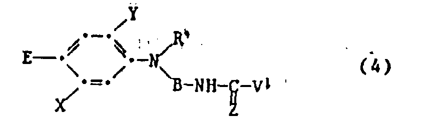 dans laquelle
E représente D-N=N- ou 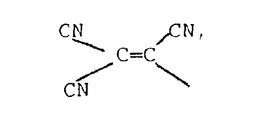 où D est le radical d'un composant diazo carbocyclique ou hétérocyclique,
X représente un atome d'hydrogène, un alkyle en C₁-C_{4'} un alcoxy en C₁-C_{4'} un acylamino, un atome d'halogène, un alkylsulfonylamino en C₁-C₄ ou un groupe de formule -NH-CO-NHQ, où Q représente un atome d'hydrogène, un alkyle en C₁-C₄ ou un phényle,
Y représente un atome d'hydrogène ou d'halogène, un alkyle en C₁-C_{4'} un alcoxy en C₁-C_{4'} un C₁-C₄-alcoxy-C₁-C₄₋alkyle ou un C₁-C₄₋alcoxy-C₂-C₄-alcoxy,
R⁴ représente un alkyle en C₁-C_{12'} un alcényle en C₂-Ce ou un phényle, ou Y et R⁴ forment, avec l'atome d'azote et les deux atomes de carbone qui les relient, un cycle à 5 ou 6 éléments,
B représente un radical de formules 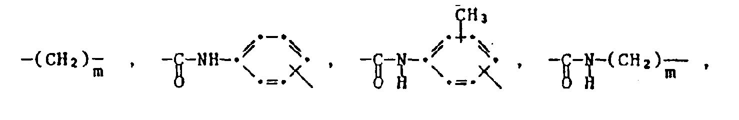 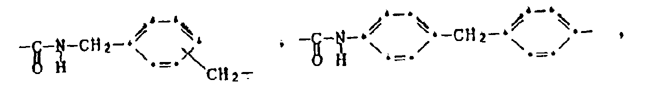 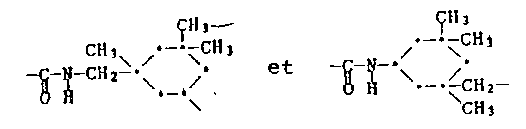 dans lesquelles
m représente un nombre entier entre 2 et 6,
Z représente O ou S et
V¹ 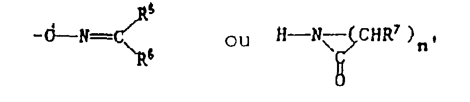 où
R⁵ et R⁶ représentent, indépendamment l'un de l'autre, un alkyle en C₁-C₆ ou R⁵ et R⁶ forment, avec l'atome de carbone qui les relie, un radical cyclopentyle, cyclohexyle ou cycloheptyle,
R⁷ représente un groupe méthyle ou un atome d'hydrogène et
n' représente 4, 5, 6 ou 7.

8. Colorants de dispersion selon la revendication 7, dans lesquels E représente D-N=N-.

9. Colorants de dispersion selon les revendications 7 et 8, dans lesquels D représente un radical benzothiazolyle ou benzisothiazolyle, qui est non substitué ou substitué une ou deux fois par du brome, un nitro ou du chlore ou est un radical phényle qui est substitué une ou deux fois par un groupe nitro, chlore, cyano, méthyle ou éthylsulfonyle ou phénylazo.

10. Colorants de dispersion selon l'une des revendications 7 à 9, dans lesquels X représente un atome d'hydrogène, un groupe méthyle, méthoxy, un atome de chlore ou de brome, un acétylamino ou un uréido.

11. Colorants de dispersion selon l'une des revendications 7 à 10, dans lesquels Y représente un atome de chlore, un groupe méthyle, méthoxy, méthoxyéthyle ou méthoxyéthoxy ou surtout, un atome d'hydrogène.

12. Colorants de dispersion de formule 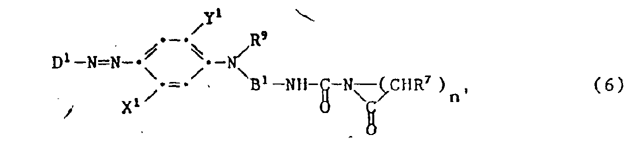 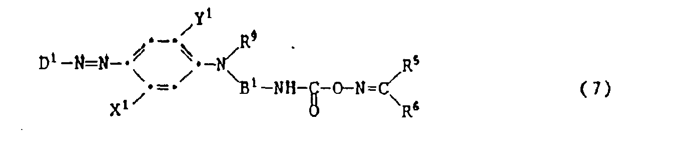 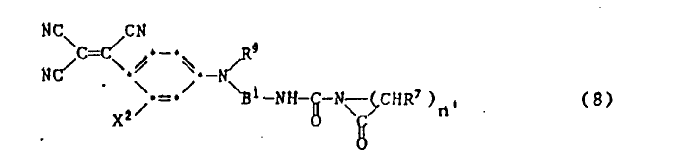 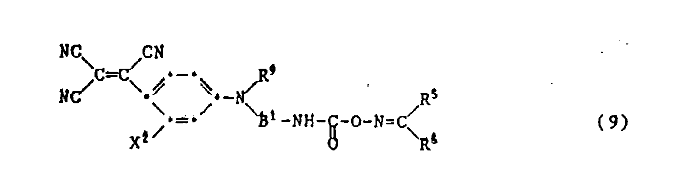 dans lesquelles
D¹ représente un radical benzothiazolyle, qui est non substitué ou qui est substitué une ou deux fois par le chlore, ou un radical phényle qui est substitué une ou deux fois par un radical nitro, chlore, cyano, méthyl-ou éthylsulfonyle ou phénylazo,
X¹ représente un atome d'hydrogène, un groupe méthyle, méthoxy, un atome de chlore ou de brome ou un acétylamino,
Y¹ représente un atome de chlore, un groupe méthyle, méthoxy, méthoxyéthyle, méthoxyéthoxy ou un atome d'hydrogène,
X² représente un atome d'hydrogène, un groupe méthyle, méthoxy, ou un atome de chlore ou de brome,
R⁹ représente un radical alkyle en C₁-C₄ qui est non substitué ou qui est substitué par un hydroxy, un cyano, un alcoxy en C₁-C₄ ou un phényle,
B¹ représente un radical de formule 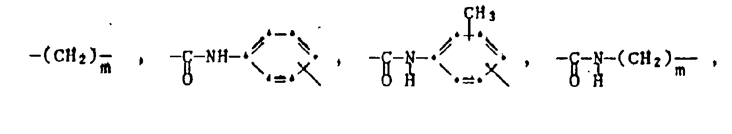 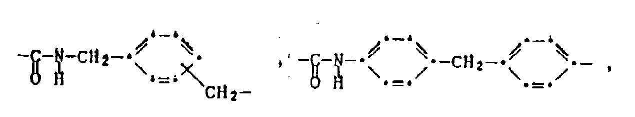 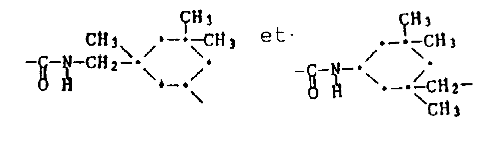 dans lesquelles
m est un nombre entier entre 2 et 6,
R⁵ et R⁶ représentent, indépendamment l'un de l'autre, un alkyle en C₁-Ce ou bien R⁵ et R⁶ forment, avec l'atome de carbone qui les relie, un radical cyclopentyle, cyclohexyle ou cycloheptyle,
R⁷ est un groupe méthyle ou un atome d'hydrogène, et
n' représente 4, 5, 6 ou 7.

13. Procédé de préparation des colorants selon la revendication 7, caractérisé en ce qu'on fait réagir une amine diazotée de formule 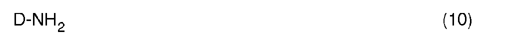 ou du tétracyanoéthylène avec un composé de formule

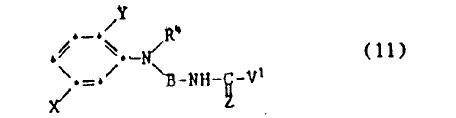

14. Procédé de préparation des colorants de formule (4) selon la revendication 7, dans lesquels V' représente un radical oxime, caractérisé en ce que l'on fait réagir un composé de formule 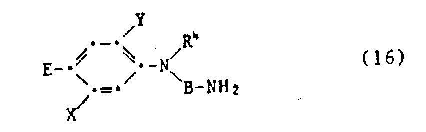 avec un ester de l'acide halogénoformique d'une oxime de formule

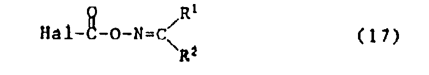

15. Procédé de préparation de colorants de formule 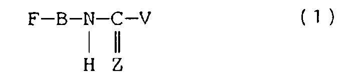 ou 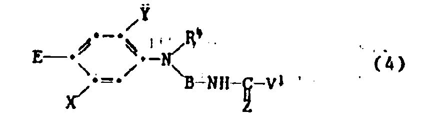 dans lesquelles B représente un élément de pont de formule 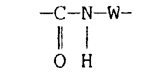 où W représente un radical organique bivalent et où les autres symboles ont les significations indiquées dans les revendications 1 et 7, caractérisé en ce qu'on fait réagir un diisocyanate de formule 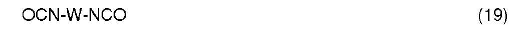 dans un ordre de succession quelconque avec un colorant contenant un groupe réactionnel -OH ou -NH et avec une oxime de formule (2) ou un lactame de formule (3).

16. Composés de formule 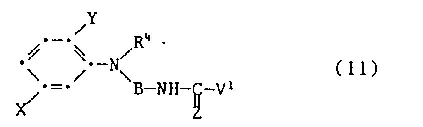 dans laquelle X, Y, R⁴, B, Z et V ont les significations indiquées dans la revendication 7.

17. Procédé de préparation des composés selon la revendication 16, caractérisé en ce qu'on fait réagir un isocyanate d'halogénoalkyle ou un isothiocyanate d'halogénoalkyle de formule 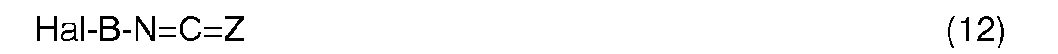 avec une oxime ou un lactame de formule 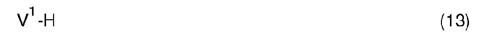 et que l'on fait réagir le produit de réaction obtenu de formule 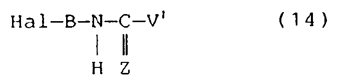 avec un composé de formule

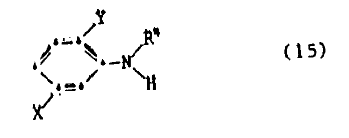

18. Utilisation des colorants de dispersion selon l'une des revendications 1 à 12, pour la coloration ou l'impression de matériaux fibreux, hydrophobes, semi-synthétiques ou synthétiques et, en particulier, de matériaux textiles.

19. Utilisation selon la revendication 18 pour la coloration ou l'impression de matériaux textiles formés de fibres de polyester.

20. Procédé de coloration ou d'impression de matériaux fibreux hydrophobes semi-synthétiques ou synthétiques, et en particulier des matériaux textiles, caractérisé en ce que l'on applique sur le matériau un ou plusieurs des composés définis par les revendications 1 à 12 ou qu'on les incorpore à ce matériau et qu'on effectue ensuite un post-traitement thermique pendant 30 à 500 secondes entre 100 et 240°C.

21. Procédé selon la revendication 20, caractérisé en ce que l'on effectue le post-traitement thermique pendant 45 à 200 secondes entre 180 et 220°C.

22. Matériau coloré ou imprimé selon la revendication 20 ou 21.

## Revendications (Revendications pour l'(les) Etat(s) contractant(s) suivant(s) : ES)

1. Procédé de préparation des colorants de dispersion de formule 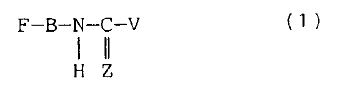 dans laquelle
F représente le radical d'un colorant exempt de groupes solubilisant dans l'eau,
B est un élément de pont ou une liaison directe,
Z est O ou S, et
V représente le radical d'un groupe H-V, où H-V est une oxime de formule 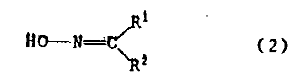 ou un lactame de formule 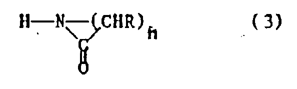 où,
R¹ et R² représentent, indépendamment l'un de l'autre, un alkyle ou un aryle, éventuellement substitutés, ou bien R¹ et R² forment avec l'atome de carbone qui les relie, un cycle cycloaliphatique,
R représente un atome d'hydrogène ou un groupe alkyle, et
n représente un nombre entier entre 4 et 11,
caractérisé en que l'on fait réagir un colorant F-H avec un composé de formule 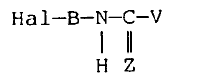 dans laquelle F, B, Z et V ont les significations indiquées à propos de la formule (1) et où Hal représente du chlore ou du brome.

2. Procédé selon la revendication 1, caractérisé en ce que F représente un radical de colorant appartenant à l'une des classes de colorant suivantes: colorant nitro, colorants nitrodiphénylamine, colorant méthine, colorants quinoléine, colorants aminonaphtoquinone, colorants coumarine et, en particulier, colorants anthraquinone, colorants tricyanovinyle et colorants azo tels que colorants monoazo et diazo.

3. Procédé selon l'une des revendications 1 ou 2, caractérisé en ce que B représente un groupe alcylène linéaire ou ramifié ayant de 2 à 8 atomes de carbone ou l'un des groupes suivants: 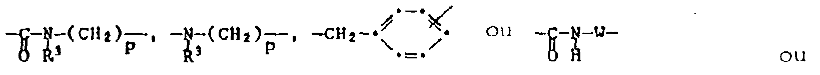 où R³ représente un atome d'hydrogène ou un alkyle en C₁-C₆, p un nombre entier compris entre 1 et 8 et W un radical organique bivalent.

4. Procédé selon l'une des revendications 1 à 3, caractérisé en ce que Z représente un atome d'oxygène.

5. Procédé selon l'une des revendications 1 à 4, caractérisé en ce que R¹ et R² représentent, indépendamment l'un de l'autre, un alkyle en C₁-C_{12'} un phényle, un naphtyle ou un pyridyle, ou bien R¹ et R² forment avec l'atome de carbone qui les relie, un groupe cyclopentyle, cyclohexyle ou cycloheptyle.

6. Procédé selon l'une des revendications 1 à 4, caractérisé en ce que R représente un groupe méthyle ou un atome d'hydrogène et n représente 4, 5 ou 6.

7. Procédé de préparation des colorants de dispersion de formule 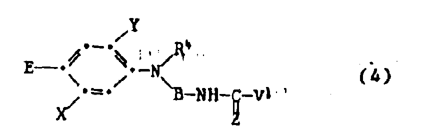 dans laquelle
E représente D-N=N- ou 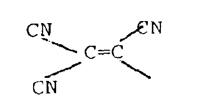 où D est le radical d'un composant diazo carbocyclique ou hétérocyclique,
X représente un atome d'hydrogène, un alkyle en C₁-C_{4'} un alcoxy en C₁-C_{4'} un acylamino, un atome d'halogène, un alkylsulfonylamino en C₁-C₄ ou un groupe de formule -NH-CO-NHQ, où Q représente un atome d'hydrogène, un alkyle en C₁-C₄ ou un phényle,
Y représente un atome d'hydrogène ou d'halogène, un alkyle en C₁-C_{4'} un alcoxy en C₁-C_{4'} un C₁-C₄-alcoxy-C₁-C₄₋alkyle ou un C₁-C₄₋alcoxy-C₂-C₄-alcoxy,
R⁴ représente un alkyle en C₁-C_{12'} un alcényle en C₂-C₆ ou un phényle, ou Y et R⁴ forment, avec l'atome d'azote et les deux atomes de carbones qui les relient, un cycle à 5 ou 6 éléments,
B représente un radical de formules 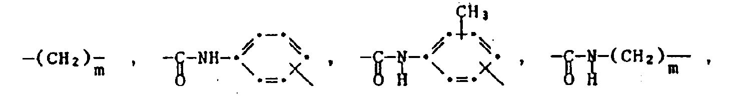 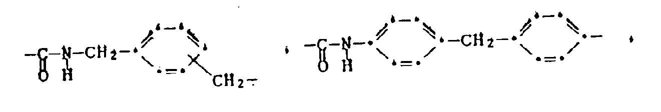 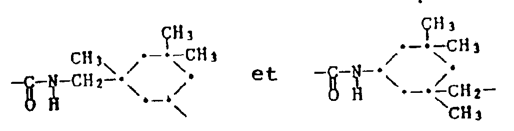 dans lesquelles
m représente un nombre entier entre 2 et 6,
Z représente O ou S et
V¹ 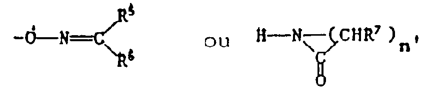 où
R⁵ et R⁶ représentent, indépendamment l'un de l'autre, un alkyle en C₁-C₆ ou R⁵ et R⁶ forment, avec l'atome de carbone qui les relie, un radical cyclopentyle, cyclohexyle ou cycloheptyle,
R⁷ représente un groupe méthyle ou un atome d'hydrogène et
n' représente 4, 5, 6 ou 7.
caractérisé en ce qu'on fait réagir une amine diazotée de formule 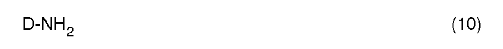 ou du tétracyanoéthylène avec un composé de formule

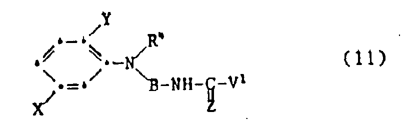

8. Procédé selon la revendication 7, dans lequel E représente D-N=N-.

9. Procédé selon les revendications 7 et 8, dans lequel D représente un radical benzothiazolyle ou benzisothiazolyle, qui est non substitué ou substitué une ou deux fois par du brome, du nitro ou du chlore ou est un radical phényle qui est substitué une ou deux fois par un groupe nitro, chlore, cyan, méthyl- ou éthylsulfonyle ou phénylazo.

10. Procédé selon l'une des revendications 7 à 9, dans lequel X représente un atome d'hydrogène, un groupe méthyle ou méthoxy, un atome de chlore ou de brome, un groupe acétylamino ou uréido.

11. Procédé selon l'une des revendications 7 à 10, dans lequel Y représente un atome de chlore, un groupe méthyle, méthoxy, méthoxyéthyle ou méthoxyéthoxy ou surtout, un atome d'hydrogène.

12. Procédé de préparation des colorants de formule (4) selon la revendication 7, dans lequel V¹ représente un radical oxime, caractérisé en ce que l'on fait réagir un composé de formule 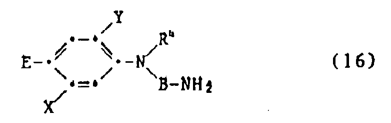 avec un ester de l'acide halogénoformique d'une oxime de formule

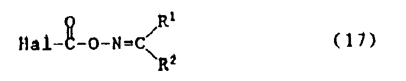

13. Procédé de préparation de colorants de formule 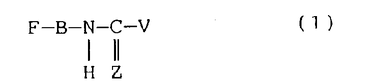 ou 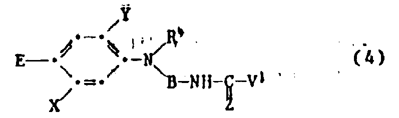 dans lesquelles B représente un élément de pont de formule 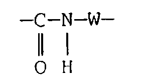 où W représente un radical organique bivalent et où les autres symboles ont les significations indiquées dans les revendications 1 et 7, caractérisé en ce qu'on fait réagir un diisocyanate de formule 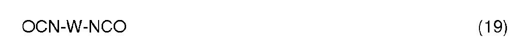 dans un ordre de succession quelconque avec un colorant contenant un groupe réactionnel -OH ou -NH et avec une oxime de formule (2) ou un lactame de formule (3).

14. Procédé selon l'une des revendications 7, 12 ou 13 pour la préparation des colorants de formule 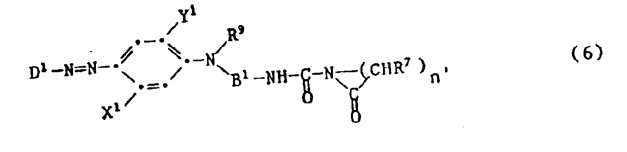 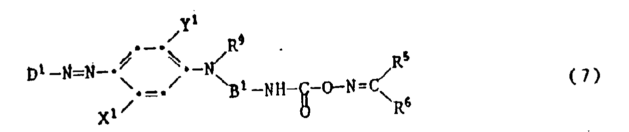 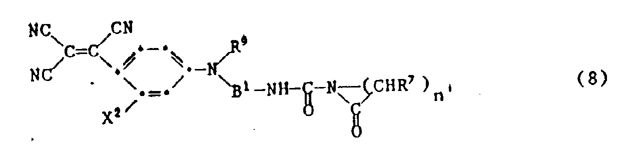 ou 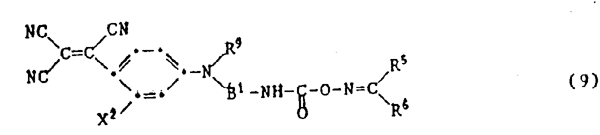 dans lesquelles
D¹ représente un radical benzothiazolyle, qui est non substitué ou qui est substitué une ou deux fois par le chlore, ou un radical phényle qui est substitué une ou deux fois par un radical nitro, chlore, cyano, méthyl-ou éthylsulfonyle ou phénylazo,
X¹ représente un atome d'hydrogène, un groupe méthyl, méthoxy un atome de chlore ou de brome ou un acétylamino,
Y¹ représente un atome de chlore, un groupe méthyl, méthoxy, méthoxyéthyle, méthoxyéthoxy ou un atome d'hydrogène,
X² représente un atome d'hydrogène, un groupe méthyle, méthoxy, ou un atome de chlore ou de brome,
R⁹ représente un radical alkyle en C₁-C₄ qui est non substitué ou qui est substitué par un hydroxy, un cyano, un alcoxy en C₁-C₄ ou un phényle,
R¹ représente un radical de formules 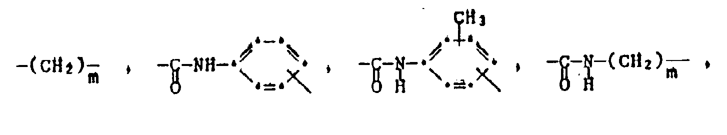 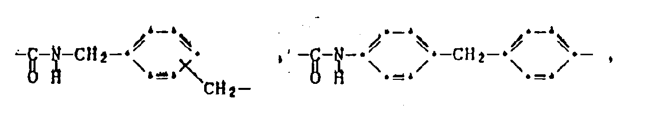 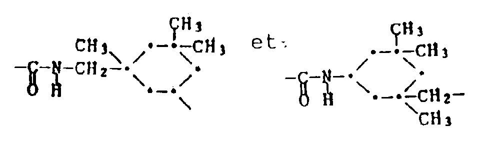 dans lesquelles
m est un nombre entier entre 2 et 6,
R⁵ et R⁶ représentent, indépendamment l'un de l'autre, un alkyle en C₁-C₆ ou bien R⁵ et R⁶ forment, avec l'atome de carbone qui les relie, un radical cyclopentyle, cyclohexyle ou cycloheptyle,
R⁷ est un groupe méthyle ou un atome d'hydrogène, et
n' représente 4, 5, 6 ou 7.

5. Procédé de préparation des composés de formule 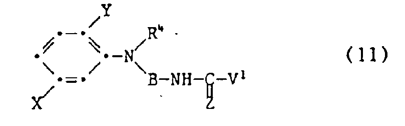 où X, Y, R⁴, B, Z et V ont les significations indiquées dans la revendication 7,
caractérisé en ce que l'on fait réagir un isocyanate d'halogénoalkyle ou un isothiocyanate d'halogénoalkyle de formule 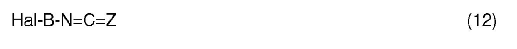 avec une oxime ou un lactame de formule 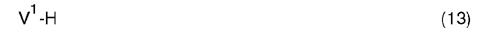 et que l'on fait réagir le produit de réaction obtenu de formule 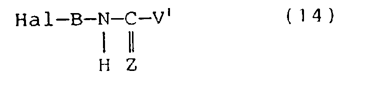 avec un composé de formule

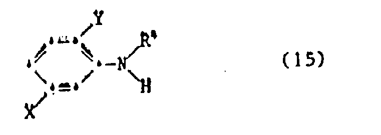

16. Utilisation des colorants de dispersion selon l'une des revendications 1 à 12, pour la coloration ou l'impression de matériaux fibreux, hydrophobes, semi-synthétiques ou synthétiques et, en particulier, de matériaux textiles.

17. Utilisation selon la revendication 16 pour la coloration ou l'impression de matériaux textiles formés de fibres de polyester.

18. Procédé de coloration ou d'impression de matériaux fibreux hydrophobes semi-synthétiques ou synthétiques, et en particulier des matériaux textiles, caractérisé en ce que l'on applique sur le matériau un ou plusieurs des composés définis par les revendications 1 à 12 ou qu'on les incorpore à ce matériau et qu'on effectue ensuite un post-traitement thermique pendant 30 à 500 secondes entre 100 et 240°C.

19. Procédé selon la revendication 18, caractérisé en ce que l'on effectue le post-traitement thermique pendant 45 à 200 secondes entre 180 et 220°C.

20. Matériau coloré ou imprimé selon la revendication 18 ou 19.
